# EUROPEAN PATENT SPECIFICATION

(11) **EP 4 265 274 B1**
(45) Date of publication and mention of the grant of the patent: **19.03.2025**
(21) Application number: 20965585.1
(22) Date of filing: 18.12.2020
(51) Int. Cl.: A61K 47/68, C07K 16/28, A61P 35/00, A61K 39/00

(54) **B7-H3 TARGETING ANTIBODY-DRUG CONJUGATE, AND PREPARATION METHOD THEREFOR AND USE THEREOF**
GEGEN B7-H3 GERICHTETES ANTIKÖRPER-WIRKSTOFF-KONJUGAT UND HERSTELLUNGSVERFAHREN DAFÜR UND VERWENDUNG DAVON
CONJUGUÉ ANTICORPS-MÉDICAMENT CIBLANT B7-H3, SON PROCÉDÉ DE PRÉPARATION ET SON UTILISATION

(43) Date of publication of application: 25.10.2023
(73) Proprietor: Shanghai Fudan-Zhangjiang Bio-Pharmaceutical Co., Ltd., Shanghai 201210 (CN)
(72) Inventor: GUO, Qingsong, Shanghai 201210 (CN); SHEN, Yijun, Shanghai 201210 (CN); YANG, Tong, Shanghai 201210 (CN); BAO, Bin, Shanghai 201210 (CN); GAO, Bei, Shanghai 201210 (CN); WU, Fang, Shanghai 201210 (CN); XU, Jun, Shanghai 201210 (CN)
(74) Representative: Dehns
(86) International application number: PCT/CN2020/137472
(87) International publication number: WO 2022/126569

(56) References cited:
- EP-A1- 3 991 754
- WO-A1-2017/214335
- WO-A1-2019/039483
- WO-A1-2020/031936
- WO-A2-2016/070089
- CN-A- 104 755 494
- CN-A- 110 305 213
- POUDEL YAM B. ET AL: "Chemical Modification of Linkers Provides Stable Linker-Payloads for the Generation of Antibody-Drug Conjugates", ACS MEDICINAL CHEMISTRY LETTERS, vol. 11, no. 11, 12 November 2020 (2020-11-12), US, pages 2190 - 2194, XP093125915, ISSN: 1948-5875, DOI: 10.1021/acsmedchemlett.0c00325

## Description

### TECHNICAL FIELD

The present disclosure relates to the field of biomedicine, and in particularly relates to a B7-H3-targeting antibody-drug conjugate, a preparation method therefor, and a use thereof.

### BACKGROUND

B7-H3, also known as CD276, was first reported in 2001 (Chapoval AI et al., Nat Immmunol 2001, 2(3):269-274), and it is considered not to belong to butyrophilin and myelin oligodendrocyte glycoprotein because the protein thereof lacks a heptad structure and B30.2 domain, and it is identified as a member of the immunoglobulin superfamily, B7 family (Chapoval AI et al., Nat Immmunol 2001, 2(3):269-274), and it differs from other members of the family such as PD-L1, B7-H4, CD80, CD86 in that B7-H3 exists in the human body in the form of two different variants, namely 2IgB7-H3 and 4IgB7-H3, wherein 4IgB7-H3 is an exon duplication of 2IgB7-H3, which mainly exists in the form of 4IgB7-H3 in human body (Sun M et al., The Journal of Immunology 2002, 168(12):6294-6297; Ling V et al., Genomics 2003, 82(3):365-377; Steinberger P et al., J IMMUNOL 2004, 172(4):2352-2359), while only 2IgB7-H3 structure is contained in mice (Sun M et al., The Journal of Immunology 2002, 168(12):6294-6297). The results of the study showed that the 2IgB7-H3 of the natural mouse and the 4IgB7-H3 of the human show similar functions without functional differences (Ling V et al., Genomics 2003, 82(3):365-377; Hofmeyer KA et al., Proc Natl Acad Sci U S A 2008, 105(30):10277-10278.), the crystal structure shows that the FG loop of the IgV region of the protein is an important epitope for serving the function of B7-H3 (Vigdorovich V et al., Structure 2013, 21(5):707-717).

Although the mRNA level of B7-H3 is widely expressed, for example, high B7-H3 mRNA expression level can be detected in many tissues and organs of the human body, including heart, liver, placenta, prostate, testis, uterus, pancreas, small intestine, and colon. However, the protein expression level is relatively limited to non-immune cells such as resting fibroblasts, endothelial cells, osteoblasts, amniotic fluid stem cells, as well as the surface of induced antigen-presenting cells and NK cells (Hofmeyer KA et al., Proc Natl Acad Sci U S A 2008, 105(30):10277-10278; Yi KH et al., Immunol Rev 2009, 229(1):145-151; Picarda E et al., CLIN CANCER RES 2016, 22(14):3425-3431). The protein level of B7-H3 is lowly expressed in normal healthy tissues. For example, the low protein level of B7-H3 can be detected in tissues such as liver, lung, bladder, testis, prostate, breast, placenta, and lymphoid organs of normal humans, but B7-H3 protein is overexpressed in a large number of malignant tumors and is a marker antigen of tumor cells. Studies have shown that B7-H3 can be highly expressed in many cancers such as prostate cancer, ovarian cancer, colorectal cancer, renal cell carcinoma, non-small cell lung cancer, pancreatic cancer, melanoma, gastric cancer, bladder cancer, malignant glioma, and osteosarcoma, particularly highly and abnormally expressed in many cancers such as head and neck cancer, renal cancer, brain glioma, and thyroid cancer (Roth TJ et al., CANCER RES 2007, 67(16):7893-7900; Zang X et al., MODERN PATHOL 2010, 23(8):1104-1112; Ingebrigtsen VA et al., INT J CANCER 2012, 131(11):2528-2536; Sun J et al., Cancer Immunology, Immunotherapy 2010, 59(8):1163-1171; Crispen PL et al., CLIN CANCER RES 2008, 14(16):5150-5157; Zhang G et al., LUNG CANCER 2009, 66(2):245-249; Yamato I et al., Br J Cancer 2009, 101(10):1709-1716; Tekle C et al., INT J CANCER 2012, 130(10):2282-2290; Katayama A et al., INT J ONCOL 2011, 38(5):1219-1226; Wu CP et al., World J Gastroenterol 2006, 12(3):457-459; Wu D et al., ONCOL LETT 2015, 9(3):1420-1424). B7-H3 is not only expressed on tumor cells, but it is also highly expressed on tumor neovascular endothelial cells, and is a very broad-spectrum tumor marker antigen. High expression of B7-H3 protein can promote cancer progression, which is associated with poor prognosis and poorer survival benefits for patients.

Although early research results have shown that B7-H3 can stimulate the function of activated T cells, promote the proliferation of CD4 and CD8 cells and the secretion of IFN-γ, continuous in-depth research has shown that B7-H3, as an immune checkpoint, is a negative regulator molecule of T cells, which mainly plays the role of inhibiting T cell function and downregulating T cell activity. Studies by Woong-Kyung Suh and Durbaka V. R. Prasad have shown that mouse B7-H3 protein can significantly inhibit the proliferation of CD4 and CD8 cells in a dose-dependent manner (Suh W et al., NAT IMMUNOL 2003, 4(9):899-906; Prasad DVR et al., The Journal of Immunology 2004, 173(4):2500-2506). Studies by Judith Leitner et al. also have shown that human 4Ig-B7-H3Ig and 2Ig-B7-H3Ig can inhibit the proliferation of T cells in vitro, and inhibit the secretion of related cytokines (IFN-γ, IL-2, IL-10, IL-13) (Leitner J et al., EUR J IMMUNOL 2009, 39(7):1754-1764), further analysis has pointed out that B7-H3 mainly inhibits the production of IL-2 to mediate the inhibition of T cell proliferation. Antibodies targeting and neutralizing B7-H3 in mice can significantly promote the progression of experimental autoimmune encephalomyelitis (EAE) and promote the proliferation of CD4 cells, which objectively illustrates the function of B7-H3 in inhibiting T cells in vivo (Prasad DVR et al., The Journal of Immunology 2004, 173(4):2500-2506). In Woong-Kyung Suh's study, B7-H3-deficient mice also showed the earlier occurrence of experimental autoimmune encephalomyelitis (caused by Th1 cells) than wild-type mice under immune EAE conditions, which indicates that B7-H3 mainly inhibits Th1 cells (Suh W et al., NAT IMMUNOL 2003, 4(9):899-906). As mentioned above, there is controversy about the function of B7-H3 on T cells, but the function for T cell promotion by B7-H3 has only appeared in mice studies, and there is no yet report on the promotion of T cell function by human B7-H3. Although the receptors of B7-H3 have not been identified, the main point of view in the academic circle is that B7-H3 is a negative regulator molecule of T cells.

Based on the fact that B7-H3 can inhibit T cell activity and thus mediate tumor cells escape from immune surveillance, it is effective to block the binding of B7-H3 to unknown receptors to mediate T cell activation and inhibit tumor cell activity. For example, the existing clinical results of Enoblituzumab show that it has different degrees of remission for different tumors, and has a good curative effect, but there are still many patients with occurrences of disease progression. Therefore, there is still a large clinical unmet need for the simple development of monoclonal antibodies against B7-H3, and the existing clinical results show that its antitumor effect needs to be further improved.

Antibody-drug conjugate (ADC) is a new generation of antibody-targeted therapeutic drugs, which is mainly used in the treatment of cancer tumors. ADC drugs consist of three parts: a small molecule cytotoxic drug (Drug), an antibody (Antibody), and a linker (Linker) that links the antibody with the cytotoxic drug. The small molecule cytotoxic drug is bound to the antibody protein by chemical coupling. ADC drugs utilize antibodies to specifically recognize and guide small molecule drugs to cancer cell targets expressing cancer-specific antigens, and enter the cancer cells through endocytosis. The linker part is broken under the action of intracellular low pH value environment or lysosomal protease, releasing small molecular cytotoxic drugs, so as to achieve the effect of specifically killing cancer cells without damaging normal tissue cells. Therefore, ADC drugs have the characteristics of the targeted specificity of antibodies and the high toxicity of small molecule toxins to cancer cells at the same time, greatly expanding the effective therapeutic window of the drug. Clinical studies have proven that ADC drugs have high efficacy and are relatively stable in the blood, and can effectively reduce the toxicity of small molecule cytotoxic drugs (chemotherapy drugs) to the circulatory system and healthy tissues, and are currently a hot spot in the development of anticancer drugs internationally.

Yam B Poudel et al. (ACS MEDICINAL CHEMISTRY LETTERS, vol.11, No.11, 12 November 2020, pages 2190-2194) reports on the chemical modification of linkers to provide stable linker-payloads for the generation of antibody-drug conjugates.

CN104755494, WO2020031936 and WO2019039483 disclose B7-H3-targeting ADCs comprising exatecan.

### CONTENT OF THE PRESENT INVENTION

The technical problem to be solved by the present disclosure is to overcome the current deficiency of limited types of antibody-drug conjugates, and the present disclosure provides a B7-H3-targeting antibody-drug conjugate, a preparation method therefor, and a use thereof.

The antibody-drug conjugates provided by the present disclosure have good targeting ability, good inhibitory effect on tumor cells that are positive for B7-H3 expression, and good druggability and high safety. The antibody-drug conjugate has an inhibitory effect on B7-H3, and also has a good inhibitory effect on at least one of NCI-N87, A375, LN-229, PA-1, MDA-MB-468, Calu-6, and Hs-700T cells.

The present disclosure solves the above technical problem through the following technical solution.

The present disclosure provides an antibody-drug conjugate, a pharmaceutically acceptable salt thereof, a solvate thereof, or a solvate of the pharmaceutically acceptable salt thereof, wherein the antibody-drug conjugate has a structure shown in formula I; 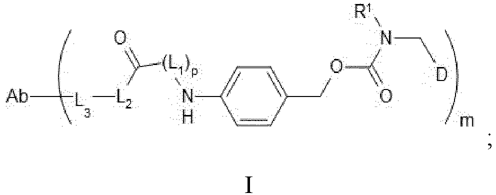
wherein Ab is a B7-H3 antibody or a variant of the B7-H3 antibody; m is 2 to 8;
the amino acid sequence of the light chain in the B7-H3 antibody is shown in SEQ ID NO: 1, and the amino acid sequence of the heavy chain is shown in SEQ ID NO: 2;
the variant of the B7-H3 antibody is at least 70%, 75%, 80%, 85%, 90%, 95%, 98%, or 99% identical to the B7-H3 antibody;
D is a cytotoxic drug topoisomerase inhibitor, wherein the cytotoxic drug topoisomerase inhibitor is 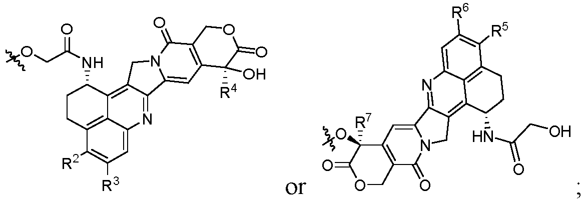 R² and R⁵ are each independently H, C₁-C₆ alkyl, or halogen; R³ and R⁶ are each independently H, C₁-C₆ alkyl, or halogen; R⁴ and R⁷ are each independently C₁-C₆ alkyl;
R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, C₃-C₁₀ cycloalkyl, C₆-C₁₄ aryl, or 5- to 14-membered heteroaryl; the heteroatom in the 5- to 14-membered heteroaryl is selected from one or more than one of N, O, and S, and the number of heteroatoms is 1, 2, 3, or 4; the R¹⁻¹, R¹⁻², and R¹⁻³ are each independently C₁-C₆ alkyl;
L₁ is independently one or more than one of a phenylalanine residue, alanine residue, glycine residue, glutamic acid residue, aspartic acid residue, cysteine residue, histidine residue, isoleucine residue, leucine residue, lysine residue, methionine residue, proline residue, serine residue, threonine residue, tryptophan residue, tyrosine residue, and valine residue; p is 2 to 4;
L₂ is 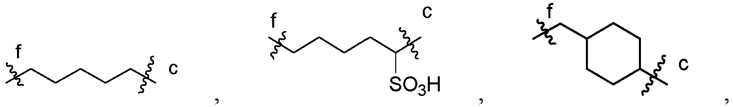 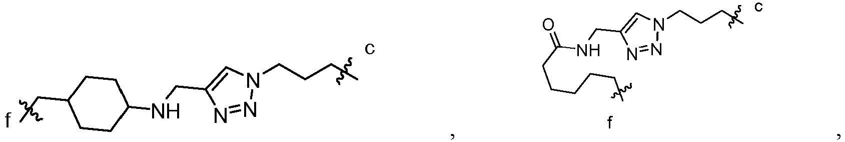 or 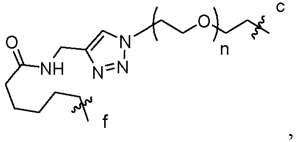 wherein n is independently 1 to 12, the c-terminal is connected to L₁ through a carbonyl group, and the f-terminal is connected to the d-terminal of L₃; 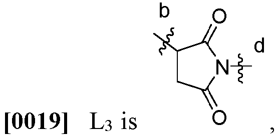 wherein the b-terminal is connected to the Ab, and the d-terminal is connected to the f-terminal of L₂.

In a preferred embodiment of the present disclosure, certain groups of the antibody-drug conjugate are defined as follows, and the definition of any unmentioned groups is as described in any of the above embodiments (this paragraph is hereinafter referred to as "in a preferred embodiment of the present disclosure"):
the b-terminal of L₃ is preferably connected to the sulfhydryl group on the antibody in the form of a thioether. Taking 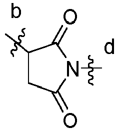 as an example, the connecting form of 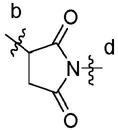 with the cysteine residue in the antibody is

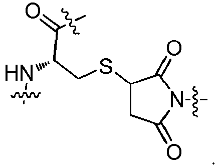

In a preferred embodiment of the present disclosure, when R² and R⁵ are each independently C₁-C₆ alkyl, the C₁-C₆ alkyl is preferably C₁-C₄ alkyl, further preferably methyl, ethyl, n-propyl, isopropyl, n-butyl, isobutyl, or tert-butyl, most preferably methyl.

In a preferred embodiment of the present disclosure, when R² and R⁵ are each independently halogen, the halogen is preferably fluorine, chlorine, bromine, or iodine, further preferably fluorine.

In a preferred embodiment of the present disclosure, when R³ and R⁶ are each independently C₁-C₆ alkyl, the C₁-C₆ alkyl is preferably C₁-C₄ alkyl, further preferably methyl, ethyl, n-propyl, isopropyl, n-butyl, isobutyl, or tert-butyl, most preferably methyl.

In a preferred embodiment of the present disclosure, when R³ and R⁶ are each independently halogen, the halogen is preferably fluorine, chlorine, bromine, or iodine, further preferably fluorine.

In a preferred embodiment of the present disclosure, when R⁴ and R⁷ are each independently C₁-C₆ alkyl, the C₁-C₆ alkyl is preferably C₁-C₄ alkyl, further preferably methyl, ethyl, n-propyl, isopropyl, n-butyl, isobutyl, or tert-butyl, most preferably ethyl.

In a preferred embodiment of the present disclosure, R² and R⁵ are each independently C₁-C₆ alkyl.

In a preferred embodiment of the present disclosure, R³ and R⁶ are each independently halogen.

In a preferred embodiment of the present disclosure, R⁴ and R⁷ are ethyl.

In a preferred embodiment of the present disclosure, D is 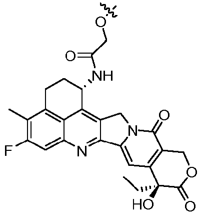 or

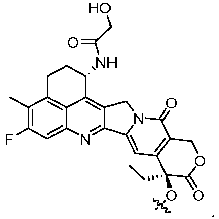

In a preferred embodiment of the present disclosure, when D is 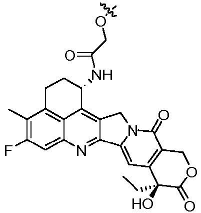 or 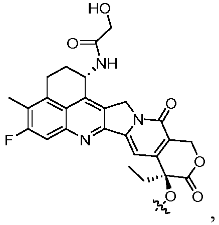 the antibody-drug conjugate can be 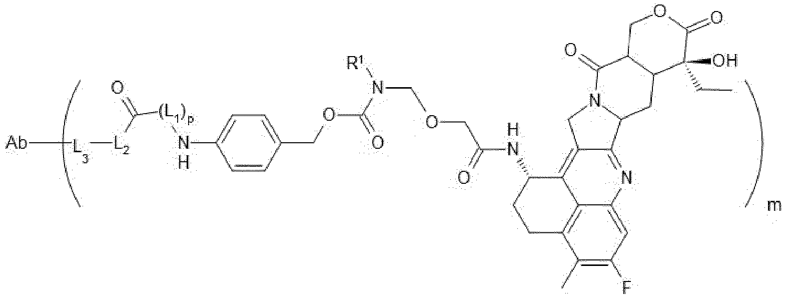 or

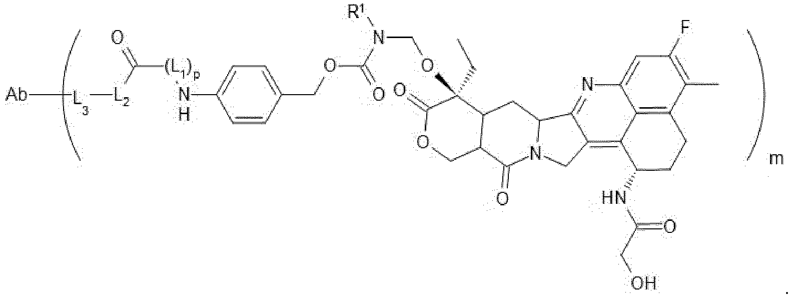

In a preferred embodiment of the present disclosure, when R¹ is C₁-C₆ alkyl substituted by one or more than one -NR¹⁻¹R¹⁻², the C₁-C₆ alkyl is preferably methyl, ethyl, n-propyl, isopropyl, n-butyl, isobutyl, or tert-butyl, more preferably ethyl.

In a preferred embodiment of the present disclosure, when R¹ is C₁-C₆ alkyl substituted by more than one -NR¹⁻¹R¹⁻², the "more than one" is two or three.

In a preferred embodiment of the present disclosure, when R¹⁻¹ and R¹⁻² are each independently C₁-C₆ alkyl, the C₁-C₆ alkyl is preferably methyl, ethyl, n-propyl, isopropyl, n-butyl, isobutyl, or tert-butyl, more preferably methyl.

In a preferred embodiment of the present disclosure, when R¹ is C₁-C₆ alkyl substituted by one or more than one -NR¹⁻¹R¹⁻², the -NR¹⁻¹R¹⁻² is preferably -N(CH₃)₂.

In a preferred embodiment of the present disclosure, when R¹ is C₁-C₆ alkyl substituted by one -NR¹⁻¹R¹⁻², the C₁-C₆ alkyl substituted by one -NR¹⁻¹R¹⁻² is preferably

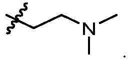

In a preferred embodiment of the present disclosure, when R¹ is C₁-C₆ alkyl substituted by one or more than one R¹⁻³S(O)₂-, the C₁-C₆ alkyl is preferably C₁-C₄ alkyl, further preferably methyl, ethyl, n-propyl, isopropyl, n-butyl, isobutyl, or tert-butyl, most preferably ethyl.

In a preferred embodiment of the present disclosure, when R¹ is C₁-C₆ alkyl substituted by more than one R¹⁻³S(O)₂-, the "more than one" is two or three.

In a preferred embodiment of the present disclosure, when R¹⁻³ is C₁-C₆ alkyl, the C₁-C₆ alkyl is preferably methyl, ethyl, n-propyl, isopropyl, n-butyl, isobutyl, or tert-butyl, more preferably methyl.

In a preferred embodiment of the present disclosure, when R¹ is C₁-C₆ alkyl substituted by one R¹⁻³S(O)₂-, the C₁-C₆ alkyl substituted by one R¹⁻³S(O)₂- is

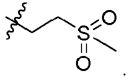

In a preferred embodiment of the present disclosure, when R¹ is C₁-C₆ alkyl, the C₁-C₆ alkyl is preferably methyl, ethyl, n-propyl, isopropyl, n-butyl, isobutyl, or tert-butyl, most preferably methyl.

In a preferred embodiment of the present disclosure, m is an integer (such as 2, 3, 4, 5, 6, 7, or 8) or non-integer, preferably 4 to 8, more preferably 6 to 8, further preferably 7 to 8, still more preferably 7.4 to 7.85, such as 7.47, 7.48, 7.52, 7.62, 7.64, 7.65, 7.67, 7.72, 7.78, 7.83, or 7.85.

In a preferred embodiment of the present disclosure, L₁ is preferably one or more than one of the phenylalanine residue, alanine residue, glycine residue, isoleucine residue, leucine residue, proline residue, and valine residue; more preferably one or more than one of the phenylalanine residue, alanine residue, glycine residue, and valine residue; further preferably the valine residue and/or the alanine residue; the "more than one" is preferably two or three; p is preferably 2.

In a preferred embodiment of the present disclosure, (L₁)ₚ is preferably 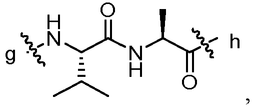 wherein the g-terminal is connected to the c-terminal of L₂ through a carbonyl group.

In a preferred embodiment of the present disclosure, n is preferably 8 to 12, such as 8, 9, 10, 11, or 12, further such as 8 or 12.

In a preferred embodiment of the present disclosure, when R¹⁻¹, R¹⁻², and R¹⁻³ are each independently and preferably C₁-C₆ alkyl, the C₁-C₆ alkyl is preferably C₁-C₄ alkyl, further preferably methyl, ethyl, n-propyl, isopropyl, n-butyl, isobutyl, or tert-butyl, most preferably methyl.

In a preferred embodiment of the present disclosure, R¹ is preferably C₁-C₆ alkyl substituted by one or more than one -NR¹⁻¹R¹⁻², C₁-C₆ alkyl substituted by one or more than one R¹⁻³S(O)₂-, C₁-C₆ alkyl, or C₃-C₁₀ cycloalkyl; more preferably C₁-C₆ alkyl substituted by one or more than one -NR¹⁻¹R¹⁻², C₁-C₆ alkyl substituted by one or more than one R¹⁻³S(O)₂-, or C₁-C₆ alkyl; further preferably C₁-C₆ alkyl substituted by one or more than one -NR¹⁻¹R¹⁻², or C₁-C₆ alkyl substituted by one or more than one R¹⁻³S(O)²-; most preferably C₁-C₆ alkyl substituted by one or more than one R¹⁻³S(O)²-.

In the present disclosure, when Ab is the B7-H3 antibody or the variant of the B7-H3 antibody, the B7-H3 antibody or the variant of the B7-H3 antibody is a residue of the B7-H3 antibody (a group formed by replacing a hydrogen on one of the sulfhydryl groups in B7-H3 antibody) or a residue of a variant of the B7-H3 antibody (a group formed by replacing a hydrogen on one of the sulfhydryl groups in the variant of the B7-H3 antibody).

In a preferred embodiment of the present disclosure, the compound of formula I is any one of the following schemes:
scheme I:
   Ab is the B7-H3 antibody or the variant of the B7-H3 antibody, preferably the B7-H3 antibody;
   D is 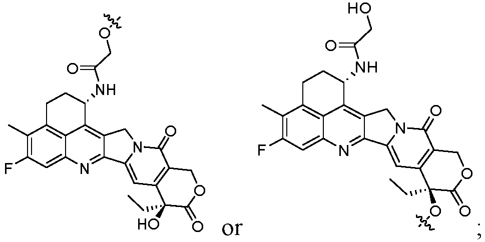
   R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, or C₃-C₁₀ cycloalkyl;
   L₁ is independently one or more than one of the phenylalanine residue, alanine residue, glycine residue, isoleucine residue, leucine residue, proline residue, and valine residue;
scheme II:
   Ab is the B7-H3 antibody or the variant of the B7-H3 antibody, preferably the B7-H3 antibody;
   D is 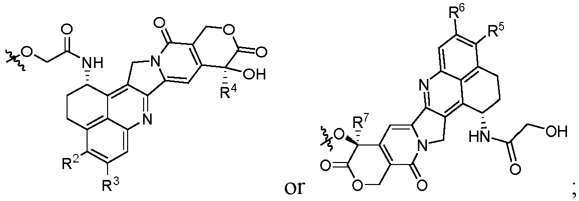 wherein R² and R⁵ are each independently C₁-C₆ alkyl; R³ and R⁶ are each independently halogen; D is preferably 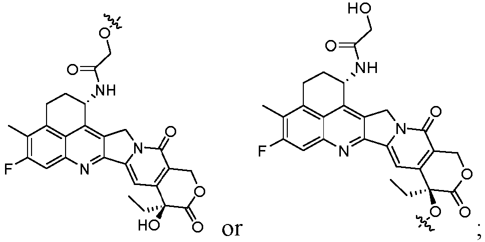
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
   L₁ is independently one or more than one of the phenylalanine residue, alanine residue, glycine residue, isoleucine residue, leucine residue, proline residue, and valine residue;
scheme III:
   Ab is the B7-H3 antibody or the variant of the B7-H3 antibody, preferably the B7-H3 antibody;
   D is 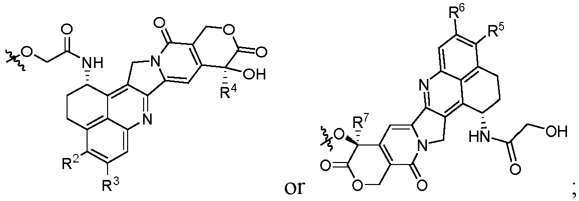 wherein R² and R⁵ are each independently C₁-C₆ alkyl; R³ and R⁶ are each independently halogen; D is preferably 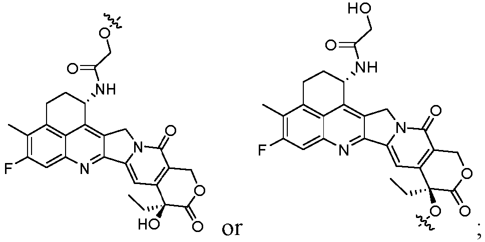
   m is 7 to 8;
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
   L₁ is independently the valine residue and/or the alanine residue;
scheme IV:
   Ab is the B7-H3 antibody or the variant of the B7-H3 antibody, preferably the B7-H3 antibody;
   D is 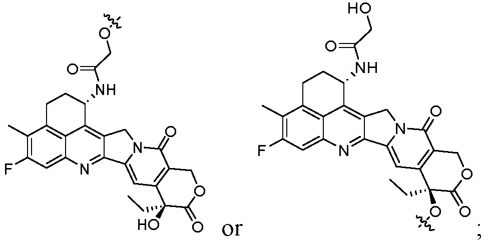
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
scheme V:
   Ab is the B7-H3 antibody or the variant of the B7-H3 antibody, preferably the B7-H3 antibody;
   D is 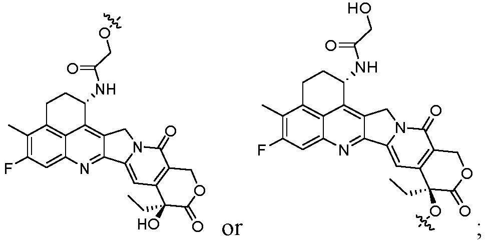
   m is 7 to 8;
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
   L₁ is independently the valine residue and/or the alanine residue.

In a preferred embodiment of the present disclosure, the antibody-drug conjugate is preferably 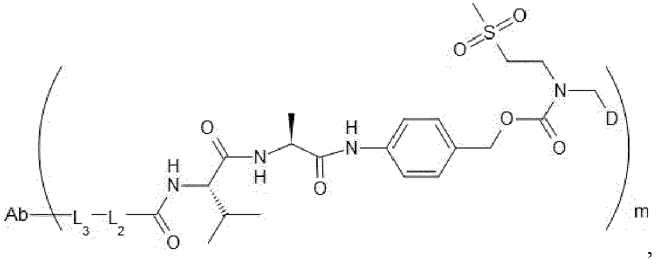 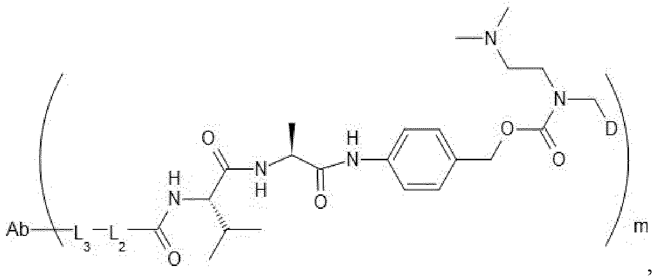 or

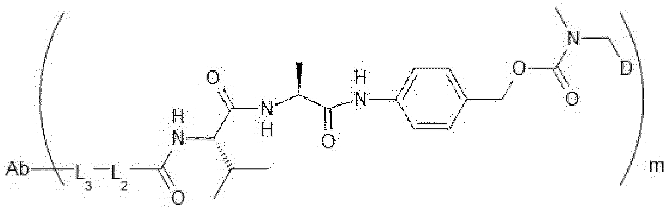

In a preferred embodiment of the present disclosure, L₂ is preferably

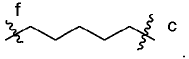

In a preferred embodiment of the present disclosure, Ab is the B7-H3 antibody; D is 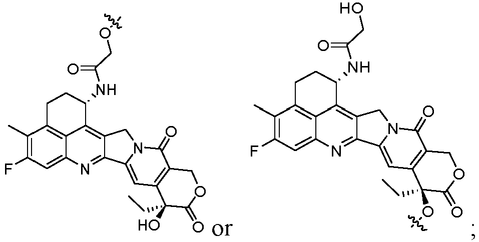 L₁ is the valine residue and/or the alanine residue, p is 2, (L₁)p is preferably 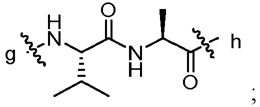 R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl, preferably C₁-C₆ alkyl substituted by one or more than one - NR¹⁻¹R¹⁻² or C₁-C₆ alkyl substituted by one or more than one R¹⁻³S(O)₂-, further preferably C₁-C₆ alkyl substituted by one or more than one R¹⁻³S(O)₂-; R¹⁻¹, R¹⁻², and R¹⁻³ are independently C₁-C₄ alkyl, preferably methyl; the C₁-C₆ alkyl substituted by one or more than one -NR¹⁻¹R¹⁻² is preferably 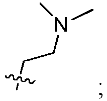 the C₁-C₆ alkyl substituted by one or more than one R¹⁻³S(O)₂- is preferably 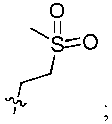 L₂ is 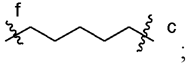 L₃ is

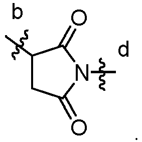

In a preferred embodiment of the present disclosure, the antibody-drug conjugate is preferably any one of the following compounds: 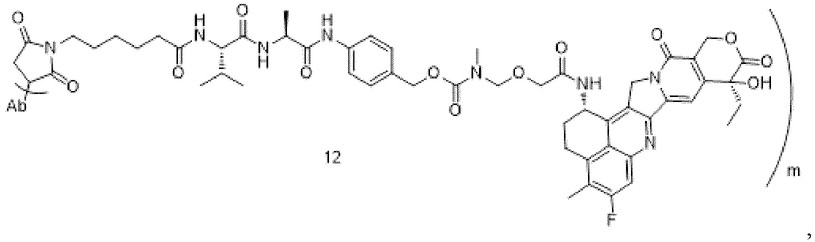 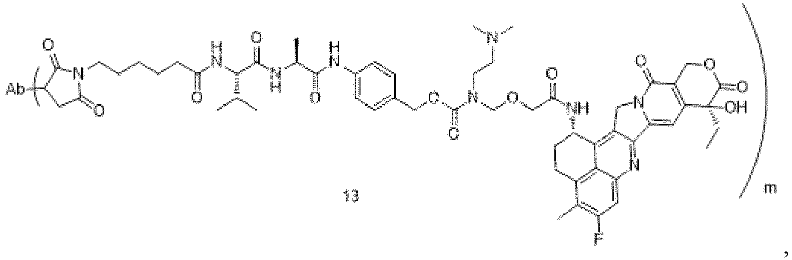 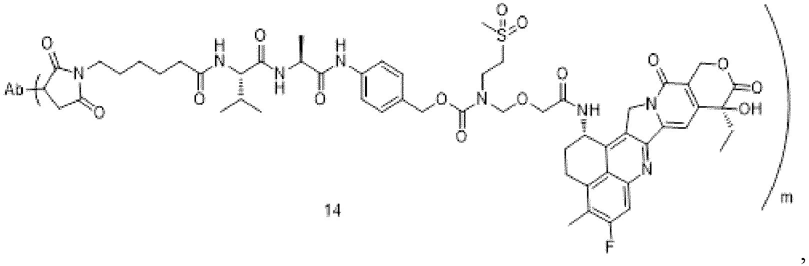 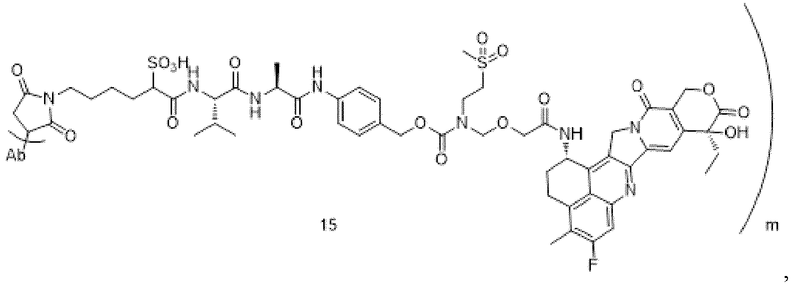 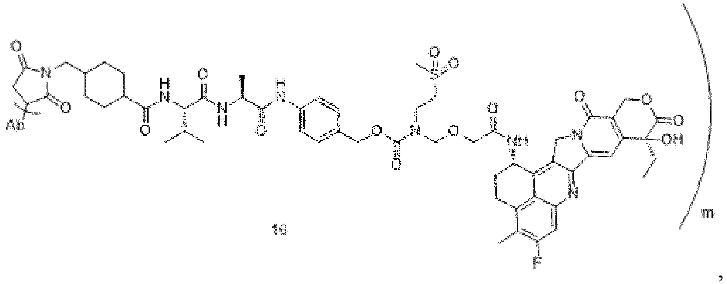 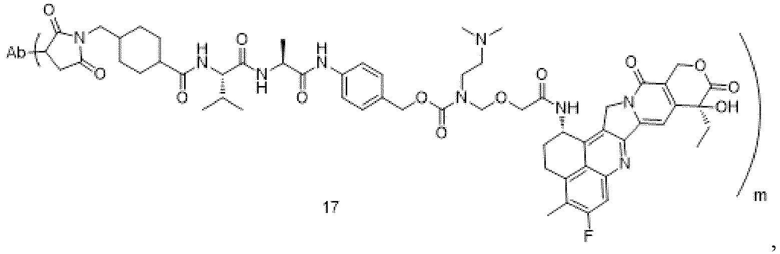 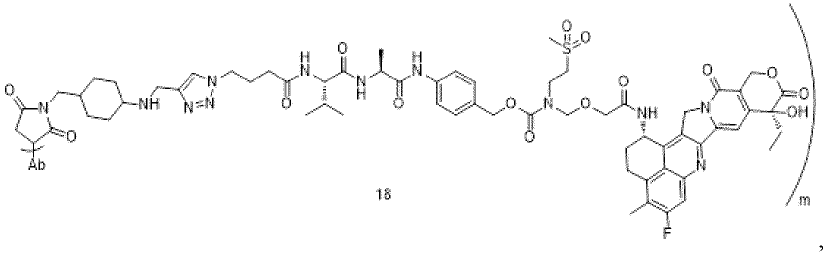 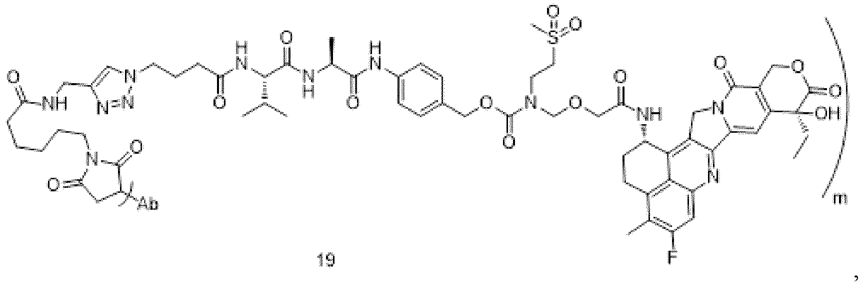 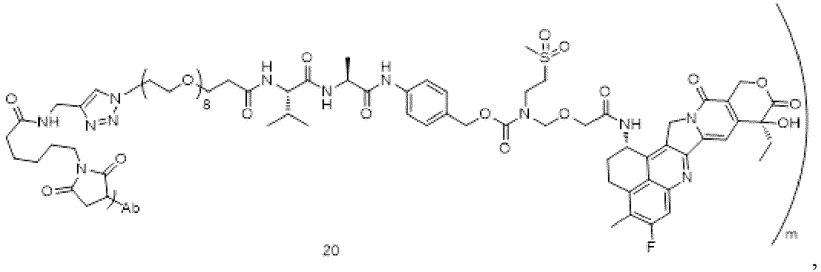 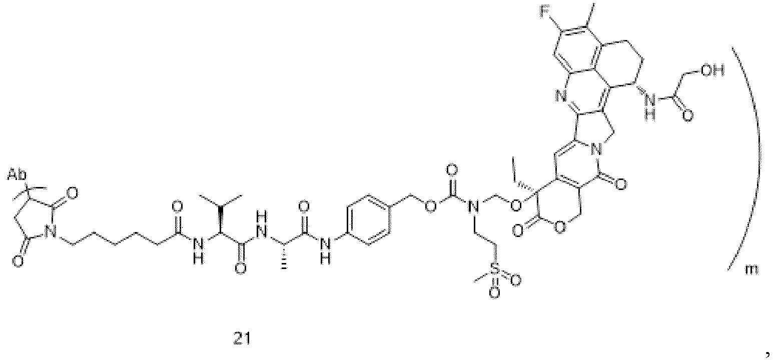 or 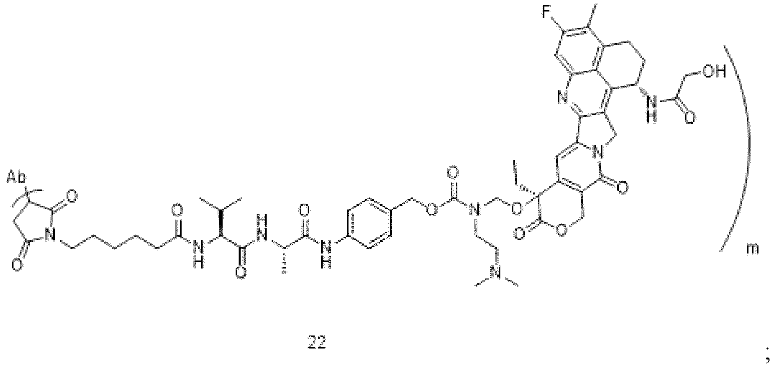 wherein, Ab is the B7-H3 antibody or the variant of the B7-H3 antibody, and m is 7.47, 7.48, 7.52, 7.62, 7.64, 7.65, 7.67, 7.72, 7.78, 7.83, or 7.85.

In a preferred embodiment of the present disclosure, the antibody-drug conjugate is preferably any one of the following compounds: 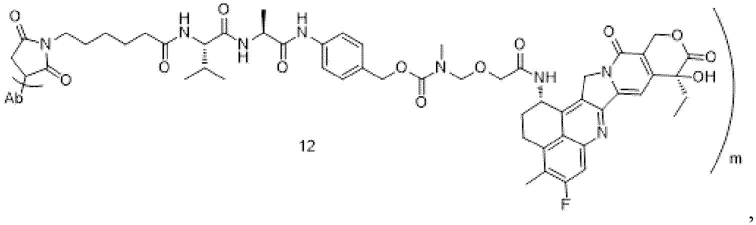
Ab is the B7-H3 antibody, and m is preferably 7.64; 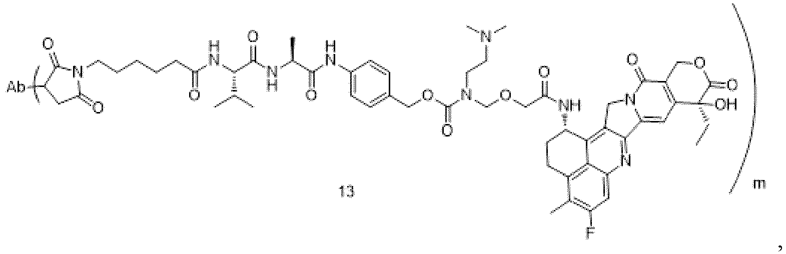
Ab is the B7-H3 antibody, and m is preferably 7.67; 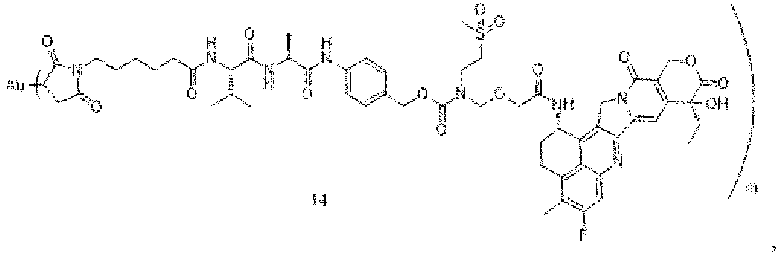
Ab is the B7-H3 antibody, and m is preferably 7.83; 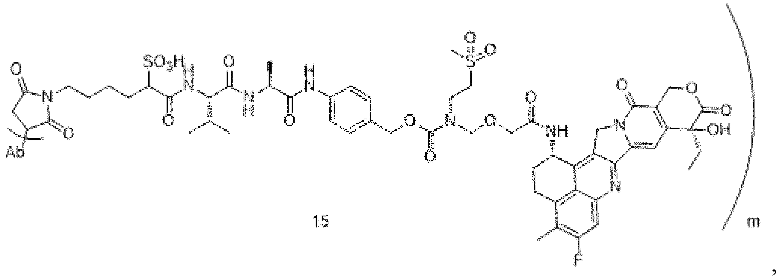
Ab is the B7-H3 antibody, and m is preferably 7.65; 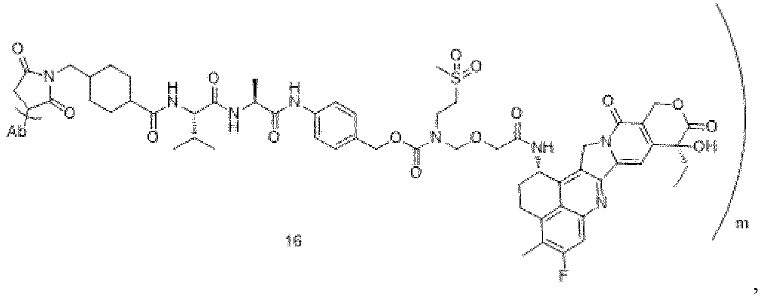
Ab is the B7-H3 antibody, and m is preferably 7.78; 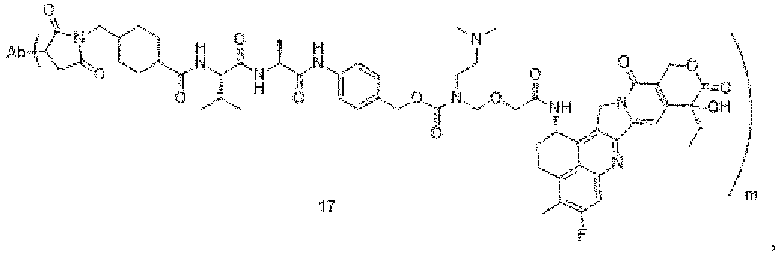
Ab is the B7-H3 antibody, and m is preferably 7.62; 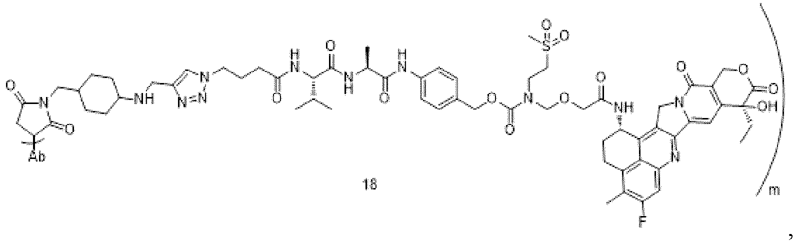
Ab is the B7-H3 antibody, and m is preferably 7.48; 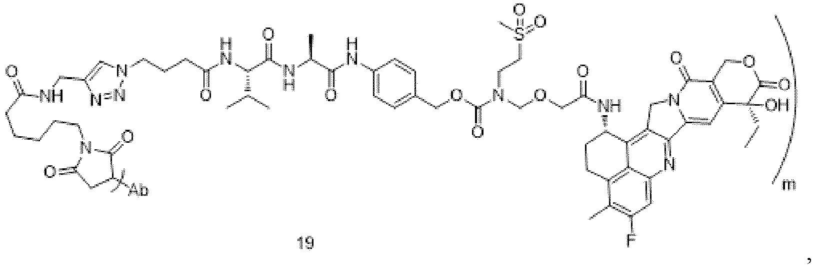
Ab is the B7-H3 antibody, and m is preferably 7.52; 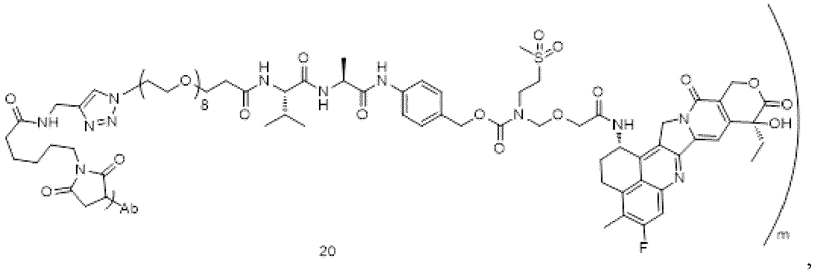
Ab is the B7-H3 antibody, and m is preferably 7.47; 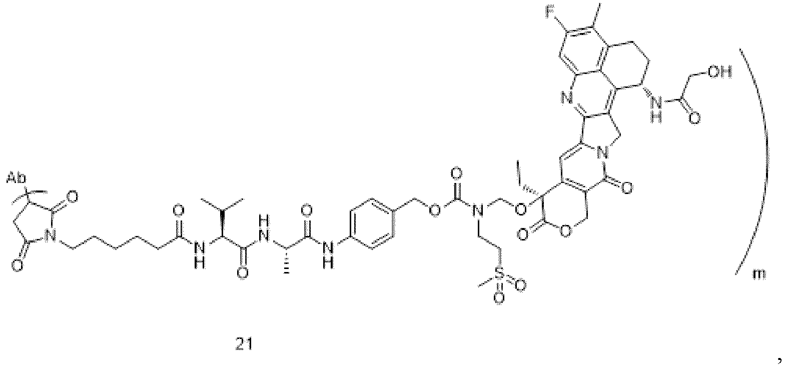
Ab is the B7-H3 antibody, and m is preferably 7.85; or 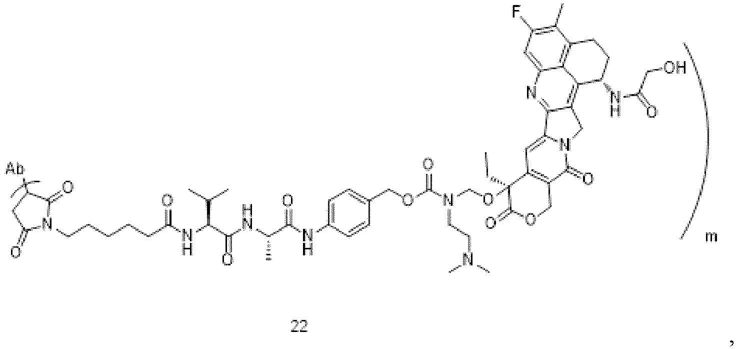
Ab is the B7-H3 antibody, and m is preferably 7.72.

The present disclosure also provides a method for preparing the antibody-drug conjugate, comprising the following step: carrying out a coupling reaction between a compound of formula II and Ab-hydrogen as shown below; 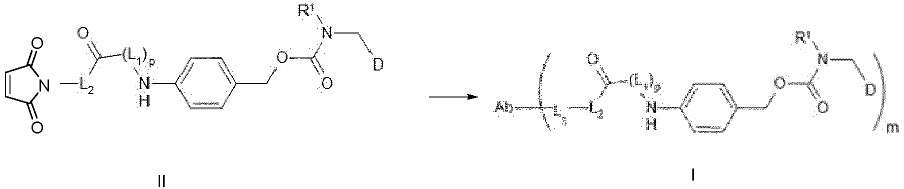 wherein L₁, L₂, L₃, R¹, p, and Ab are defined as above.

In the present disclosure, the conditions and operations of the coupling reaction can be conventional conditions and operations for the coupling reaction in the art.

The present disclosure also provides a pharmaceutical composition, comprising substance X and a pharmaceutically acceptable excipient, wherein the substance X is the antibody-drug conjugate, the pharmaceutically acceptable salt thereof, the solvate thereof, or the solvate of the pharmaceutically acceptable salt thereof.

In the pharmaceutical composition, the substance X may be used in a therapeutically effective amount.

The present disclosure also provides substance X for use in inhibiting B7-H3 protein.

The present disclosure also provides substance X for use in treating and/or preventing a tumor, and the tumor is preferably a B7-H3 positive tumor. The B7-H3 positive tumor is preferably one or more than one of B7-H3 positive lung cancer, ovarian cancer, melanoma, pancreatic cancer, breast cancer, brain glioma, prostate cancer, and gastric cancer.

In some embodiments of the present disclosure, for the lung cancer, the lung cancer cells are *NCI-1703* cells or Calu-6 cells;
in some embodiments of the present disclosure, for the ovarian cancer, the ovarian cancer cells are PA-1 cells;
in some embodiments of the present disclosure, for the melanoma, the melanoma cells are A375 cells;
in some embodiments of the present disclosure, for the pancreatic cancer, the pancreatic cancer cells are Hs-700T cells;
in some embodiments of the present disclosure, for the breast cancer, the breast cancer cells are MDA-MB-468 cells;
in some embodiments of the present disclosure, for the brain glioma, the brain glioma cells are LN-229 cells;
in some embodiments of the present disclosure, for the gastric cancer, the gastric cancer cells are NCI-N87 cells.

Unless otherwise indicated, the following terms appearing in the specification and claims of the present disclosure have the following meanings:
The pharmaceutical excipient may be an excipient widely used in the field of pharmaceutical production. The excipient is mainly used to provide a safe, stable, and functional pharmaceutical composition, and can also provide a method for the subject to dissolve the active ingredient at a desired rate after administration, or to facilitate effective absorption of the active ingredient after the subject receives administration of the composition. The pharmaceutical excipient can be an inert filler or provide a certain function, such as stabilizing the overall pH value of the composition or preventing degradation of the active ingredient in the composition. The pharmaceutical excipients may include one or more of the following excipients: buffers, chelating agents, preservatives, solubilizers, stabilizers, vehicles, surfactants, colorants, flavoring agents and sweeteners.

The term "pharmaceutically acceptable" refers to salts, solvents, excipients and the like that are generally non-toxic, safe, and suitable for patient use. The "patient" is preferably a mammal, more preferably a human being.

The term "pharmaceutically acceptable salt" refers to salt prepared from the compound of the present disclosure and a relatively non-toxic and pharmaceutically acceptable acid or alkali. When the compound of the present disclosure contains relatively acidic functional groups, an alkali addition salt can be obtained by contacting a sufficient amount of pharmaceutically acceptable alkali with the neutral form of the compound in a pure solution or an appropriate inert solvent. The pharmaceutically acceptable alkali addition salt includes, but is not limited to, lithium salt, sodium salt, potassium salt, calcium salt, aluminium salt, magnesium salt, zinc salt, bismuth salt, ammonium salt, and diethanolamine salt. When the compound of the present disclosure contains relatively alkaline functional groups, an acid addition salt can be obtained by contacting a sufficient amount of pharmaceutically acceptable acid with the neutral form of the compound in a pure solution or an appropriate inert solvent. The pharmaceutically acceptable acid includes inorganic acid, and the inorganic acid includes, but is not limited to, hydrochloric acid, hydrobromic acid, hydroiodic acid, nitric acid, carbonic acid, phosphoric acid, phosphorous acid, sulfuric acid, etc. The pharmaceutically acceptable acid includes organic acid, and the organic acid includes, but is not limited to, acetic acid, propionic acid, oxalic acid, isobutyric acid, maleic acid, malonic acid, benzoic acid, succinic acid, suberic acid, trans-butenedioic acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)), amino acid (e.g., glutamic acid and arginine), etc. When the compound of the present disclosure contains relatively acidic functional groups and relatively alkaline functional groups, it can be converted into an alkali addition salt or an acid addition salt. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining the compound of the present disclosure with a stoichiometric or non-stoichiometric amount of solvent. Solvent molecules in the solvate can exist in an ordered or non-ordered arrangements. The solvent includes, but is not limited to, water, methanol, ethanol, etc.

Natural or natural sequence B7-H3 can be isolated from nature or produced by recombinant DNA technology, chemical synthesis, or a combination of the above and similar techniques.

Antibody is interpreted in the broadest sense here, which can specifically bind to the target through at least one antigen recognition region located in the variable region of the immunoglobulin molecule, such as carbohydrate, polynucleotide, fat, polypeptide, etc. Specifically, it includes complete monoclonal antibodies, polyclonal antibodies, bispecific antibodies, and antibody fragments, as long as they have the required biological activity. Variants of antibodies of the present disclosure refer to amino acid sequence variants, and covalent derivatives of natural polypeptides, provided that the biological activity equivalent to that of natural polypeptides is retained. The difference between amino acid sequence mutants and natural amino acid sequences is generally that one or more amino acids in the natural amino acid sequence are substituted or one or more amino acids are deleted and/or inserted in the polypeptide sequence. Deletion mutants include fragments of natural polypeptides and N-terminal and/or C-terminal truncation mutants. The amino acid sequence variant is at least 70% (e.g., 70%, 75%, 80%, 85%, 90%, 95%, 98%, or 99%) identical to the natural sequence.

The antibodies of the present disclosure can be prepared using techniques well known in the art, such as hybridoma methods, recombinant DNA techniques, phage display techniques, synthetic techniques, or combinations thereof, or other techniques known in the art.

Description of the term "drug-antibody ratio"(DAR). L-D is a reactive group with the conjugation site on the antibody, L is a linker, D is a cytotoxic agent further coupled to the antibody connected to L. In the present disclosure, D is Dxd. The number of DAR of each antibody finally coupled to D is represented by m or m can also represent the number of D coupled to a single antibody. In some embodiments, m is actually an average value between 2 and 8, 4 and 8, or 6 and 8, or m is an integer of 2, 3, 4, 5, 6, 7, or 8; in some embodiments, m is an average value of 2, 4, 6, or 8; in other embodiments, m is an average value of 2, 3, 4, 5, 6, 7, or 8.

The linker refers to the direct or indirect connection between the antibody and the drug. The linker can be connected to the mAb in many ways, such as through surface lysine, reductive coupling to oxidized carbohydrates, and through cysteine residues released by reducing interchain disulfide bonds. Many ADC connection systems are known in the art, including connections based on hydrazones, disulfides, and peptides.

The term "treatment" or its equivalent expression, when used for, for example, cancer, refers to a procedure or process for reducing or eliminating the number of cancer cells in a patient or alleviating the symptoms of cancer. The "treatment" of cancer or another proliferative disorder does not necessarily mean that the cancer cells or other disorders will actually be eliminated, but the number of cells or disorders will actually be reduced or the symptoms of cancers or other disorders will actually be alleviated. Generally, the method of treating cancer will be carried out even if it has only a low probability of success, but it is still considered to induce an overall beneficial course of action considering the patient's medical history and estimated survival expectation.

The term "prevention" refers to a reduced risk of acquiring or developing a disease or disorder.

The term "cycloalkyl" refers to a saturated cyclic hydrocarbon group with three to twenty carbon atoms (e.g., C₃-C₆ cycloalkyl), including monocyclic cycloalkyl. The cycloalkyl contains 3 to 20 carbon atoms, preferably 3 to 10 carbon atoms, and more preferably 3 to 6 carbon atoms. Examples of cycloalkyl include, but are not limited to, cyclopropyl, cyclobutyl, cyclopentyl, cyclohexyl, cycloheptyl, cyclooctyl, cyclononyl, and cyclodecyl.

The term "alkyl" refers to a straight or branched chain alkyl group with a specified number of carbon atoms. Examples of alkyl groups include methyl, ethyl, n-propyl, isopropyl, n-butyl, t-butyl, isobutyl, sec-butyl, n-pentyl, isopentyl, n-hexyl, n-heptyl, n-octyl, and similar alkyl groups.

The term "halogen" refers to fluorine, chlorine, bromine, or iodine.

The term "heteroaryl" refers to an aryl group (or aromatic ring) containing 1, 2, 3, or 4 heteroatoms independently selected from N, O, and S, which may be a monocyclic aromatic system, such as furyl, pyridinyl, pyridazinyl, pyrimidinyl, pyrazinyl, thiophenyl, isoxazolyl, oxazolyl, diazolyl, imidazolyl, pyrrolyl, pyrazolyl, triazolyl, tetrazolyl, thiazolyl, and isothiazolyl.

The term "aryl" refers to any stable monocyclic or bicyclic carbocycle, wherein all rings are aromatic rings. Examples of the aryl moiety include phenyl or naphthyl.

The above preferred conditions can be combined arbitrarily to obtain preferred embodiments of the present disclosure without violating common knowledge in the art.

Unless otherwise specified, room temperature in the present disclosure refers to 20 to 30°C.

The reagents and raw materials used in the present disclosure are all commercially available.

The positive progressive effects of the present disclosure are as follows:
1. The antibody-drug conjugate containing B7-H3 antibody provided by the present disclosure has good targeting ability and has a good inhibitory effect on various tumor cells expressing B7-H3, etc.
2. In vivo studies have shown that the antibody-drug conjugate of the present disclosure has better in vitro cytotoxicity and in vivo antitumor activity.
3. The antibody-drug conjugate of the present disclosure has good solubility and good druggability. There is no abnormal phenomenon such as precipitation during the coupling preparation process, which is very conducive to the preparation of the antibody-drug conjugate.

### BRIEF DESCRIPTION OF THE DRAWINGS

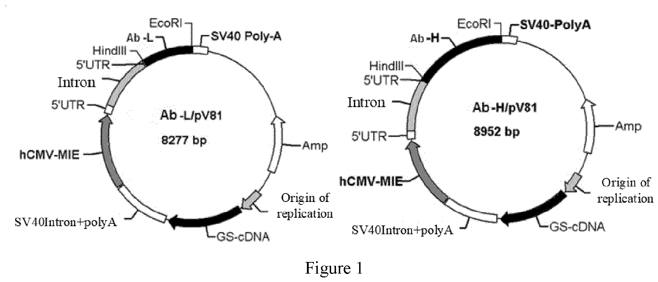

Figure 1 shows the construction of expression vectors for the light and heavy chains of an FDA016 antibody; wherein Ab-L is the light chain of the antibody, and Ab-H is the heavy chain of the antibody.

### DETAILED DESCRIPTION OF THE PREFERRED EMBODIMENT

The present disclosure will be further described below with reference to examples, but the present disclosure is not therefore limited to the scope of the examples. Experimental methods without specific conditions in the following examples are selected according to conventional methods and conditions, or according to the commercial specification.

### Description of abbreviations:

- PCR: polymerase chain reaction
- CHO: Chinese hamster ovary cells
- HTRF: homogeneous time-resolved fluorescence
- PB: phosphate buffer
- EDTA: ethylenediaminetetraacetic acid
- TECP: tris(2-carboxyethyl)phosphine
- DMSO: dimethyl sulfoxide
- DMF: N,N-dimethylformamide
- HATU: 2-(7-azabenzotriazol-1-yl)-N,N,N',N'-tetramethyluronium hexafluorophosphate
- v/v: V/V, volume ratio
- UV: ultraviolet visible light
- ELISA: enzyme-linked immunosorbent assay
- BSA: bovine serum albumin
- rpm: revolutions per minute
- FBS: fetal bovine serum

### Example 1: Preparation of B7-H3 antibody

In the present disclosure, the monoclonal antibody FDA016 with high affinity and specific targeting B7-H3 was selected, the amino acid sequence of its light chain was shown in SEQ ID NO: 1, and the amino acid sequence of its heavy chain was shown in SEQ ID NO: 2. The light and heavy chain nucleotide sequences of FDA016 were obtained by whole gene synthesis (Suzhou Genewiz). They were separately constructed into the pV81 vector (as shown in Figure 1) by double digestion with EcoR I and Hind III (purchased from TAKARA), and then transformed into Trans 1-T1 competent cells (purchased from Beijing TransGen Biotech, product number: CD501-03) by ligation, which were picked for cloning, PCR identification and sent for sequencing confirmation. Positive clones were cultured and expanded for plasmid extraction, thus obtaining the antibody light chain eukaryotic expression plasmid FDA016-L/pV81 and the antibody heavy chain eukaryotic expression plasmid FDA016-H/pV81. These two plasmids were linearized by digestion with XbaI (purchased from Takara, product number: 1093S). The light and heavy chain eukaryotic expression plasmids were transformed into CHO cells adapted to suspension growth (purchased from ATCC) at a ratio of 1.5/1 by electroporation. After electroporation, the cells were seeded at 2000 to 5000 cells/well in a 96-well plate. After 3 weeks of culture, the expression level was measured by HTRF method (homogeneous time-resolved fluorescence). The top ten cell pools in terms of expression level were selected for expansion and cryopreservation. A cell was revived into a 125 mL shake flask (culture volume of 30 mL) and cultured at 37°C, 5.0% CO₂, and 130 rpm by vibration for 3 days, then expanded to a 1000 mL shake flask (culture volume of 300 mL) and cultured at 37°C, 5.0% CO₂, and 130 rpm by vibration. Starting on the 4th day, 5 to 8% of the initial culture volume of replenishment culture medium was added every other day. The culture was ended on 10th to 12th day and the harvest liquid was centrifuged at 9500 rpm for 15 minutes to remove the cell precipitate. The supernatant was collected and filtered through a 0.22 µm filter membrane. The treated sample was purified using a MabSelect affinity chromatography column (purchased from GE) to obtain antibody FDA016.

The amino acid sequence of the FDA016 light chain is shown below:

SEQ ID NO: 1:

1 DIQMTQSPSSLSASVGDRVTITCRASESIYSYLAWYQQKPGKAPKLLVYN

51 TKTLPEGVPSRFSGSGSGTDFTLTISSLQPEDFATYYCQHHYGTPPWTFG

101 QGTRLEIKRTVAAPSVFIFPPSDEQLKSGTASVVCLLNNFYPREAKVQWK

151 VDNALQSGNSQESVTEQDSKDSTYSLSSTLTLSKADYEKHKVYACEVTHQ

201 GLSSPVTKSFNRGEC

The amino acid sequence of FDA016 heavy chain is shown below:

SEQ ID NO: 2:

1 EVQLVESGGGLVKPGGSLRLSCAASGFTFSSYGMSWVRQAPGKGLEWVAT

51 INSGGSNTYYPDSLKGRFTISRDNAKNSLYLQMNSLRAEDTAVYYCARHD

101 GGAMDYWGQGTTVTVSSASTKGPSVFPLAPSSKSTSGGTAALGCLVKDYF

151 PEPVTVSWNSGALTSGVHTFPAVLQSSGLYSLSSVVTVPSSSLGTQTYIC

201 NVNHKPSNTKVDKRVEPKSCDKTHTCPPCPAPELLGGPSVFLFPPKPKDT

251 LMISRTPEVTCVVVDVSHEDPEVKFNWYVDGVEVHNAKTKPREEQYNSTY

301 RVVSVLTVLHQDWLNGKEYKCKVSNKALPAPIEKTISKAKGQPREPQVYT

351 LPPSREEMTKNQVSLTCLVKGFYPSDIAVEWESNGQPENNYKTTPPVLDS

401 DGSFFLYSKLTVDKSRWQQGNVFSCSVMHEALHNHYTQKSLSLSPGK

### Example 2: Synthesis of linker-drug conjugates

### Example 2-1: Synthesis of LE12

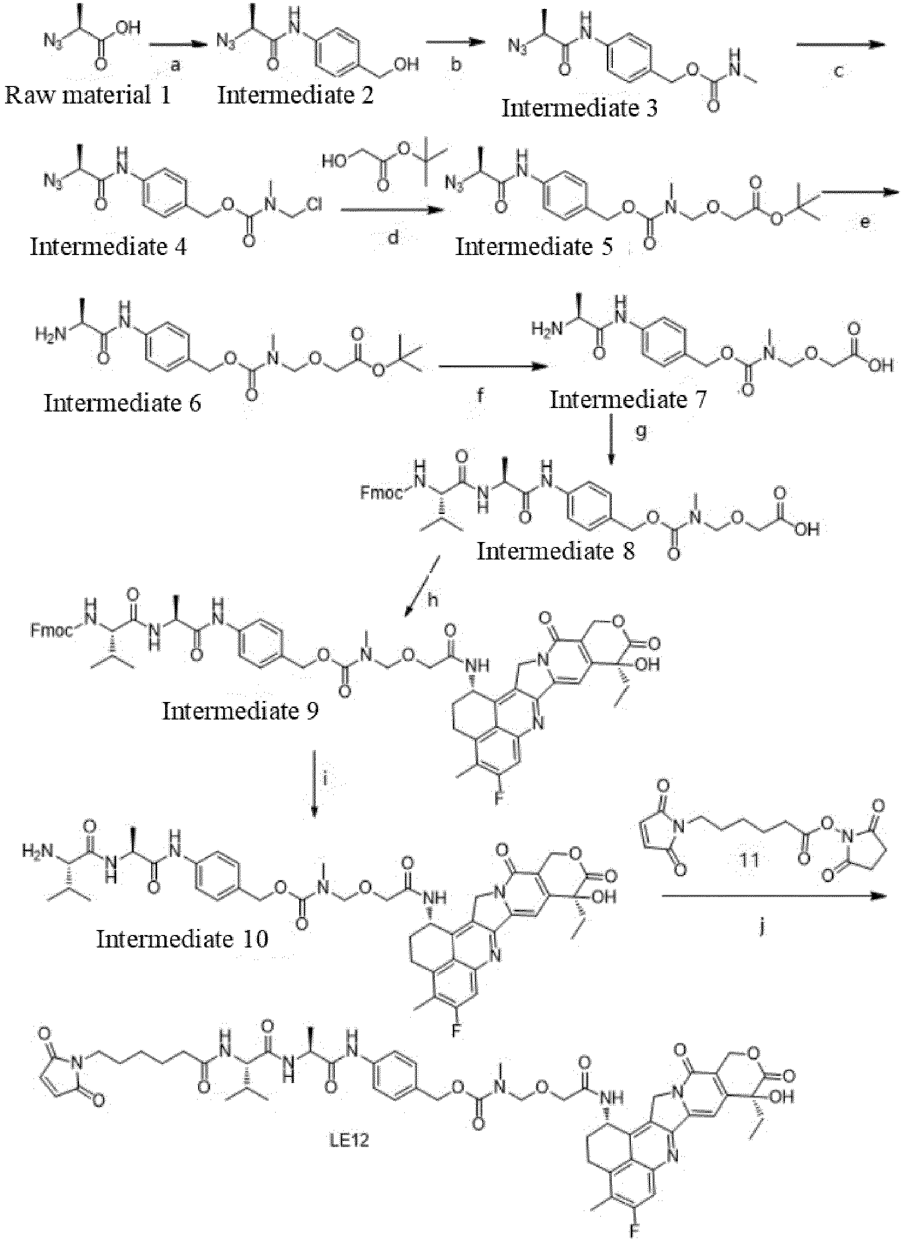

### Synthesis of Intermediate 2:

(S)-2-Azidopropionic acid (10 g, 86.9 mmol) and 4-aminobenzyl alcohol (21.40 g, 173.8 mmol) were dissolved in a mixed solvent of 300 mL of dichloromethane and methanol (in a volume ratio of 2:1), and then 2-ethoxy-1-ethoxycarbonyl-1,2-dihydroquinoline (21.49 g, 86.9 mmol) was added thereto. The reaction was carried out at room temperature for 5 hours. The solvent was then evaporated under reduced pressure and the obtained crude product was purified by silica gel column chromatography [dichloromethane: ethyl acetate = 1:1 (v/v)] to obtain intermediate 2 (16.3 g, yield of 85%), ESI-MS m/z: 221 (M+H).

### Synthesis of Intermediate 3:

Intermediate 2 (15 g, 68.2 mmol) was mixed with bis(p-nitrophenyl)carbonate (22.82 g, 75.02 mmol) and dissolved in 200 mL of anhydrous N,N-dimethylformamide, and then 25 mL of triethylamine was added thereto, and the reaction was carried out at room temperature for 2 hours. After the complete reaction of the raw materials was monitored by liquid chromatography-mass spectrometry, methylamine hydrochloride (6.91 g, 102.3 mmol) was added thereto, and the reaction was continued at room temperature for 1 hour. After the reaction was completed, most of the solvent was removed by distillation under reduced pressure, then 200 mL of water and 200 mL of ethyl acetate were added thereto. The organic phase was collected after the phases were separated, and the organic phase was dried and concentrated, and then the obtained crude product was purified by silica gel column chromatography [dichloromethane: ethyl acetate = 10:1 (v/v)] to obtain intermediate 3 (18.9 g, yield of 100%), ESI-MS m/z: 278 (M+H).

### Synthesis of Intermediate 5:

Intermediate 3 (10 g, 36.1 mmol) was mixed with polyformaldehyde (1.63 g, 54.2 mmol) and dissolved in 150 mL of anhydrous dichloromethane. Trimethylchlorosilane (6.28 g, 57.76 mmol) was slowly added thereto and the reaction was carried out at room temperature for 2 hours to obtain a crude solution of intermediate 4. The reaction was monitored by liquid chromatography-mass spectrometry after sampling and quenching with methanol. After the reaction was completed, the reaction mixture was filtered and then tert-butyl hydroxyacetate (9.54 g, 72.2 mmol) and triethylamine (10 mL, 72.2 mmol) were added to the filtrate and the reaction was continued at room temperature for 2 hours. After the reaction was completed, most of the solvent was removed by distillation under reduced pressure, and then the obtained crude product was purified by silica gel column chromatography [petroleum ether: ethyl acetate = 3:1 (v/v)] to obtain intermediate 5 (11.2 g, yield of 74%), ESI-MS m/z: 422 (M+H).

### Synthesis of Intermediate 6:

Intermediate 5 (10 g, 23.8 mmol) was dissolved in 80 mL of anhydrous tetrahydrofuran, and 80 mL of water was added thereto, and then tris(2-carboxyethylphosphine) hydrochloride (13.6 g, 47.6 mmol) was added thereto and the reaction was carried out for 4 hours at room temperature. After the reaction was completed, the tetrahydrofuran was removed by distillation under reduced pressure, and then the mixture was extracted with ethyl acetate. The obtained organic phase was dried and evaporated to remove the solvent under reduced pressure, and purified by silica gel column chromatography [dichloromethane: methanol = 10:1 (v/v)] to obtain intermediate 6 (8.1 g, yield of 86%), ESI-MS m/z: 396 (M+H).

### Synthesis of Intermediate 8:

Intermediate 6 (5 g, 12.7 mmol) was dissolved in 60 mL of a mixed solvent of dichloromethane and methanol (v/v = 2:1), and 3 mL of trifluoroacetic acid was slowly added thereto, and the reaction was carried out at room temperature for 30 min. After the reaction was completed, an equal volume of water and ethyl acetate were added thereto, and the organic phase was dried and concentrated, and the obtained crude product was directly used in the next step.

The crude product obtained from the previous step was dissolved in 50 mL of anhydrous N,N-dimethylformamide, and then Fmoc-L-valine hydroxysuccinimide ester (8.3 g, 19.1 mmol) and triethylamine (5 mL) were added thereto, and the reaction was carried out at room temperature for 2 hours. After the reaction was completed, most of the solvent was removed by distillation under reduced pressure, and then the obtained crude product was purified by silica gel column chromatography [dichloromethane: methanol = 10:1 (v/v)] to obtain intermediate 8 (5.4 g, yield of 64%), ESI-MS m/z: 661 (M+H).

### Synthesis of Intermediate 9:

Intermediate 8 (1 g, 1.5 mmol) was mixed with Exatecan methanesulfonate (0.568 g, 1 mmol) in 30 mL of anhydrous N,N-dimethylformamide, and then 2-(7-azabenzotriazol-1-yl)-N,N,N',N'-tetramethyluronium hexafluorophosphate (1.14 g, 3.0 mmol) and 2 mL of triethylamine were added thereto, and the reaction was carried out at room temperature for 2 hours. After the reaction was completed, the solvent was removed by distillation under reduced pressure, and then the obtained crude product was purified by silica gel column chromatography [chloroform: methanol = 10:1 (v/v)] to obtain intermediate 9 (0.94 g, yield of 87%), ESI-MS m/z: 1078 (M+H).

### Synthesis of Compound LE12:

Intermediate 9 (1 g, 0.929 mmol) was dissolved in 20 mL of anhydrous DMF, then 0.5 mL of 1,8-diazabicyclo[5.4.0]undec-7-ene was added thereto, and the reaction was carried out at room temperature for 1 hour. After the reaction of the raw materials was completed, N-succinimidyl 6-maleimidohexanoate (428.5 mg, 1.39 mmol) was added directly, and the reaction mixture was stirred at room temperature for 1 hour. The solvent was removed by distillation under reduced pressure, and then the obtained crude product was purified by silica gel column chromatography [chloroform: methanol = 8:1 (v/v)] to obtain the title compound (0.7 g, yield of 73%), ESI-MS m/z: 1035 (M+H).

### Example 2-2: Synthesis of Compound LE13

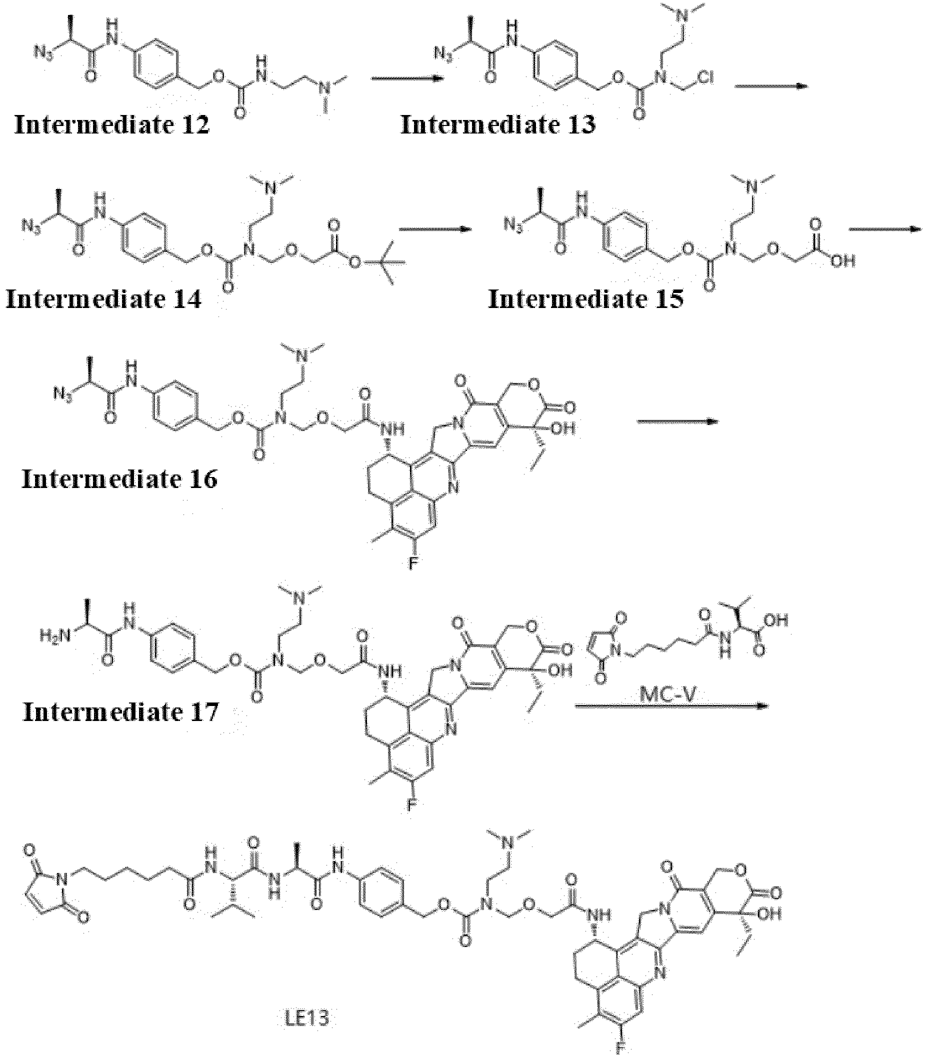

### Synthesis of Intermediate 14

Commercially available intermediate 12 (267 mg, 0.8 mmol) was mixed with paraformaldehyde (50 mg, 1.6 mmol) and dissolved in 20 mL of anhydrous dichloromethane. Then, trimethylchlorosilane (0.3 mL, 3.4 mmol) was added slowly. After the addition was completed, the reaction was carried out at room temperature for 2 hours. Then, the reaction was monitored by liquid chromatography-mass spectrometry after sampling and quenching with methanol. After the reaction was completed, the reaction mixture was filtered, and then tert-butyl 2-hydroxyacetate (211 mg, 1.6 mmol) and pempidine (0.5 mL) were added to the filtrate, and the reaction was continued at room temperature for about 2 hours. After the reaction was completed, most of the solvent was removed by distillation under reduced pressure, and the obtained crude product was purified by silica gel column chromatography [dichloromethane: methanol = 20:1 (v/v)] to obtain intermediate 14 (260 mg, yield of 68%), ESI-MS m/z: 479 (M+H).

### Synthesis of Intermediate 15

Intermediate 14 (238 mg, 0.50 mmol) was dissolved in 6 mL of a mixed solvent of dichloromethane and methanol (v/v = 2:1), and 0.3 mL of trifluoroacetic acid was slowly added thereto, and the reaction was carried out at room temperature for 30 min. After the reaction was completed, an equal volume of water and ethyl acetate were added thereto, and the organic phase was dried and concentrated, and the obtained crude product was directly used in the next step.

### Synthesis of Intermediate 16

The crude product obtained from the previous step was mixed with Exatecan methanesulfonate (170 mg, 0.30 mmol) in 5 mL of anhydrous N,N-dimethylformamide, and then 2-(7-azabenzotriazol-1-yl)-N,N,N',N'-tetramethyluronium hexafluorophosphate (341 mg, 0.90 mmol) and 0.60 mL of triethylamine were added thereto, and the reaction was carried out at room temperature for 2 hours. After the reaction was completed, the solvent was removed by distillation under reduced pressure, and then the obtained crude product was purified by silica gel column chromatography [chloroform: methanol = 10:1 (v/v)] to obtain intermediate 16 (210 mg, 83%), ESI-MS m/z: 840 (M+H).

### Synthesis of Intermediate 17

Intermediate 16 (100 mg, 0.12 mmol) was dissolved in 15 mL of anhydrous tetrahydrofuran, and 3 mL of water was added thereto, then 0.3 mL of 1 mol/L triethylphosphine aqueous solution was added thereto, and the reaction was carried out at room temperature for 4 hours. After the reaction was monitored to be completed, the reaction mixture was distilled under reduced pressure to remove tetrahydrofuran. Sodium bicarbonate was added to the remaining aqueous solution to adjust the pH to neutral, and then dichloromethane was added for extraction. The obtained organic phase was dried and evaporated under reduced pressure to remove the solvent. The obtained crude product was purified by silica gel column chromatography [dichloromethane: methanol = 10:1 (v/v)] to obtain intermediate 17 (69 mg, yield of 71%), ESI-MS m/z: 814 (M+H).

### Synthesis of Compound LE13

Intermediate 17 (120 mg, 0.15 mmol) obtained according to the previous synthesis method was mixed with the commercially available raw material MC-V(102 mg, 0.33 mmol) in 40 mL of dichloromethane, and the condensation agent 2-ethoxy-1-ethoxycarbonyl-1,2-dihydroquinoline (82 mg, 0.33 mmol) was added to react overnight at room temperature. After the reaction was completed, the solvent was evaporated under reduced pressure and the obtained crude product was purified by silica gel column chromatography [dichloromethane: methanol = 10:1 (v/v)] to obtain compound LE13 (116 mg, yield of 70%), ESI-MS m/z: 1106.5 (M+H).

### Example 2-3: Synthesis of Compound LE14

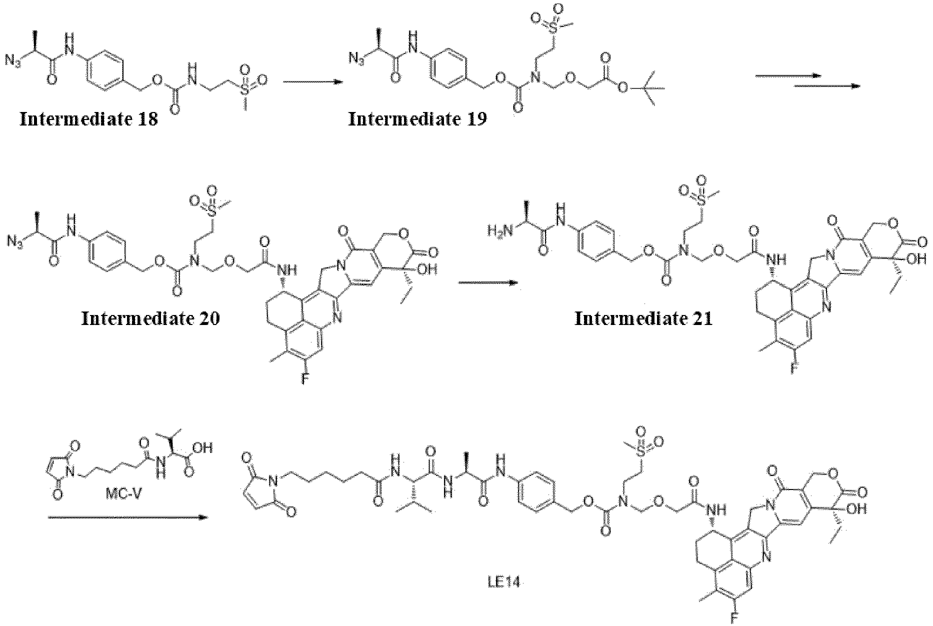

### Synthesis of Intermediate 19

Commercially available intermediate 18 (300 mg, 0.8 mmol) was mixed with polyformaldehyde (50 mg, 1.6 mmol) and dissolved in 20 mL anhydrous dichloromethane. Then, trimethylchlorosilane (0.3 mL, 3.4 mmol) was slowly added thereto, and the reaction was carried out at room temperature for 2 hours. The reaction was monitored by liquid chromatography-mass spectrometry after sampling and quenching with methanol. After the reaction was completed, the reaction mixture was filtered and then tert-butyl 2-hydroxyacetate (211 mg, 1.6 mmol) and triethylamine (0.22 m, 1.6 mmol) were added to the filtrate. The reaction was continued at room temperature for about 2 hours. After the reaction was completed, most of the solvent was removed by distillation under reduced pressure and the obtained crude product was purified by silica gel column chromatography [dichloromethane: methanol = 20:1 (v/v)] to obtain intermediate 19 (349 mg, yield of 85%), ESI-MS m/z: 514 (M+H), ¹H NMR (400 MHz, CDCl₃) δ 8.13 (s, 1H), 7.56 (d, J = 7.5 Hz, 2H), 7.35 (s, 2H), 5.14 (s, 2H), 4.91 (s, 2H), 4.25 (q, J = 7.1 Hz, 1H), 3.99 (d, J = 42.5 Hz, 2H), 3.85 (t, J = 6.2 Hz, 2H), 3.40 (dd, J = 18.5, 7.6 Hz, 2H), 2.89 (d, J = 48.6 Hz, 3H), 1.65 (d, J = 6.8 Hz, 3H), 1.46 (s, 9H).

### Synthesis of Intermediate 20

Intermediate 19 (257 mg, 0.50 mmol) was dissolved in 6 mL of a mixed solvent of dichloromethane and methanol (v/v = 2:1), and 0.3 mL of trifluoroacetic acid was slowly added thereto, and the reaction was carried out at room temperature for 30 min. After the reaction was completed, an equal volume of water and ethyl acetate were added thereto, and the organic phase was dried and concentrated, and the obtained crude product was directly used in the next step.

The obtained crude product was mixed with Exatecan methanesulfonate (170 mg, 0.30 mmol) in 5 mL of anhydrous N,N-dimethylformamide, and then 2-(7-azabenzotriazol-1-yl)-N,N,N',N'-tetramethyluronium hexafluorophosphate (341 mg, 0.90 mmol) and 0.60 mL of triethylamine were added thereto, and the reaction was carried out at room temperature for 2 hours. After the reaction was completed, the solvent was removed by distillation under reduced pressure, and then the obtained crude product was purified by silica gel column chromatography [dichloromethane: methanol = 20:1 (v/v)] to obtain intermediate 20 (212 mg, yield of 81%), ESI-MS m/z: 875 (M+H). ¹H NMR (400 MHz, CDCl₃) δ 8.27 (d, J = 34.7 Hz, 1H), 7.63 - 7.35 (m, 5H), 7.21 - 7.10 (m, 1H), 5.71 - 5.48 (m, 2H), 5.24 - 4.95 (m, 3H), 4.95 - 4.72 (m, 4H), 4.45 (s, 1H), 4.33 - 3.97 (m, 3H), 3.75 (s, 2H), 3.39 - 2.99 (m, 4H), 2.76 (d, J = 15.3 Hz, 3H), 2.43 - 2.15 (m, 5H), 2.04 (s, 1H), 1.94 - 1.75 (m, 2H), 1.62 (d, J = 6.6 Hz, 3H), 1.11 - 0.89 (m, 3H).

### Synthesis of Intermediate 21

Intermediate 20 (77 mg, 0.09 mmol) was dissolved in 12 mL of anhydrous tetrahydrofuran, and 3 mL of water was added thereto, then 0.3 mL of 1 mol/L triethylphosphine aqueous solution was added thereto, and the reaction was carried out at room temperature for 4 hours. After the reaction was completed, the reaction mixture was distilled under reduced pressure to remove tetrahydrofuran. Sodium bicarbonate was added to the remaining aqueous solution to adjust the pH to neutral, and then dichloromethane was added for extraction. The obtained organic phase was dried and evaporated under reduced pressure to remove the solvent. The obtained crude product was purified by silica gel column chromatography [dichloromethane: methanol = 10:1 (v/v)] to obtain intermediate 21 (53 mg, yield of 69%), ESI-MS m/z: 849 (M+H). ¹H NMR (400 MHz, DMSO) δ 8.52 (s, 1H), 7.79 (d, J = 10.8 Hz, 1H), 7.67 - 7.55 (m, 2H), 7.47 - 7.21 (m, 3H), 6.51 (s, 1H), 5.60 (s, 1H), 5.52 - 5.32 (m, 2H), 5.30 - 5.11 (m, 2H), 5.11 - 4.94 (m, 2H), 4.94 - 4.74 (m, 2H), 4.02 (s, 2H), 3.81 - 3.66 (m, 2H), 3.60 - 3.35 (m, 4H), 3.24 - 3.08 (m, 2H), 2.94 (d, J = 30.8 Hz, 3H), 2.39 (s, 3H), 2.28 - 2.04 (m,2H), 2.00 - 1.73 (m, 2H), 1.22 (d, J = 6.6 Hz, 3H), 0.96 - 0.70 (m, 3H).

### Synthesis of Compound LE14

Intermediate 21 (134 mg, 0.16 mmol) was mixed with the commercially available raw material MC-V (102 mg, 0.33 mmol) in 40 mL of dichloromethane, and the condensation agent 2-ethoxy-1-ethoxycarbonyl-1,2-dihydroquinoline (82 mg, 0.33 mmol) was added to react overnight at room temperature. After the reaction was completed, the solvent was evaporated under reduced pressure and the obtained crude product was purified by silica gel column chromatography [dichloromethane: methanol=10:1 (v/v)] to obtain compound LE14 (137 mg, yield of 75%), ESI-MS m/z: 1141.4 (M+H). ¹H NMR (400 MHz, DMSO) δ 9.97 (s, 1H), 8.52 (s, 1H), 8.27 - 8.09 (m, 1H), 7.88 - 7.70 (m, 2H), 7.63 - 7.51 (m, 2H), 7.28 (s, 3H), 6.99 (s, 2H), 6.51 (s, 1H), 5.59 (s, 1H), 5.50 - 5.32 (m, 2H), 5.17 (s, 2H), 4.98 (s, 2H), 4.85 (d, J = 17.3 Hz, 2H), 4.43 - 4.33 (m, 1H), 4.21 - 4.12 (m, 1H), 4.03 (s, 2H), 3.74 - 3.64 (m, 2H), 3.20 - 3.03 (m, 3H), 3.02 - 2.84 (m, 4H), 2.36 (s, 3H), 2.23 - 2.09 (m, 4H), 2.01 - 1.90 (m, 1H), 1.90 - 1.78 (m, 2H), 1.55 - 1.39 (m, 4H), 1.30 (d, J = 6.7 Hz, 3H), 1.23 - 1.11 (m, 2H), 0.93 - 0.77 (m, 9H).

### Example 2-4: Synthesis of Compound LE15-LE20

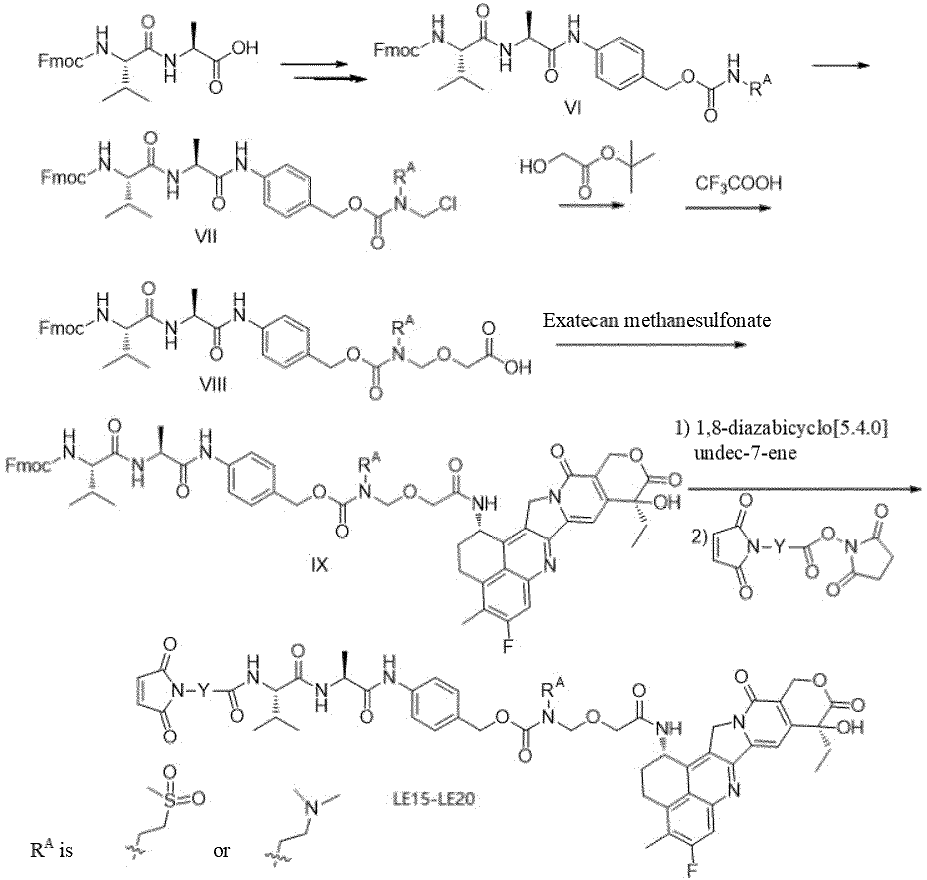

Intermediate VI could be prepared by replacing the methylamine hydrochloride in step b with the corresponding commercially available amino compound, using Fmoc-L-valyl-L-alanine as the starting material and referring to steps a and b in the synthesis method of intermediate 3 in Example 2-1. The subsequent steps were carried out starting from intermediate VI, and with the same methods as those in steps c, d, f, and h of Example 2-1, and intermediate IX similar to intermediate 9 was obtained. Then, following the same steps i and j as Example 6, the amino protecting group was removed, and condensed with different commercially available maleimide compounds to obtain the final product. The structures of the amino compounds and maleimides used are shown in Table 1. Compound LE15: graywhite solid, ESI-MS m/z: 1121.2 (M+H); compound LE16: light yellow solid, ESI-MS m/z: 1167.1 (M+H); compound LE17: yellow solid, ESI-MS m/z: 1132.3 (M+H); compound LE18: light yellow solid, ESI-MS m/z: 1305.4 (M+H); compound LE19: light yellow solid, ESI-MS m/z: 1307.4 (M+H); compound LE20: light yellow solid, ESI-MS m/z: 1337.6 (M+H).

**Table 1. Intermediates used for the synthesis of LE15 to LE20**

| Product | R^{A} | Amino Compound | Maleimide Structure | 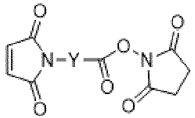 |
|---|---|---|---|---|
| LE15 | 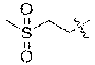 | Methylsulfonyl ethylamine hydrochloride | 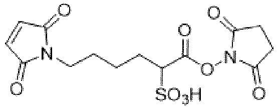 | |
| LE16 | 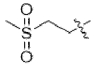 | Methylsulfonyl ethylamine hydrochloride | 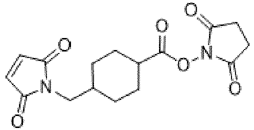 | |
| LE17 | 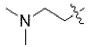 | Dimethylethylamine hydrochloride | 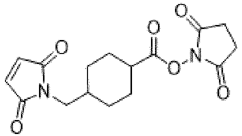 | |
| LE18 | 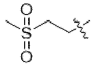 | Methylsulfonyl ethylamine hydrochloride | 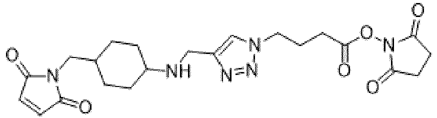 | |
| LE19 | 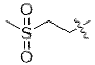 | Methylsulfonyl ethylamine hydrochloride | 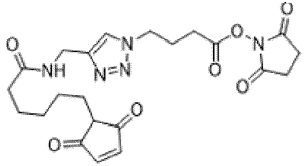 | |
| LE20 | 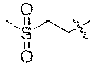 | Methylsulfonyl ethylamine hydrochloride | 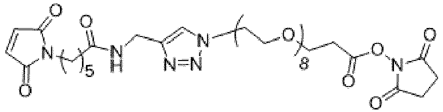 | |

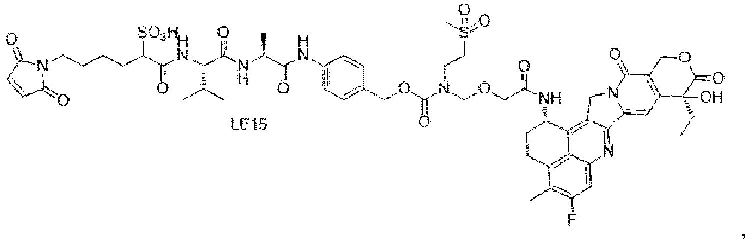

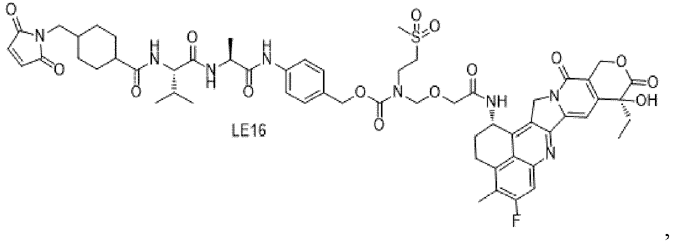

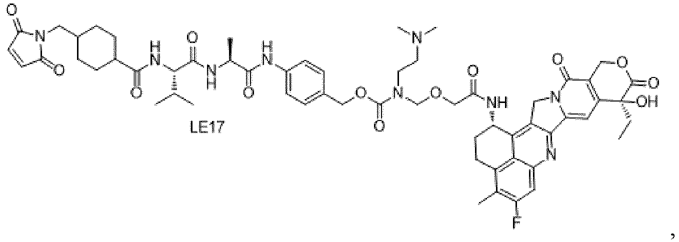

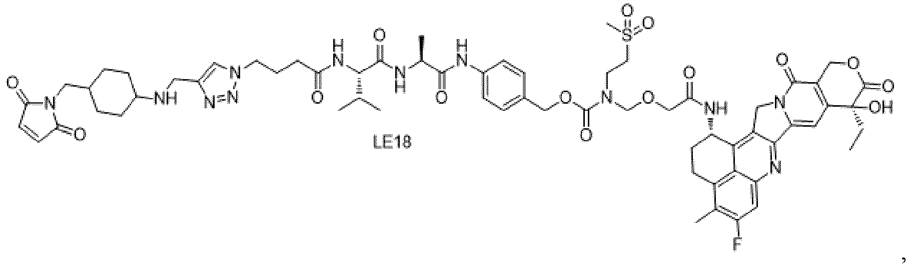

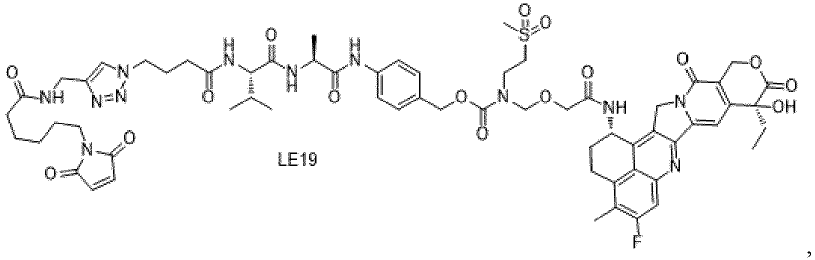

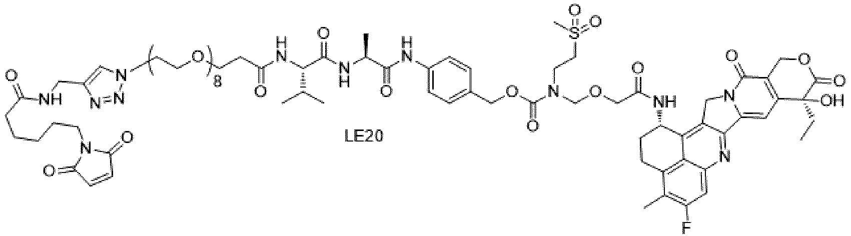

### Example 2-5: Synthesis of Compounds LE21 and LE22

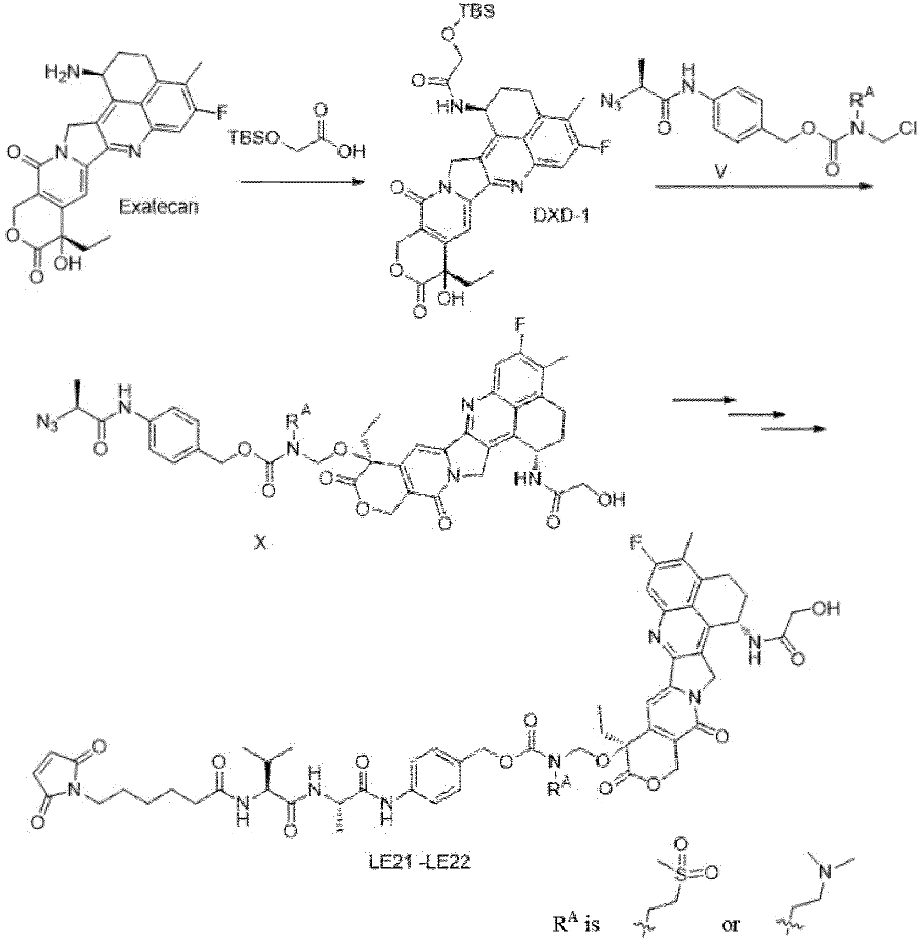

### Synthesis of Compound DXD-1

Commercially available Exatecan methanesulfonate (0.568 g, 1 mmol) was mixed with commercially available 2-(tert-butyldimethylsilyloxy)acetic acid (CAS: 105459-05-0, 0.38 g, 2 mmol) in 20 mL anhydrous dichloromethane. The condensation agent HATU (0.76 g, 2 mmol) and 1 mL of pyridine were added thereto and stirred at room temperature for 2 hours. After the reaction was completed, the solvent was evaporated to dryness under reduced pressure, and the obtained crude product was purified by column chromatography [dichloromethane: methanol = 50:1 (v/v)] to obtain title compound DXD-1 (0.55 g, yield of 90%), ESI-MS m/z: 608.1 (M+H). ¹H NMR (400 MHz, CDCl₃) δ 7.73 (d, J = 10.5 Hz, 1H), 7.64 (s, 1H), 7.05 (d, J = 9.2 Hz, 1H), 5.80 - 5.62 (m, 2H), 5.41 - 5.14 (m, 4H), 4.29 - 4.15 (m, 2H), 4.08-4.03 (m, 1H), 3.27 - 3.07 (m, 2H), 2.45 (s, 3H), 2.38 - 2.28 (m, 2H), 1.96 - 1.81 (m, 2H), 1.04 (t, J = 7.4 Hz, 3H), 0.80 (s, 9H), 0.11 (s, 3H), 0.03 (s, 3H).

### Preparation of Intermediate V

Intermediate V could be prepared by replacing the methylamine hydrochloride in step b with the corresponding commercially available amino compound, referring to the preparation method of compound 4 in Example 2-1.

### Synthesis of LE21 to LE22

Intermediate V was reacted with DXD-1, and then treated with 10% trifluoroacetic acid/dichloromethane solution to obtain intermediate X. Then, intermediate X was reacted according to the subsequent steps e, g, i, and j of compound 5 in Example 2-1: Intermediate X was reduced to obtain an amino compound, and the amino compound was then condensed with Fmoc-L-valine hydroxysuccinimide ester. Then the Fmoc protecting group of the amino group was removed from the obtained product, and the obtained amino product was then reacted with N-succinimidyl 6-maleimidohexanoate to obtain the final product. Compound LE21: yellow solid, ESI-MS m/z: 1141.2 (M+H); compound LE22: yellow solid, ESI-MS m/z: 1106.6 (M+H).

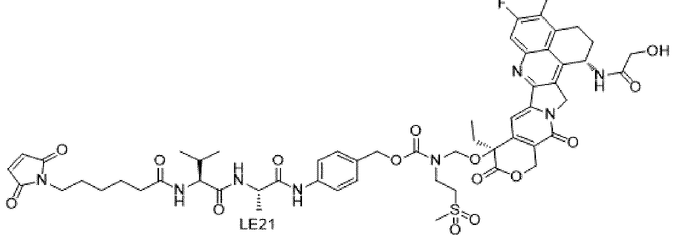

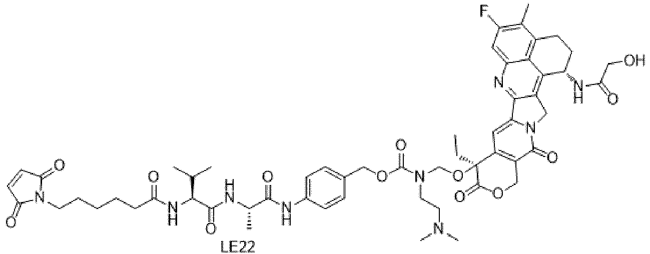

### Example 2-6: Synthesis of Compound LS13 (comparative example)

Referring to the synthesis method of LE15 in Examples 2 to 4, SN-38 (7-ethyl-10-hydroxycamptothecin) was reacted with intermediate VII (R¹ is methylsulfonyl ethyl) to obtain compound LS13 after deprotection, condensation and other steps: ¹H NMR (400 MHz, DMSO) δ 9.92 (d, *J* = 22.4 Hz, 1H), 8.14 (s, 1H), 8.08 (d, *J* = 9.1 Hz, 1H), 7.81 (d, *J =* 8.0 Hz, 1H), 7.70 - 7.50 (m, 3H), 7.47 (d, *J* = 7.2 Hz, 1H), 7.34 (d, *J* = 7.2 Hz, 1H), 7.27 (s, 1H), 7.20 (s, 1H), 6.98 (s, 2H), 6.51 (s, 1H), 5.61 (s, 2H), 5.48 - 5.35 (m, 2H), 5.27 (s, 2H), 5.10 (d, *J* = 20.6 Hz, 2H), 4.36 (s, 1H), 4.21 - 4.07 (m, 1H), 3.84 (s, 2H), 3.48 (s, 2H), 3.21 - 2.92 (m, 6H), 2.25 - 2.04 (m, 2H), 2.04 - 1.78 (m, 3H), 1.55 - 1.36 (m, 4H), 1.36 - 1.10 (m, 9H), 0.95 - 0.71 (m, 10H).

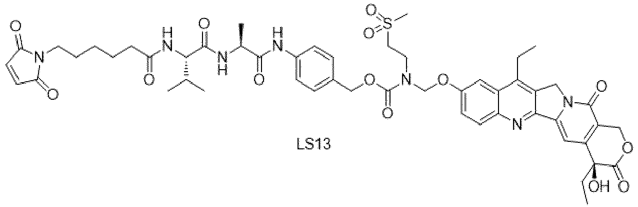

### Example 2-7: Synthesis of Compound GGFG-Dxd (comparative example)

Compound GGFG-Dxd was prepared according to the known synthesis method reported in WO2015146132A1. ESI-MS m/z: 1034.5 (M+H), ¹H-NMR (400 MHz, DMSO-*d₆*) δ 8.61 (t, *J =* 6.4 Hz, 1H), 8.50 (d, *J =* 8.5 Hz, 1H), 8.28 (t, *J =* 5.1 Hz, 1H), 8.11 (d, *J =* 7.5 Hz, 1H), 8.05 (t, *J* = 5.7 Hz, 1H), 7.99 (t, *J* = 5.9 Hz, 1H), 7.77 (d, *J* = 11.0 Hz, 1H), 7.31 (s, 1H), 7.25 - 7.16 (m, 5H), 6.98 (s, 2H), 6.51 (s, 1H), 5.59 (dt, *J =* 7.4, 4.1 Hz, 1H), 5.41 (s, 2H), 5.20 (s, 2H), 4.64 (d, *J* = 6.1 Hz, 2H), 4.53 - 4.40 (m, 1H), 4.02 (s, 2H), 3.74 - 3.37 (m, 8H), 3.18 - 3.00 (m, 2H), 3.04 - 2.97 (m, 1H), 2.77 (dd, *J* = 13.5, 9.4 Hz, 1H), 2.38 (s, 3H), 2.19 (dd, *J=* 14.9, 8.5 Hz, 2H), 2.11 - 2.05 (m, 2H), 1.86 (dd, *J* = 14.0, 6.7 Hz, 2H), 1.45 (s, 4H), 1.20 - 1.14 (m, 2H), 0.87 (t, *J* = 7.1 Hz, 3H).

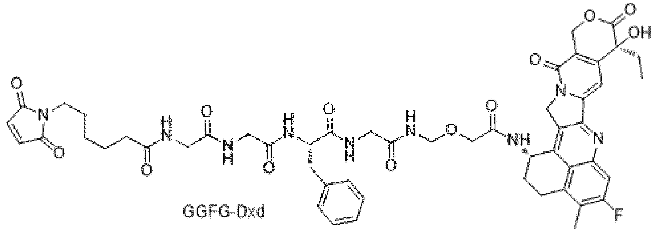

### Example 3: Preparation of Antibody-Drug Conjugates

The antibody FDA016 against B7-H3 was prepared according to the method of Example 1 and was replaced into 50 mM PB/1.0 mM EDTA buffer (pH 7.0) using a G25 desalting column. 12 equivalents of TECP were added thereto and the mixture was stirred at 37°C for 2 hours to fully open the disulfide bonds between the antibody chains. Then, phosphoric acid was used to adjust the pH of the reduced antibody solution to 6.0 and the temperature of the water bath was lowered to 25°C for coupling reaction. The linker-drug conjugates LE12 to LE22, LS13, and GGFG-Dxd prepared according to the above Example 2 were dissolved in DMSO respectively and 12 equivalents of linker-drug conjugate were added dropwise to the reduced antibody solution. Additional DMSO was added to a final concentration of 10% (v/v) and the reaction was stirred at 25°C for 0.5 hours. After the reaction was completed, the sample was filtered through a 0.22 µm membrane. The tangential flow filtration system was used to purify and remove unconjugated small molecules. The buffer was a 50 mM PB/1.0 mM EDTA solution (pH 6.0). After purification, a final concentration of 6% sucrose was added and stored in a -20°C refrigerator. The absorbance values were measured at 280 nm and 370 nm by UV method, respectively, and the DAR value was calculated. The results are shown in Table 2 below.

The coupling reaction was carried out in the same manner as in this example and all samples were prepared according to the highest DAR (i.e., excessive coupling). The occurrence of precipitation during each coupling reaction was observed and the polymer ratio and recovery rate after each coupling reaction were calculated. The results are also shown in Table 2.

**Table 2 Coupling conditions for preparing different antibody-drug conjugates (ADCs)**

| ADC Number | Antibody | Linker-Drug Conjugate | DAR Value | Whether Precipitation | Aggregation Ratio | Recovery Rate |
|---|---|---|---|---|---|---|
| FDA016-LE12 | FDA016 | LE12 | 7.64 | No | 0.5% | 89% |
| FDA016-LE13 | FDA016 | LE13 | 7.67 | No | 0.2% | 98% |
| FDA016-LE14 | FDA016 | LE14 | 7.83 | No | 0.1% | 94% |
| FDA016-LE15 | FDA016 | LE15 | 7.65 | No | 0.3% | 90% |
| FDA016-LE16 | FDA016 | LE16 | 7.78 | No | 0.2% | 85% |
| FDA016-LE17 | FDA016 | LE17 | 7.62 | No | 0.2% | 86% |
| FDA016-LE18 | FDA016 | LE18 | 7.48 | No | 0.3% | 90% |
| FDA016-LE19 | FDA016 | LE19 | 7.52 | No | 0.2% | 89% |
| FDA016-LE20 | FDA016 | LE20 | 7.47 | No | 0.2% | 88% |
| FDA016-LE21 | FDA016 | LE21 | 7.85 | No | 0.3% | 90% |
| FDA016-LE22 | FDA016 | LE22 | 7.72 | No | 0.2% | 89% |
| FDA016-LS13 | FDA016 | LS13 | 7.68 | No | 0.5% | 92% |
| FDA016-8201 | FDA016 | GGFG-Dxd | 7.81 | No | 0.3% | 90% |

| | | | | | | |
|---|---|---|---|---|---|---|
| "/" indicates that the recovery rate is not calculated | | | | | | |

In practical research, it was found that the linker-drug conjugate GGFG-Dxd also produced precipitation when coupled with other antibodies and had a high aggregation ratio, which lacked universality. However, when most of the linker-drug conjugates of the present technical solution were attempted to be coupled with different antibodies including F016, no precipitation was produced and the aggregation ratio was within the normal range, indicating that the antibody-drug conjugates provided by the present disclosure have good solubility and druggability, and that no precipitation during the coupling process is also very conducive to the preparation of antibody-drug conjugates.

### Effect Example 1: In Vitro Killing Activity Evaluation of Antibody-Drug Conjugates

Calu-6 (ATCC) cells were selected as the cell line for in vitro activity detection. 2000 cells per well were seeded in a 96-well cell culture plate and cultured for 20 to 24 hours. The antibody-drug conjugates prepared according to the method of Example 3 were formulated into test solutions with 11 concentration gradients of 1000, 166.7, 55.6, 18.6, 6.17, 2.06, 0.69, 0.23, 0.08, 0.008, and 0 nM using L15 cell culture medium containing 10% FBS. The diluted test solutions were added to the culture plate containing the seeded cells at 100 µL/well and incubated for 144 hours at 37°C in a 5% CO₂ incubator. CellTiter-Glo^{®} Luminescent Cell Viability Assay Reagent (promega, G9243) (50 µL/well) was added and the plate was shaken at 500 rpm at room temperature for 10 minutes to mix well. The data were read using a SpectraMaxL microplate reader (OD 570 nm, reading at 2 s intervals) and the IC50 results were calculated as shown in Table 3.

Using the same method as above, the cytotoxic activity of each antibody-drug conjugate against multiple tumor cells of NCI-N87, A375, Hs-700T, LN-229, MDA-MB-468, PA-1, and Raji purchased from ATCC was tested. The results are shown in Table 3. From the results in Table 3, it can be seen that the antibody-drug conjugates provided by the present disclosure have excellent in vitro killing activity against cells such as Calu-6, NCI-N87, A375, Hs-700T, LN-229, MDA-MB-468, and PA-1. However, the antibody-drug conjugate has no killing activity on Raji-negative cells, indicating that the prepared ADC has specific targeted killing activity.

**Table 3: In vitro killing activity of antibody-drug conjugates**

| ADC Number | IC50 (nM) | | | | | | | |
|---|---|---|---|---|---|---|---|---|
| | **Calu-6 Cell** | **NCI-N87 Cell** | **A375 Cell** | **Hs-700T Cell** | **LN-229 Cell** | **MDA-MB-468 Cell** | **PA-1 Cell** | **Raji Cell** |
| FDA016-LE12 | 1.2 | 7.8 | 0.98 | 3.1 | Not Tested | Not Tested | Not Tested | Not Tested |
| FDA016-LE13 | 2.0 | 10.38 | 1.23 | 2.78 | Not Tested | 15.64 | 20.32 | Not Tested |
| FDA016-LE14 | 0.8 | 5.2 | 0.62 | 2.5 | 3.5 | 9.87 | 8.93 | >500 |
| FDA016-LE15 | 0.93 | 4.98 | 1.12 | 3.23 | 5.28 | 12.38 | 10.23 | >500 |
| FDA016-LE16 | 1.34 | 7.89 | Not Tested | Not Tested | 6.79 | 17.38 | Not Tested | >500 |
| FDA016-LE17 | 0.82 | Not Tested | Not Tested | 3.52 | 3.78 | 16.54 | Not Tested | >500 |
| FDA016-LE18 | 0.96 | Not Tested | Not Tested | Not Tested | 3.28 | Not Tested | Not Tested | >500 |
| FDA016-LE19 | 1.13 | Not Tested | 0.96 | Not Tested | 3.62 | Not Tested | Not Tested | Not Tested |
| FDA016-LE20 | Not Tested | 6.78 | Not Tested | Not Tested | 4.23 | Not Tested | Not Tested | Not Tested |
| FDA016-LE21 | Not Tested | 7.53 | 3.23 | Not Tested | 10.32 | 22.32 | Not Tested | Not Tested |
| FDA016-LE22 | Not Tested | 10.62 | 2.32 | Not Tested | Not Tested | 13.58 | 7.68 | Not Tested |
| FDA016-LS13 | 53.23 | 69.32 | >100 | >100 | Not Tested | Not Tested | Not Tested | Not Tested |
| FDA016-GGFG-Dxd | 1.2 | 6.7 | 0.76 | 3.8 | 5.6 | 10.56 | Not Tested | >500 |

### Effect Example 2: In Vitro Plasma Stability Assay

This example evaluates the stability of the antibody-drug conjugate prepared according to the method of Example 3 in human plasma. Specifically, in this example, the antibody-drug conjugate of Example 3 was added to human plasma and placed in a 37°C water bath for 1, 3, 7, 14, 21, and 28 days. An internal standard (Exatecan as an internal standard substance) was added and extracted and then detected by high-performance liquid chromatography to detect the release of free drugs. The results are shown in Table 4.

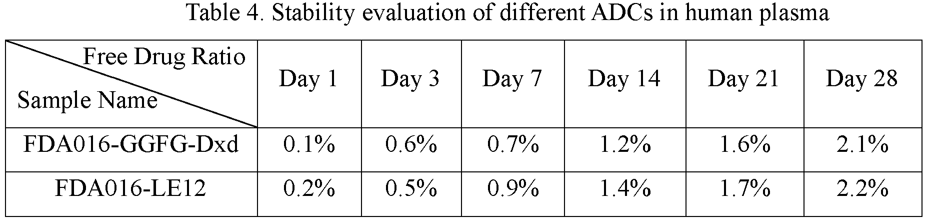

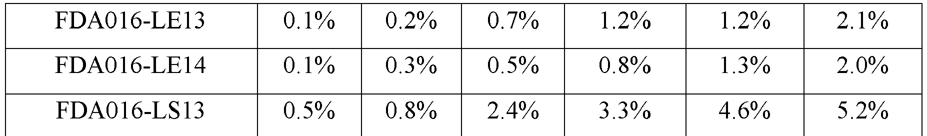

The plasma stability results show that the antibody-drug conjugate provided by the present disclosure has good plasma stability.

### Effect Example 3: In Vitro Enzyme Digestion Experiment of Linker-Drug Conjugates

The linker-drug conjugate (LE14 and GGFG-Dxd) was co-incubated with cathepsin B in three different pH (5.0, 6.0, 7.0) buffers. Samples were taken at different time points and entered into a high-performance liquid chromatography-mass spectrometry instrument. The external standard method (with DXD as the external standard) was used to determine the release percentage of the drug. The experimental results (as shown in Table 5) show that GGFG-Dxd has a slow speed of enzyme digestion within the pH range used, while the **Linker-drug conjugate** LE14 employed by the present disclosure can be quickly enzymatically digested within the pH range of 5.0 to 7.0.

**Table 5. In vitro enzyme digestion of LE14 and GGFG-Dxd at different pH**

| Time (h) | Percentage of drug release in the sample % | | | | | |
|---|---|---|---|---|---|---|
| | GGFG-Dxd | | | LE14 | | |
| | pH 5.0 | pH 6.0 | pH 7.0 | pH 5.0 | pH 6.0 | pH 7.0 |
| 0 | 22.0 | 22.35 | 22.16 | 13.82 | 14.32 | 16.59 |
| 1 | 24.82 | 24.8 | 25.76 | 96.0 | 96.12 | 98.32 |
| 2 | 25.85 | 27.32 | 29.45 | 98.35 | 96.75 | 98.45 |
| 3 | 27.46 | 29.32 | 32.00 | 99.12 | 98.52 | 99.12 |
| 4 | 29.68 | 32.0 | 34.72 | 99.21 | 98.45 | 99.12 |
| 5 | 31.72 | 33.15 | 37.17 | 99.45 | 98.92 | 99.87 |
| 6 | 34.17 | 36.38 | 38.43 | 98.23 | 99.15 | 99.47 |

### Effect Example 4: In Vitro Enzyme Digestion Experiment of FDA016-LS13 (comparative example)

NCI-N87 cell line was selected as the experimental cell lines. After the sample was incubated in cathepsin B system (100 mM sodium acetate-acetic acid buffer, 4 mM dithiothreitol, pH 5.0) at 37°C for 4 hours, the obtained sample was diluted with culture medium to different concentrations. 8 concentrations (1.5 to 10-fold dilution) were set from 70 nM to 0.003 nM of SN-38 concentration. The killing (inhibitory) ability of the cell line was observed for 144 hours. The IC50 value was calculated by reading the fluorescence data after chemical luminescent staining with CellTiter-Glo^{®} Luminescent Cell Viability Assay.

The above enzyme digestion samples obtained by incubating in a cathepsin B system at 37°C for 4 hours were precipitated with an appropriate amount of ethanol to remove protein and detected by high-performance liquid chromatography to release small molecule compounds. The 4-hour release rate was measured with an equal amount of SN-38 as a reference, and the results showed that the release rate reached 99%.

The experimental results (as shown in Table 6) show that after enzyme digestion treatment, the cytotoxic activity of FDA016-LS13 is almost the same as that of SN-38 at an equivalent dose, which also indicates that FDA016-LS13 has almost completely released SN-38 under the action of cathepsin B and played a role. However, FDA016-LS13 may have undergone unpredictable changes when it is endocytosed into lysosomes, resulting in SN-38 not being able to function effectively.

**Table 6. Changes in killing activity of FDA016-LS13 on NCI-N87 cell line before and after enzyme digestion by cathepsin B system**

| | | |
|---|---|---|
| | IC50 (based on SN-38 equivalent, nM) | |

| Sample | Before Enzyme Digestion | After Enzyme Digestion |
|---|---|---|
| FDA016-LS13 | > 100 nM | 6.32 nM |
| SN-38 | 7.13nM | 7.45 nM |

### Effect Example 5: Testing Antitumor Activity of FDA016-LE14 in Calu-6 Human Lung Cancer Model

6- to 8-week-old female Balb/c nude mice were subcutaneously injected with 5×10⁶ human lung cancer cells (Calu-6) dissolved in 100 µL of PBS solution on the right side of the neck and back. When the tumor grew to an average volume of 150 to 200 mm³, mice were randomly divided into 5 groups according to tumor size and mouse weight, with 6 animals in each group. The groups were blank control group, 5 mg/kg FDA016-GGFG-Dxd group, 10 mg/kg FDA016-GGFG-Dxd group, 5 mg/kg FDA016 -LE14 group, and 10 mg/kg FDA016-LE14 group, respectively, administered intraperitoneally once a week. The animal weight and tumor volume were measured twice a week, and the survival status of the experimental animals was observed during the experiment process. As shown in Table 7, the average tumor volume of the mice in the blank control group was 2107.51 mm³ at the end of treatment. The average tumor volume of the FDA016-GGFG-Dxd treatment group at 5.0 mg/kg was 72.35 mm³ on the 14th day after the end of treatment, and the average tumor volume of the FDA016-GGFG-Dxd treatment group at 10 mg/kg was 3.28 mm³ on the 14th day after the end of treatment. The average tumor volume of the FDA016-LE14 treatment group at 5.0 mg/kg was 50.48 mm³ on the 14th day after the end of treatment, and the average tumor volume of the FDA016-LE14 treatment group at 10 mg/kg was 0.00 mm³ on the 14th day after the end of treatment. The experimental results show that FDA016-LE14 has good in vivo antitumor activity, and all experimental mice have no death or weight loss, indicating that FDA016-LE14 has good safety.

**Table 7. Antitumor activity of FDA016-LE14 in Calu-6 human lung cancer model**

| **Grou -ping** | **Observation Days** | | | | | | | | | | |
|---|---|---|---|---|---|---|---|---|---|---|---|
| | **13** | **16** | **20** | **23** | **27** | **30** | **34** | **37** | **41** | **44** | **48** |
| Average Tumor Volume/mm³ | | | | | | | | | | | |
| **01** | 150.65 | 205.67 | 538.37 | 783.23 | 946.54 | 1238.48 | 1472.54 | 1753.15 | 2107.51 | / | / |
| **02** | 150.45 | 185.32 | 117.78 | 113.26 | 79.88 | 46.40 | 44.56 | 42.81 | 49.38 | 62.53 | 72.3 5 |
| **03** | 150.83 | 182.35 | 93.56 | 39.61 | 10.68 | 7.75 | 5.83 | 4.28 | 3.91 | 3.83 | 3.28 |
| **04** | 150.52 | 174.27 | 114.94 | 108.40 | 74.98 | 42.34 | 37.45 | 39.12 | 42.53 | 47.85 | 50.4 8 |
| **05** | 150.46 | 168.08 | 89.23 | 32.13 | 4.15 | 2.12 | 1.35 | 1.12 | 0.00 | 0.00 | 0.00 |
| Standard Deviation | | | | | | | | | | | |
| **01** | 10.37 | 31.73 | 32.42 | 77.06 | 85.50 | 81.66 | 92.36 | / | / | / | / |
| **02** | 10.81 | 30.80 | 21.55 | 17.67 | 11.96 | 14.21 | 12.45 | 10.13 | 8.38 | 6.23 | 6.27 |
| **03** | 11.13 | 23.15 | 23.03 | 19.12 | 13.67 | 2.19 | 1.83 | 0.28 | 1.83 | 1.35 | 0.56 |
| **04** | 10.74 | 27.49 | 22.75 | 13.43 | 15.76 | 12.31 | 15.57 | 20.69 | 13.81 | 9.14 | 6.58 |
| **05** | 10.93 | 18.63 | 20.43 | 11.58 | 3.27 | 1.83 | 1.56 | 0.70 | 0.00 | 0.00 | 0.00 |

Note: Group 01 is the blank control group; group 02 is the 5 mg/kg FDA016-GGFG-Dxd group; group 03 is the 10 mg/kg FDA016-GGFG-Dxd group; group 04 is the 5 mg/kg FDA016-LE14 group; group 05 is the 10 mg/kg FDA016-LE14 group.

## Claims

1. An antibody-drug conjugate, a pharmaceutically acceptable salt thereof, a solvate thereof, or a solvate of the pharmaceutically acceptable salt thereof, wherein the antibody-drug conjugate has a structure shown in formula **I;** 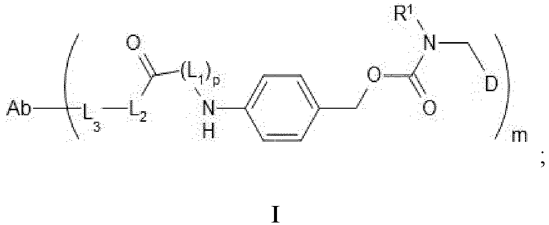
wherein Ab is a B7-H3 antibody or a variant of the B7-H3 antibody; m is 2 to 8;
the amino acid sequence of the light chain in the B7-H3 antibody is shown in SEQ ID NO: 1, and the amino acid sequence of the heavy chain is shown in SEQ ID NO: 2;
the variant of the B7-H3 antibody is at least 70%, 75%, 80%, 85%, 90%, 95%, 98%, or 99% identical to the B7-H3 antibody;
D is a cytotoxic drug topoisomerase inhibitor, wherein the cytotoxic drug topoisomerase inhibitor is 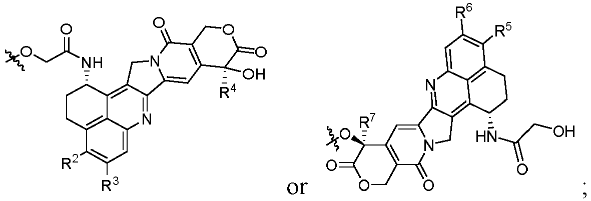 R² and R⁵ are each independently H, C₁-C₆ alkyl, or halogen; R³ and R⁶ are each independently H, C₁-C₆ alkyl, or halogen; R⁴ and R⁷ are each independently C₁-C₆ alkyl;
R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, C₃-C₁₀ cycloalkyl, C₆-C₁₄ aryl, or 5- to 14-membered heteroaryl; the heteroatom in the 5- to 14-membered heteroaryl is selected from one or more than one of N, O, and S, and the number of heteroatoms is 1, 2, 3, or 4; the R¹⁻¹, R¹⁻², and R¹⁻³ are each independently C₁-C₆ alkyl;
L₁ is independently one or more than one of a phenylalanine residue, alanine residue, glycine residue, glutamic acid residue, aspartic acid residue, cysteine residue, histidine residue, isoleucine residue, leucine residue, lysine residue, methionine residue, proline residue, serine residue, threonine residue, tryptophan residue, tyrosine residue, and valine residue; p is 2 to 4;
L₂ is 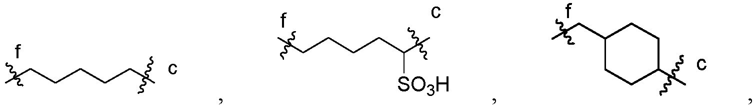 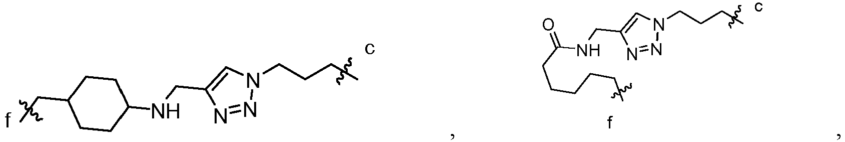 or 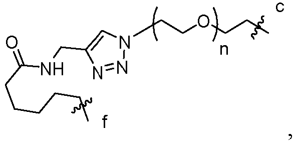 wherein n is independently 1 to 12, the c-terminal is connected to L₁ through a carbonyl group, and the f-terminal is connected to the d-terminal of L₃;
L₃ is 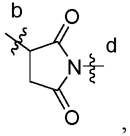 wherein the b-terminal is connected to the Ab, and the d-terminal is connected to the f-terminal of L₂.

2. The antibody-drug conjugate, the pharmaceutically acceptable salt thereof, the solvate thereof, or the solvate of the pharmaceutically acceptable salt thereof according to claim 1, wherein
the b-terminal of L₃ is connected to the sulfhydryl group on the antibody in the form of a thioether;
and/or, when R¹ is C₁-C₆ alkyl substituted by one or more than one -NR¹⁻¹R¹⁻², the C₁-C₆ alkyl is methyl, ethyl, n-propyl, isopropyl, n-butyl, isobutyl, or tert-butyl, preferably ethyl;
and/or, when R¹ is C₁-C₆ alkyl substituted by more than one -NR¹⁻¹R¹⁻², the "more than one" is two or three;
and/or, when R¹⁻¹ and R¹⁻² are each independently C₁-C₆ alkyl, the C₁-C₆ alkyl is methyl, ethyl, n-propyl, isopropyl, n-butyl, isobutyl, or tert-butyl, preferably methyl;
and/or, when R¹ is C₁-C₆ alkyl substituted by one or more than one R¹⁻³S(O)₂-, the C₁-C₆ alkyl is C₁-C₄ alkyl, preferably methyl, ethyl, n-propyl, isopropyl, n-butyl, isobutyl, or tert-butyl, more preferably ethyl;
and/or, when R¹ is C₁-C₆ alkyl substituted by more than one R¹⁻³S(O)₂-, the "more than one" is two or three;
and/or, when R¹⁻³ is C₁-C₆ alkyl, the C₁-C₆ alkyl is methyl, ethyl, n-propyl, isopropyl, n-butyl, isobutyl, or tert-butyl, preferably methyl;
and/or, when R¹ is C₁-C₆ alkyl, the C₁-C₆ alkyl is methyl, ethyl, n-propyl, isopropyl, n-butyl, isobutyl, or tert-butyl, preferably methyl;
and/or, m is an integer or non-integer, preferably 4 to 8, more preferably 6 to 8, further preferably 7 to 8;
and/or, p is 2;
and/or, n is 8 to 12;
and/or, when R¹⁻¹, R¹⁻², and R¹⁻³ are independently C₁-C₆ alkyl, the C₁-C₆ alkyl is C₁-C₄ alkyl.

3. The antibody-drug conjugate, the pharmaceutically acceptable salt thereof, the solvate thereof, or the solvate of the pharmaceutically acceptable salt thereof according to claim 1, wherein
when R² and R⁵ are each independently C₁-C₆ alkyl, the C₁-C₆ alkyl is C₁-C₄ alkyl, further preferably methyl, ethyl, n-propyl, isopropyl, n-butyl, isobutyl, or tert-butyl, most preferably methyl;
and/or, when R² and R⁵ are each independently halogen, the halogen is fluorine, chlorine, bromine, or iodine, preferably fluorine;
and/or, when R³ and R⁶ are each independently C₁-C₆ alkyl, the C₁-C₆ alkyl is C₁-C₄ alkyl, further preferably methyl, ethyl, n-propyl, isopropyl, n-butyl, isobutyl, or tert-butyl, most preferably methyl;
and/or, when R³ and R⁶ are each independently halogen, the halogen is fluorine, chlorine, bromine, or iodine, further preferably fluorine;
and/or, when R⁴ and R⁷ are each independently C₁-C₆ alkyl, the C₁-C₆ alkyl is C₁-C₄ alkyl, further preferably methyl, ethyl, n-propyl, isopropyl, n-butyl, isobutyl, or tert-butyl, most preferably ethyl;
and/or, when R¹ is C₁-C₆ alkyl substituted by one or more than one -NR¹⁻¹R¹⁻², the -NR¹⁻¹R¹⁻² is -N(CH₃)₂;
and/or, when R¹ is C₁-C₆ alkyl substituted by one -NR¹⁻¹R¹⁻², the C₁-C₆ alkyl substituted by one -NR¹⁻¹R¹⁻² is 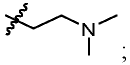
and/or, when R¹ is C₁-C₆ alkyl substituted by one R¹⁻³S(O)₂-, the C₁-C₆ alkyl substituted by one R¹⁻³S(O)₂- is 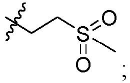
and/or, m is 7.4 to 7.85, such as 7.47, 7.48, 7.52, 7.62, 7.64, 7.65, 7.67, 7.72, 7.78, 7.83, or 7.85;
and/or, (L₁)ₚ is 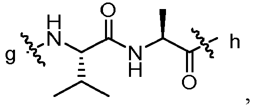 wherein the g-terminal is connected to the c-terminal of L₂ through a carbonyl group;
and/or, n is 8, 9, 10, 11, or 12;
and/or, when R¹⁻¹, R¹⁻², and R¹⁻³ are independently C₁-C₆ alkyl, the C₁-C₆ alkyl is methyl, ethyl, n-propyl, isopropyl, n-butyl, isobutyl, or tert-butyl, preferably methyl.

4. The antibody-drug conjugate, the pharmaceutically acceptable salt thereof, the solvate thereof, or the solvate of the pharmaceutically acceptable salt thereof according to claim 1, wherein
R² and R⁵ are each independently C₁-C₆ alkyl;
and/or, R³ and R⁶ are each independently halogen;
and/or, R⁴ and R⁷ are ethyl;
and/or, L₁ is one or more than one of the phenylalanine residue, alanine residue, glycine residue, isoleucine residue, leucine residue, proline residue, and valine residue; preferably one or more than one of the phenylalanine residue, alanine residue, glycine residue, and valine residue; further preferably the valine residue and/or the alanine residue, and the "more than one" is preferably two or three;
and/or, R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, or C₃-C₁₀ cycloalkyl; preferably C₁-C₆ alkyl substituted by one or more than one -NR¹⁻¹R¹⁻², C₁-C₆ alkyl substituted by one or more than one R¹⁻³S(O)₂-, or C₁-C₆ alkyl; further preferably C₁-C₆ alkyl substituted by one or more than one -NR¹⁻¹R¹⁻², or C₁-C₆ alkyl substituted by one or more than one R¹⁻³S(O)₂-; most preferably C₁-C₆ alkyl substituted by one or more than one R¹⁻³S(O)₂-;
preferably, D is

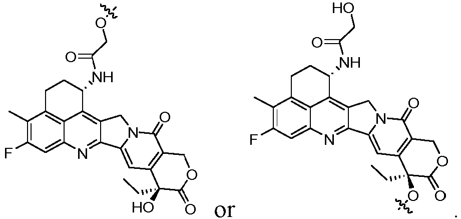

5. The antibody-drug conjugate, the pharmaceutically acceptable salt thereof, the solvate thereof, or the solvate of the pharmaceutically acceptable salt thereof according to any one of claims 1 to 4, wherein the antibody-drug conjugate is any one of the following schemes:
scheme I:
Ab is the B7-H3 antibody or the variant of the B7-H3 antibody, preferably the B7-H3 antibody;
D is 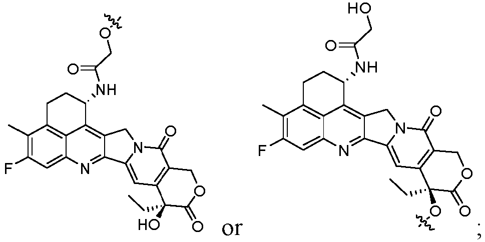
R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, or C₃-C₁₀ cycloalkyl;
L₁ is independently one or more than one of the phenylalanine residue, alanine residue, glycine residue, isoleucine residue, leucine residue, proline residue, and valine residue;
scheme II:
Ab is the B7-H3 antibody or the variant of the B7-H3 antibody, preferably the B7-H3 antibody;
D is 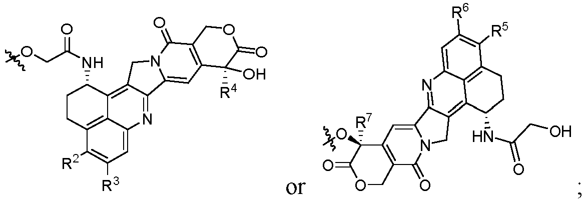 wherein R² and R⁵ are each independently C₁-C₆ alkyl; R³ and R⁶ are each independently halogen; D is preferably 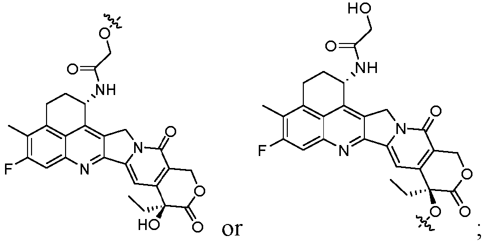
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
L₁ is independently one or more than one of the phenylalanine residue, alanine residue, glycine residue, isoleucine residue, leucine residue, proline residue, and valine residue;
scheme III:
Ab is the B7-H3 antibody or the variant of the B7-H3 antibody, preferably the B7-H3 antibody;
D is 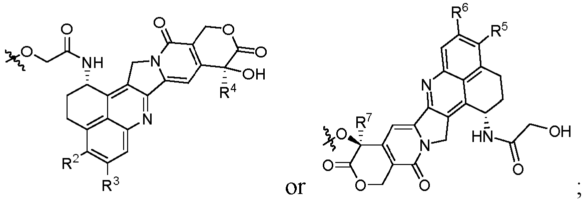 wherein R² and R⁵ are each independently C₁-C₆ alkyl; R³ and R⁶ are each independently halogen; D is preferably 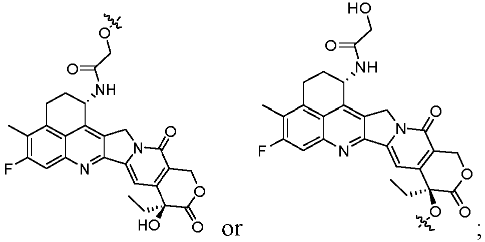
m is 7 to 8;
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
L₁ is independently the valine residue and/or the alanine residue;
scheme IV:
Ab is the B7-H3 antibody or the variant of the B7-H3 antibody, preferably the B7-H3 antibody;
D is 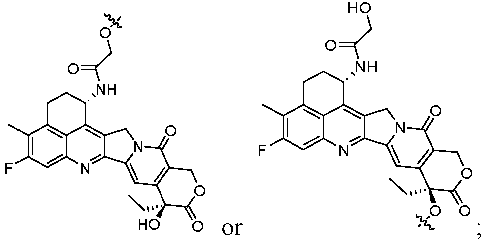
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
scheme V:
Ab is the B7-H3 antibody or the variant of the B7-H3 antibody, preferably the B7-H3 antibody;
D is 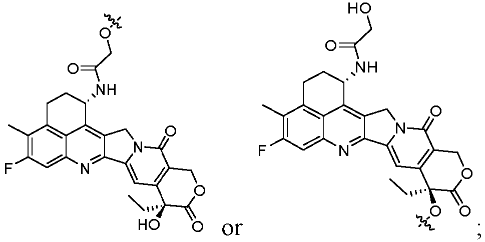
m is 7 to 8;
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
L₁ is independently the valine residue and/or the alanine residue.

6. The antibody-drug conjugate, the pharmaceutically acceptable salt thereof, the solvate thereof, or the solvate of the pharmaceutically acceptable salt thereof according to claim 1, wherein the antibody-drug conjugate is any one of the following compounds: 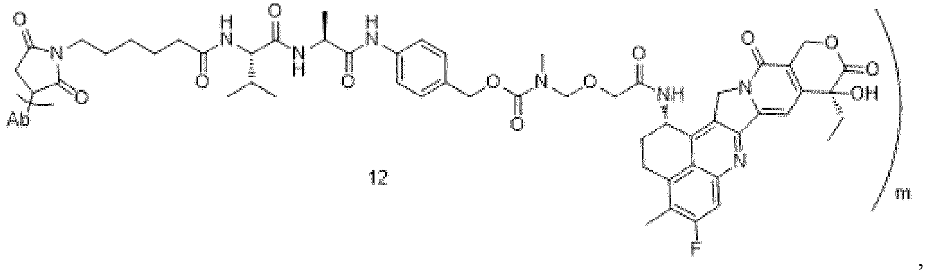 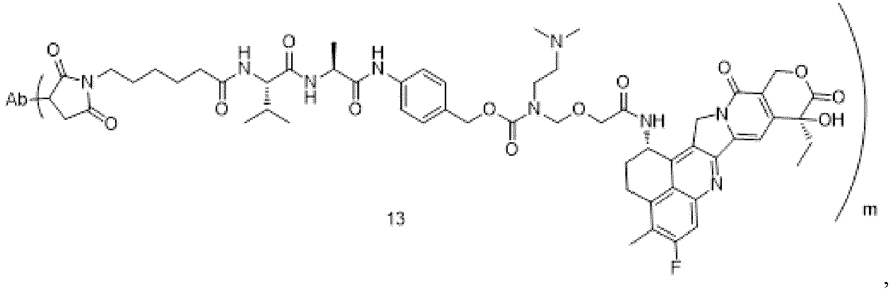 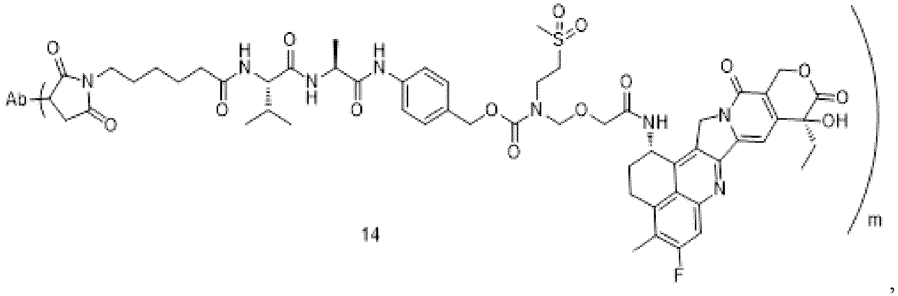 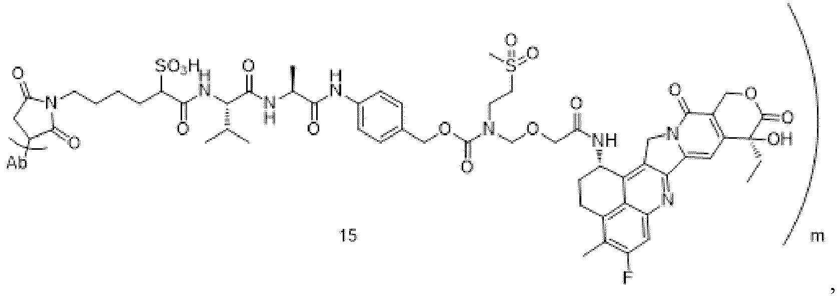 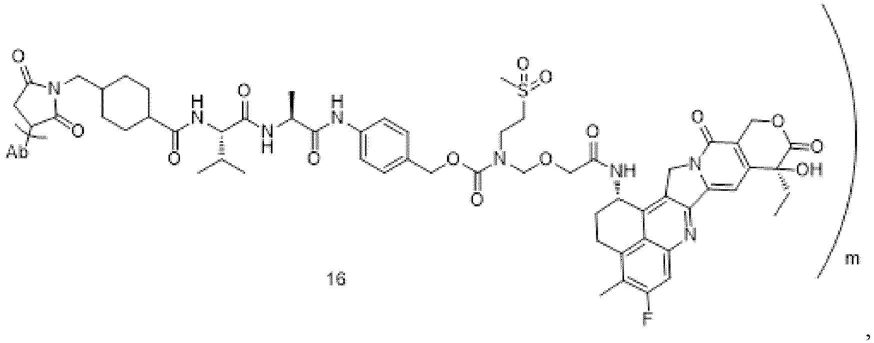 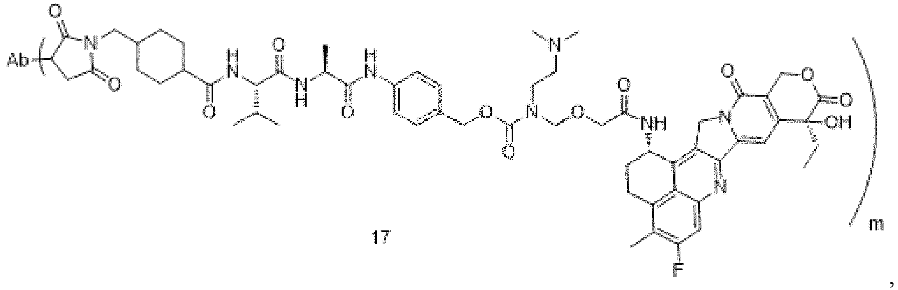 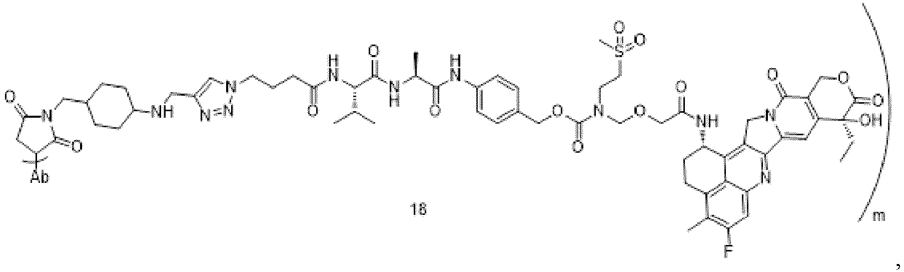 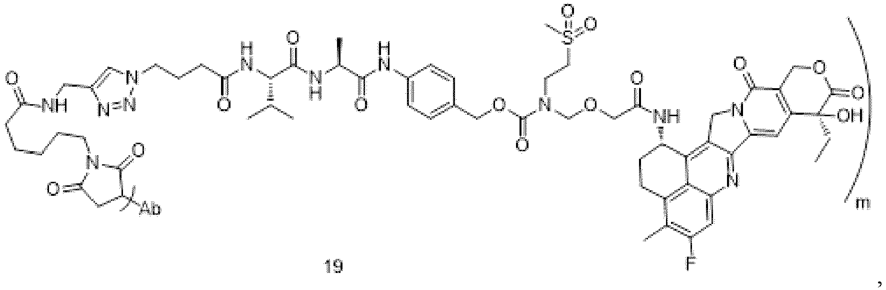 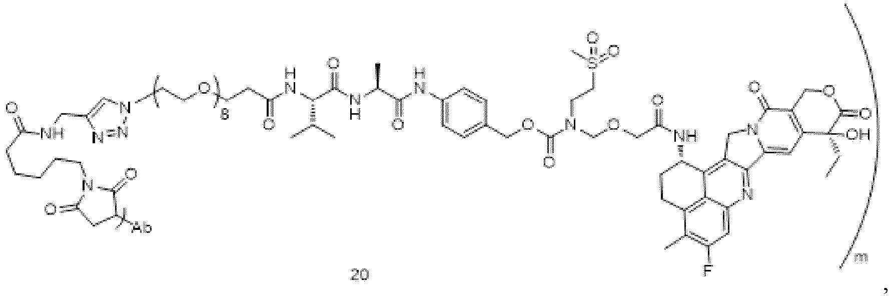 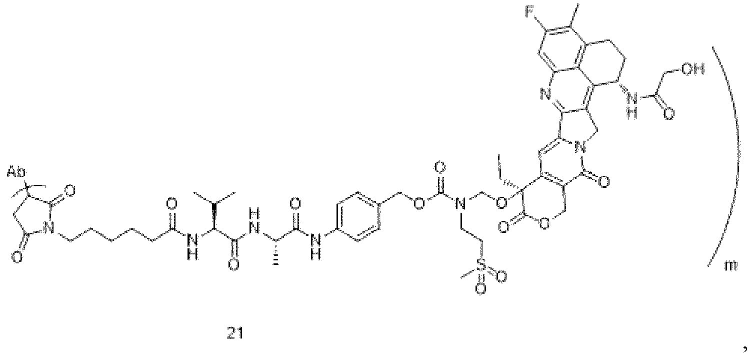 or 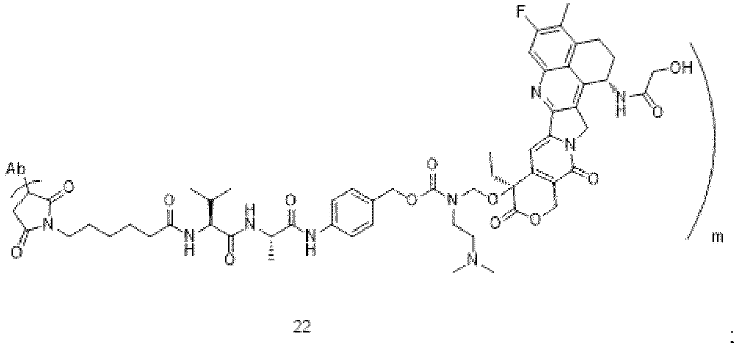 wherein, Ab is the B7-H3 antibody or the variant of the B7-H3 antibody, and m is 7.47, 7.48, 7.52, 7.62, 7.64, 7.65, 7.67, 7.72, 7.78, 7.83, or 7.85.

7. The antibody-drug conjugate, the pharmaceutically acceptable salt thereof, the solvate thereof, or the solvate of the pharmaceutically acceptable salt thereof according to claim 1, wherein the antibody-drug conjugate is any one of the following compounds: 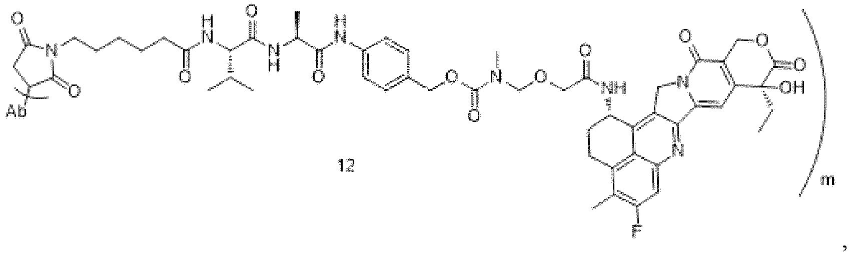 Ab is the B7-H3 antibody, and m is 7.64; 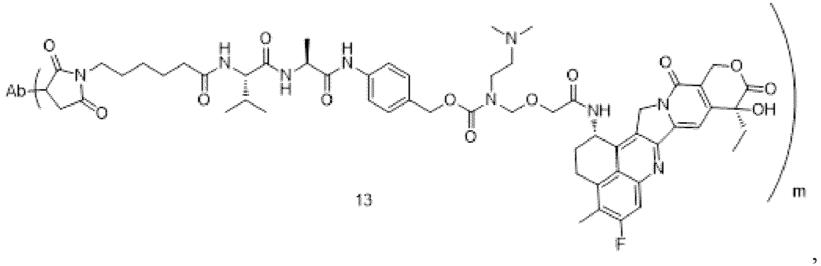 Ab is the B7-H3 antibody, and m is 7.67; 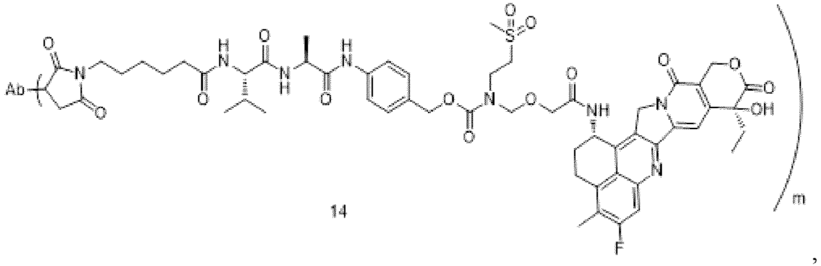 Ab is the B7-H3 antibody, and m is 7.83; 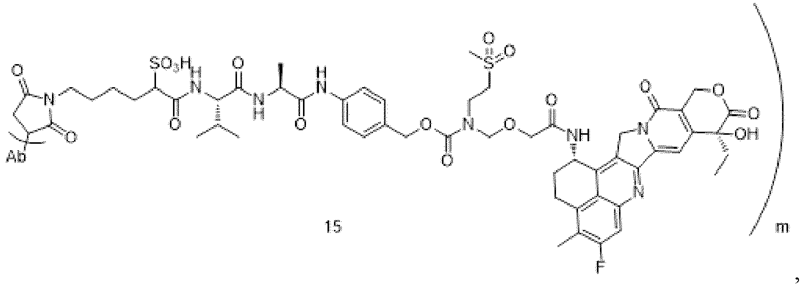 Ab is the B7-H3 antibody, and m is 7.65; 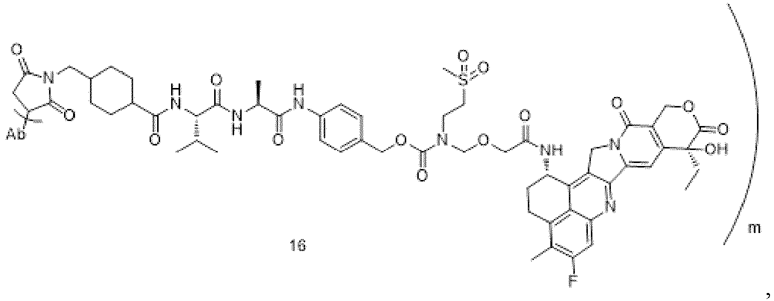 Ab is the B7-H3 antibody, and m is 7.78; 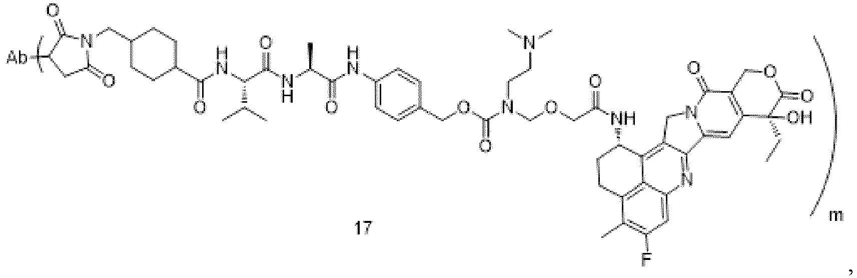 Ab is the B7-H3 antibody, and m is 7.62; 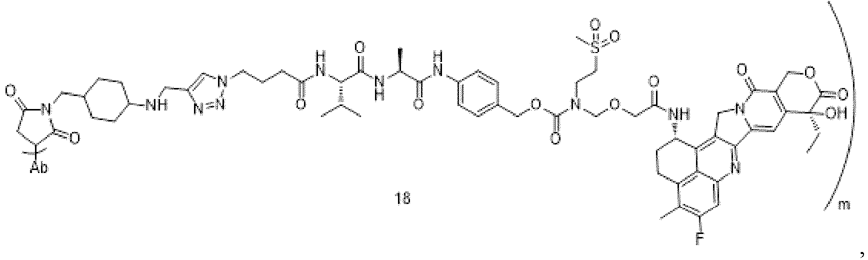 Ab is the B7-H3 antibody, and m is 7.48; 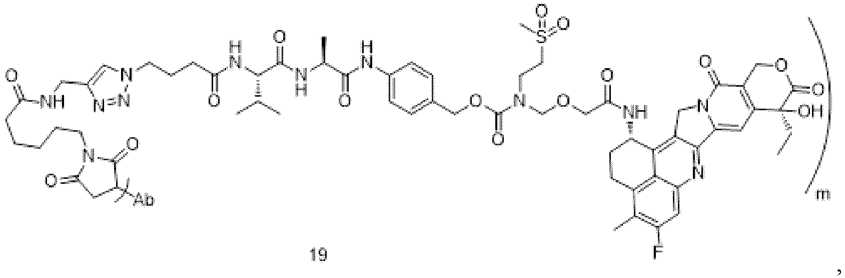 Ab is the B7-H3 antibody, and m is 7.52; 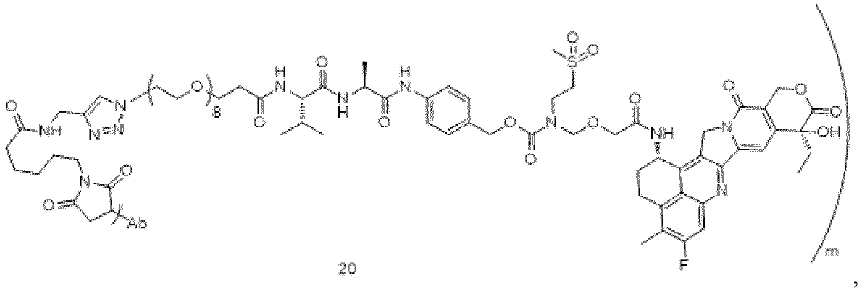 Ab is the B7-H3 antibody, and m is 7.47; 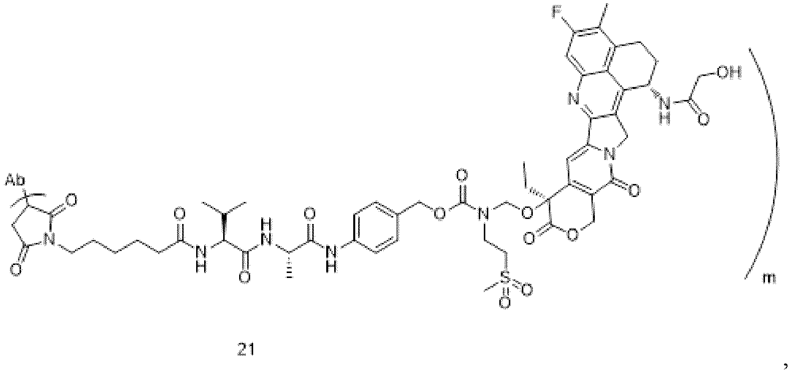 Ab is the B7-H3 antibody, and m is 7.85; or 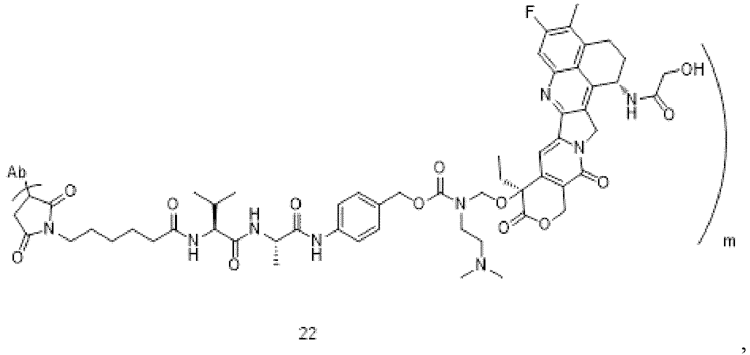 Ab is the B7-H3 antibody, and m is 7.72.

8. A method for preparing the antibody-drug conjugate according to any one of claims 1 to 7, comprising the following steps: carrying out a coupling reaction between a compound of formula II and Ab-hydrogen as shown below;

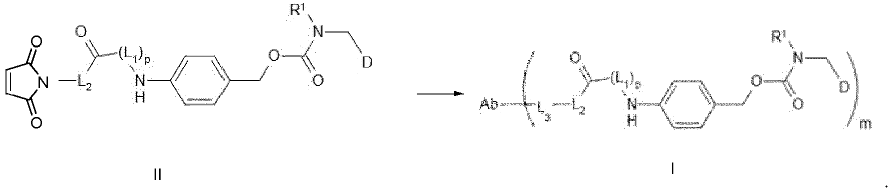

9. A pharmaceutical composition, comprising substance X and a pharmaceutically acceptable excipient, wherein the substance X is the antibody-drug conjugate, the pharmaceutically acceptable salt thereof, the solvate thereof, or the solvate of the pharmaceutically acceptable salt thereof according to any one of claims 1 to 7, the amount of the substance X is preferably a therapeutically effective amount.

10. Substance X for use in inhibiting B7-H3 protein, wherein the substance X is the antibody-drug conjugate, the pharmaceutically acceptable salt thereof, the solvate thereof, or the solvate of the pharmaceutically acceptable salt thereof according to any one of claims 1 to 7.

11. Substance X for use in treating and/or preventing a tumor, wherein the substance X is the antibody-drug conjugate, the pharmaceutically acceptable salt thereof, the solvate thereof, or the solvate of the pharmaceutically acceptable salt thereof according to any one of claims 1 to 7.

12. Substance X for use according to claim 11, wherein the tumor is a B7-H3 positive tumor; and the B7-H3 positive tumor is one or more than one of B7-H3 positive lung cancer, ovarian cancer, melanoma, pancreatic cancer, breast cancer, brain glioma, prostate cancer, and gastric cancer.

13. Substance X for use according to claim 12, wherein the lung cancer cells are *NCI-1703* cells or Calu-6 cells;
and/or, the ovarian cancer cells are PA-1 cells;
and/or, the melanoma cells are A375 cells;
and/or, the pancreatic cancer cells are Hs-700T cells;
and/or, the breast cancer cells are MDA-MB-468 cells;
and/or, the brain glioma cells are LN-229 cells;
and/or, the gastric cancer cells are NCI-N87 cells.

## Patentansprüche

1. Antikörper-Wirkstoff-Konjugat, pharmazeutisch verträgliches Salz davon, Solvat davon oder Solvat des pharmazeutisch verträglichen Salzes davon, wobei das Antikörper-Wirkstoff-Konjugat eine in Formel **I** gezeigte Struktur aufweist; 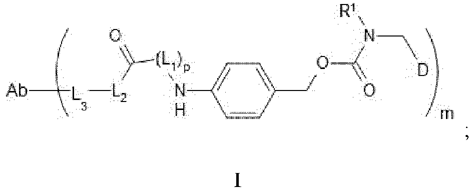
wobei Ab ein B7-H3-Antikörper oder eine Variante des B7-H3-Antikörpers ist; m 2 bis 8 ist;
die Aminosäuresequenz der leichten Kette im B7-H3-Antikörper in SEQ. ID-Nr. 1 gezeigt ist, und die Aminosäuresequenz der schweren Kette in SEQ. ID-Nr. 2 gezeigt ist;
die Variante des B7-H3-Antikörpers mindestens 70 %, 75 %, 80 %, 85 %, 90 %, 95 %, 98 %, oder 99 % mit dem B7-H3-Antikörper identisch ist;
D ein zytotoxischer Wirkstoff-Topoisomerase-Inhibitor ist, wobei der zytotoxische Wirkstoff-Topoisomerase-
Inhibitor 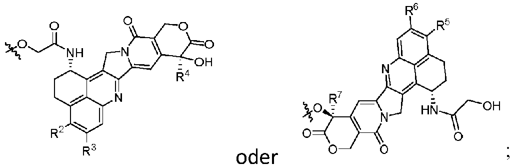
ist, R² und R⁵ jeweils unabhängig H, C₁-C₆-Alkyl oder Halogen sind; R³ und R⁶ jeweils unabhängig H, C₁-C₆-Alkyl oder Halogen sind; R⁴ und R⁷ jeweils unabhängig C₁-C₆-Alkyl sind;
R¹ C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, C₁-C₆-Alkyl, C₃-C₁₀-Cycloalkyl, C₆-C₁₄-Aryl oder 5- bis 14-gliedriges Heteroaryl ist; das Heteroatom im 5- bis 14-gliedrigen Heteroaryl ausgewählt ist aus einem oder mehr als einem von N, O und S, und die Anzahl der Heteroatome 1, 2, 3, oder 4 beträgt; R¹⁻¹, R¹⁻² und R¹⁻³ jeweils unabhängig C₁-C₆-Alkyl sind;
L₁ unabhängig einer oder mehr als einer von einem Phenylalaninrest, Alaninrest, Glycinrest, Glutaminsäurerest, Asparaginsäurerest, Cysteinrest, Histidinrest, Isoleucinrest, Leucinrest, Lysinrest, Methioninrest, Prolinrest, Serinrest, Threoninrest, Tryptophanrest, Tyrosinrest und Valinrest ist; p 2 bis 4 ist;
L₂ 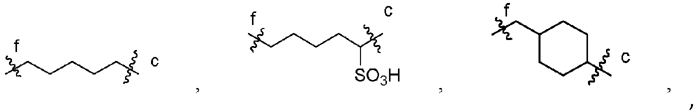 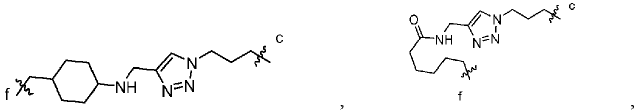 oder 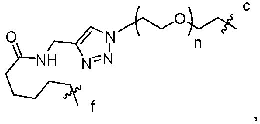 ist, wobei n unabhängig 1 bis 12 ist, der c-Terminus über eine Carbonylgruppe mit L₁ verbunden ist und der f-Terminus mit dem d-Terminus von L₃ verbunden ist;
L₃ 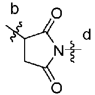 ist, wobei der b-Terminus mit dem Ab verbunden ist und der d-Terminus mit dem f-Terminus von L₂ verbunden ist.

2. Antikörper-Wirkstoff-Konjugat, pharmazeutisch verträgliches Salz davon, Solvat davon oder Solvat des pharmazeutisch verträglichen Salzes davon nach Anspruch 1, wobei
der b-Terminus von L₃ in Form eines Thioethers mit der Sulfhydrylgruppe des Antikörpers verbunden ist;
und/oder wenn R¹ C₁-C₆-Alkyl ist, das durch ein oder mehr als ein -NR¹⁻¹R¹⁻² substituiert ist, das C₁-C₆-Alkyl Methyl, Ethyl, n-Propyl, Isopropyl, n-Butyl, Isobutyl oder tert-Butyl, bevorzugt Ethyl, ist;
und/oder wenn R¹ C₁-C₆-Alkyl ist, das durch mehr als ein -NR¹⁻¹R¹⁻² substituiert ist, das "mehr als ein" zwei oder drei ist;
und/oder wenn R¹⁻¹ und R¹⁻² jeweils unabhängig C₁-C₆-Alkyl sind, das C₁-C₆-Alkyl Methyl, Ethyl, n-Propyl, Isopropyl, n-Butyl, Isobutyl oder tert-Butyl, bevorzugt Methyl, ist;
und/oder wenn R¹ C₁-C₆-Alkyl ist, das durch ein oder mehr als ein R¹⁻³S(O)₂₋substituiert ist, das C₁-C₆-Alkyl C₁-C₄-Alkyl, bevorzugt Methyl, Ethyl, n-Propyl, Isopropyl, n-Butyl, Isobutyl oder tert-Butyl, bevorzugter Ethyl, ist;
und/oder wenn R¹ C₁-C₆-Alkyl ist, das durch mehr als ein R¹⁻³S(O)₂₋substituiert ist, das "mehr als ein" zwei oder drei ist;
und/oder wenn R¹⁻³ C₁-C₆-Alkyl ist, das C₁-C₆-Alkyl Methyl, Ethyl, n-Propyl, Isopropyl, n-Butyl, Isobutyl oder tert-Butyl, bevorzugt Methyl, ist;
und/oder wenn R¹ C₁-C₆-Alkyl ist, das C₁-C₆-Alkyl Methyl, Ethyl, n-Propyl, Isopropyl, n-Butyl, Isobutyl oder tert-Butyl, bevorzugt Methyl, ist;
und/oder m eine ganze Zahl oder keine ganze Zahl, bevorzugt 4 bis 8, bevorzugter 6 bis 8, weiter bevorzugt 7 bis 8 ist;
und/oder p 2 ist;
und/oder n 8 bis 12 ist;
und/oder wenn R¹⁻¹, R¹⁻² und R¹⁻³ unabhängig C₁-C₆-Alkyl sind, das C₁-C₆-Alkyl C₁-C₄-Alkyl ist.

3. Antikörper-Wirkstoff-Konjugat, pharmazeutisch verträgliches Salz davon, Solvat davon oder Solvat des pharmazeutisch verträglichen Salzes davon nach Anspruch 1, wobei
wenn R² und R⁵ jeweils unabhängig C₁-C₆-Alkyl sind, das C₁-C₆-Alkyl C₁-C₄-Alkyl, weiter bevorzugt Methyl, Ethyl, n-Propyl, Isopropyl, n-Butyl, Isobutyl oder tert-Butyl, besonders bevorzugt Methyl, ist;
und/oder wenn R² und R⁵ jeweils unabhängig Halogen sind, das Halogen Fluor, Chlor, Brom oder Iod, bevorzugt Fluor, ist;
und/oder wenn R³ und R⁶ jeweils unabhängig C₁-C₆-Alkyl sind, das C₁-C₆-Alkyl C₁-C₄-Alkyl, weiter bevorzugt Methyl, Ethyl, n-Propyl, Isopropyl, n-Butyl, Isobutyl oder tert-Butyl, besonders bevorzugt Methyl, ist;
und/oder wenn R³ und R⁶ jeweils unabhängig Halogen sind, das Halogen Fluor, Chlor, Brom oder Iod, weiter bevorzugt Fluor, ist;
und/oder wenn R⁴ und R⁷ jeweils unabhängig C₁-C₆-Alkyl sind, das C₁-C₆-Alkyl C₁-C₄-Alkyl, weiter bevorzugt Methyl, Ethyl, n-Propyl, Isopropyl, n-Butyl, Isobutyl oder tert-Butyl, besonders bevorzugt Ethyl, ist;
und/oder wenn R¹ C₁-C₆-Alkyl ist, das durch ein oder mehr als ein -NR¹⁻¹R¹⁻² substituiert ist, -NR¹⁻¹R¹⁻² -N(CH₃)₂ ist;
und/oder wenn R¹ C₁-C₆-Alkyl ist, das durch ein -NR¹⁻¹R¹⁻² substituiert ist, das durch ein -NR¹⁻¹R¹⁻² substituierte C₁-C₆-Alkyl 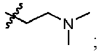 ist,
und/oder wenn R¹ C₁-C₆-Alkyl ist, das durch ein R¹⁻³S(O)₂₋ substituiert ist, das durch ein R¹⁻³S(O)₂- substituierte C₁-C₆-Alkyl 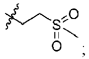 ist,
und/oder m 7,4 bis 7,85, wie etwa 7,47, 7,48, 7,52, 7,62, 7,64, 7,65, 7,67, 7,72, 7,78, 7,83 oder 7,85 ist;
und/oder (L₁)ₚ 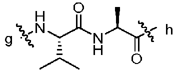
ist, wobei der g-Terminus über eine Carbonylgruppe mit dem c-Terminus von L₂ verbunden ist;
und/oder n 8, 9, 10, 11 oder 12 ist;
und/oder wenn R¹⁻¹, R¹⁻² und R¹⁻³ unabhängig C₁-C₆-Alkyl sind, das C₁-C₆-Alkyl Methyl, Ethyl, n-Propyl, Isopropyl, n-Butyl, Isobutyl oder tert-Butyl, bevorzugt Methyl, ist.

4. Antikörper-Wirkstoff-Konjugat, pharmazeutisch verträgliches Salz davon, Solvat davon oder Solvat des pharmazeutisch verträglichen Salzes davon nach Anspruch 1, wobei
R² und R⁵ jeweils unabhängig C₁-C₆-Alkyl sind;
und/oder R³ und R⁶ jeweils unabhängig Halogen sind;
und/oder R⁴ und R⁷ Ethyl sind;
und/oder L₁ einer oder mehr als einer von dem Phenylalaninrest, Alaninrest, Glycinrest, Isoleucinrest, Leucinrest, Prolinrest und Valinrest ist; bevorzugt einer oder mehr als einer von dem Phenylalaninrest, Alaninrest, Glycinrest und Valinrest ist; weiter bevorzugt der Valinrest und/oder der Alaninrest ist, und "mehr als einer" bevorzugt zwei oder drei ist;
und/oder R¹ C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, C₁-C₆-Alkyl oder C₃-C₁₀-Cycloalkyl ist; bevorzugt C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, oder C₁-C₆-Alkyl; weiter bevorzugt C₁-C₆-Alkyl, das durch ein oder mehr als ein -NR¹⁻¹R¹⁻² substituiert ist, oder C₁-C₆-Alkyl, das durch ein oder mehr als ein R¹⁻³S(O)₂₋ substituiert ist; besonders bevorzugt C₁-C₆-Alkyl, das durch ein oder mehr als ein R¹⁻³S(O)₂₋ substituiert ist;
bevorzugt, D ist

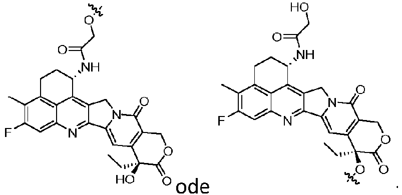

5. Antikörper-Wirkstoff-Konjugat, pharmazeutisch verträgliches Salz davon, Solvat davon oder Solvat des pharmazeutisch verträglichen Salzes davon nach einem der Ansprüche 1 bis 4, wobei das Antikörper-Wirkstoff-Konjugat eines der folgenden Schemata ist:
Schema I:
Ab ist der B7-H3-Antikörper oder die Variante des B7-H3-Antikörpers, bevorzugt der B7-H3-Antikörper;
D ist 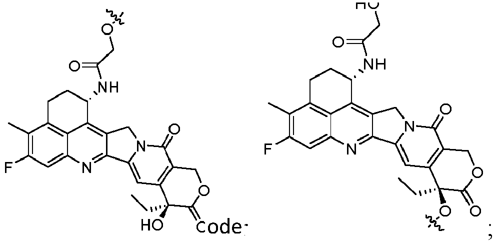
R¹ ist C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, C₁-C₆-Alkyl oder C₃-C₁₀-Cycloalkyl;
L₁ ist unabhängig einer oder mehr als einer von dem Phenylalaninrest, Alaninrest, Glycinrest, Isoleucinrest, Leucinrest, Prolinrest und Valinrest;
Schema II:
Ab ist der B7-H3-Antikörper oder die Variante des B7-H3-Antikörpers, bevorzugt der B7-H3-Antikörper;
D ist 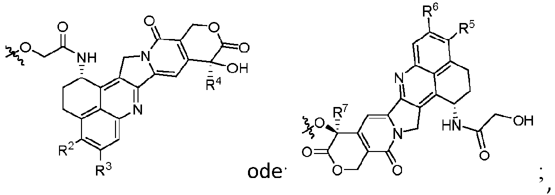 wobei R² und R⁵ jeweils unabhängig C₁-C₆-Alkyl sind; R³ und R⁶ jeweils unabhängig Halogen sind; D bevorzugt ist 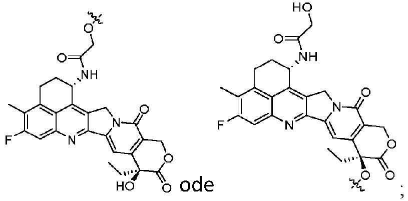
R¹ ist C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, oder C₁-C₆-Alkyl;
L₁ ist unabhängig einer oder mehr als einer von dem Phenylalaninrest, Alaninrest, Glycinrest, Isoleucinrest, Leucinrest, Prolinrest und Valinrest;
Schema III:
Ab ist der B7-H3-Antikörper oder die Variante des B7-H3-Antikörpers, bevorzugt der B7-H3-Antikörper;
D ist 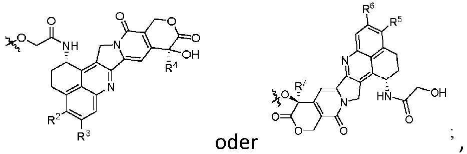 wobei R² und R⁵ jeweils unabhängig C₁-C₆-Alkyl sind; R³ und R⁶ jeweils unabhängig Halogen sind; D bevorzugt ist 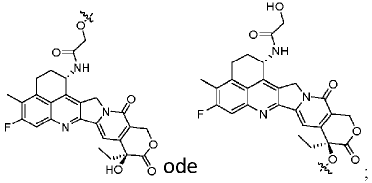
m ist 7 bis 8;
R¹ ist C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, oder C₁-C₆-Alkyl;
L₁ ist unabhängig der Valinrest und/oder der Alaninrest;
Schema IV:
Ab ist der B7-H3-Antikörper oder die Variante des B7-H3-Antikörpers, bevorzugt der B7-H3-Antikörper;
D ist 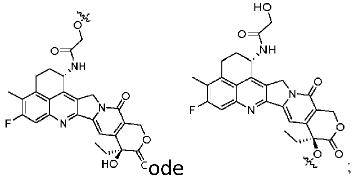
R¹ ist C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, oder C₁-C₆-Alkyl;
Schema V:
Ab ist der B7-H3-Antikörper oder die Variante des B7-H3-Antikörpers, bevorzugt der B7-H3-Antikörper;
D ist 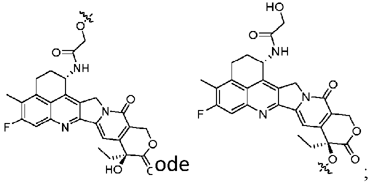
m ist 7 bis 8;
R¹ ist C₁-C₆-Alkyl, das durch ein oder mehr als ein -NR¹⁻¹R¹⁻² substituiert ist, C₁-C₆-Alkyl, das durch ein oder mehr als ein R¹⁻³S(O)₂₋ substituiert ist, oder C₁-C₆-Alkyl;
L₁ ist unabhängig der Valinrest und/oder der Alaninrest.

6. Antikörper-Wirkstoff-Konjugat, pharmazeutisch verträgliches Salz davon, Solvat davon oder Solvat des pharmazeutisch verträglichen Salzes davon nach Anspruch 1,
wobei das Antikörper-Wirkstoff-Konjugat eine der folgenden Verbindungen ist: 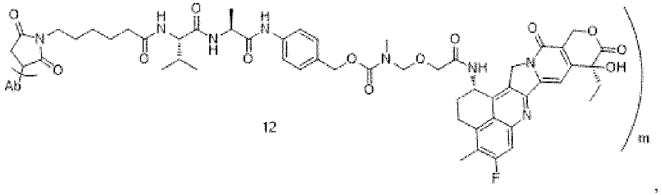 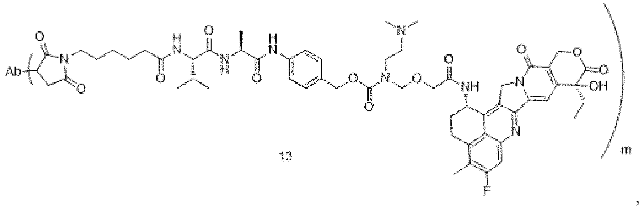 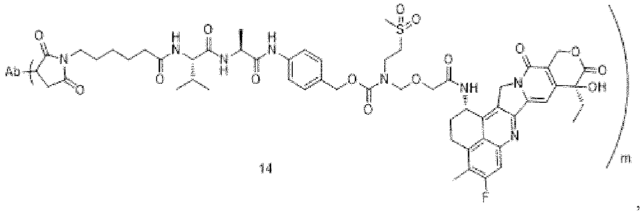 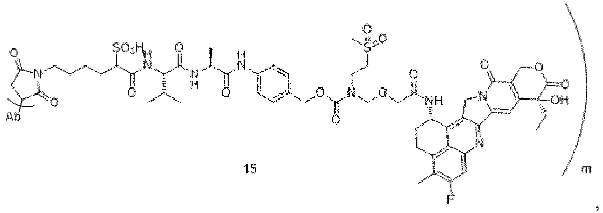 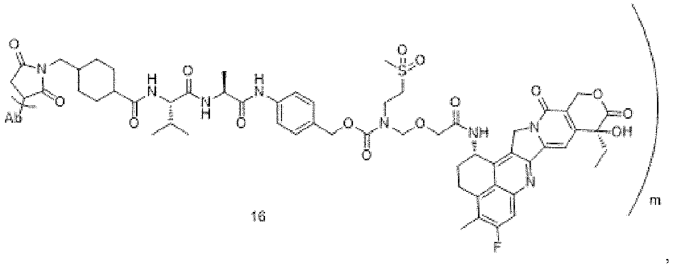 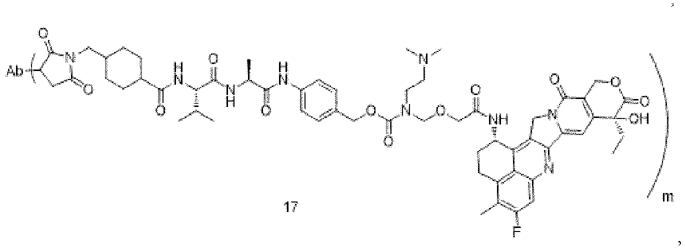 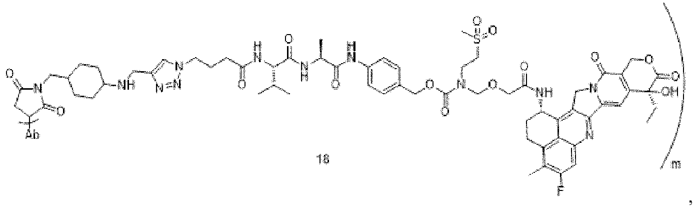 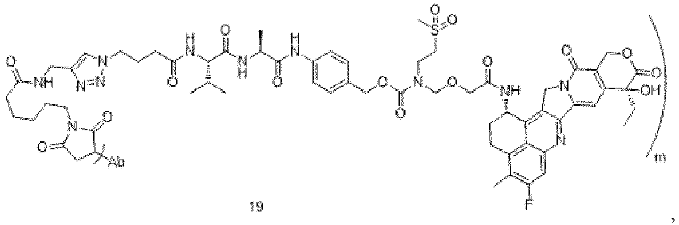 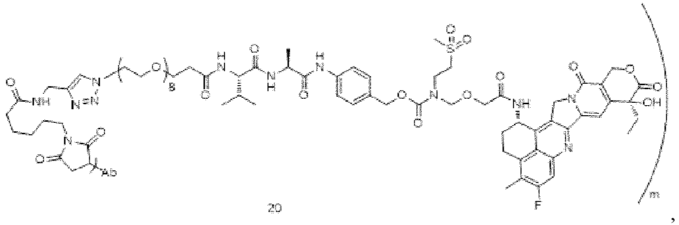 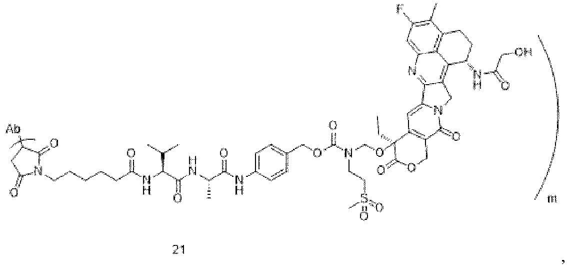 ode 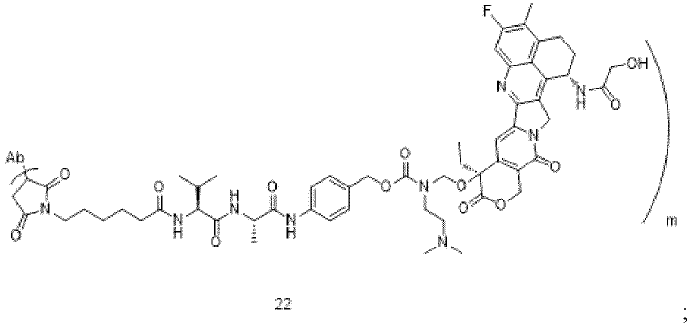 wobei Ab der B7-H3-Antikörper oder die Variante des B7-H3-Antikörpers ist und m 7,47, 7,48, 7,52, 7,62, 7,64, 7,65, 7,67, 7,72, 7,78, 7,83 oder 7,85 ist.

7. Antikörper-Wirkstoff-Konjugat, pharmazeutisch verträgliches Salz davon, Solvat davon oder Solvat des pharmazeutisch verträglichen Salzes davon nach Anspruch 1, wobei das Antikörper-Wirkstoff-Konjugat eine der folgenden Verbindungen ist: 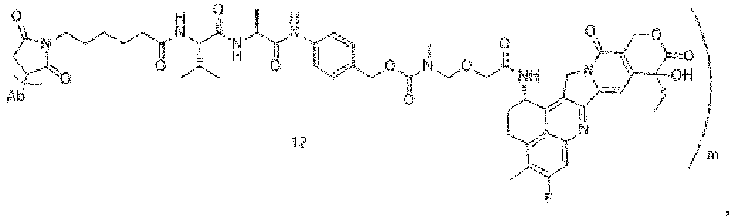 Ab ist der B7-H3-Antikörper und m ist 7,64; 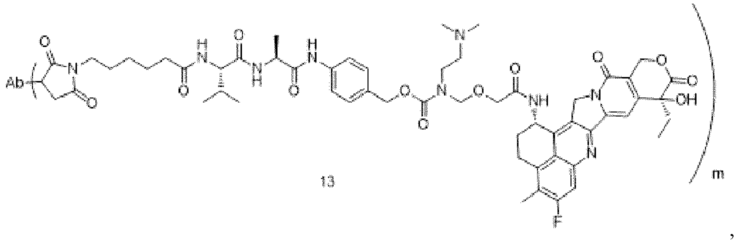 Ab ist der B7-H3-Antikörper und m ist 7,67; 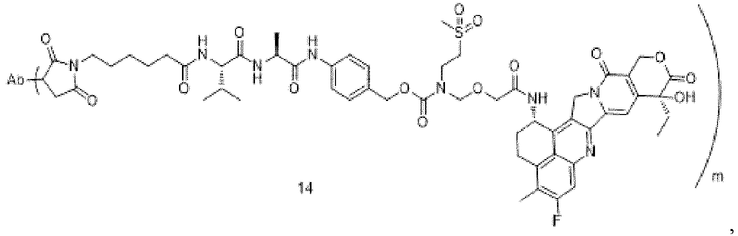 Ab ist der B7-H3-Antikörper und m ist 7,83; 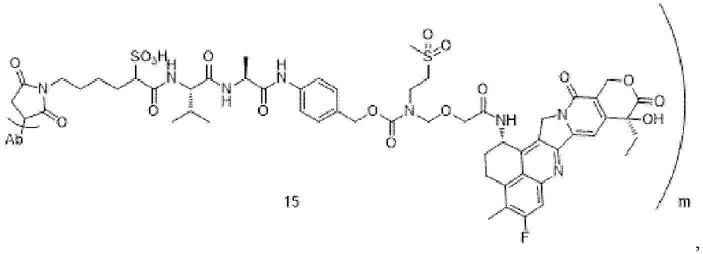 Ab ist der B7-H3-Antikörper und m ist 7,65; 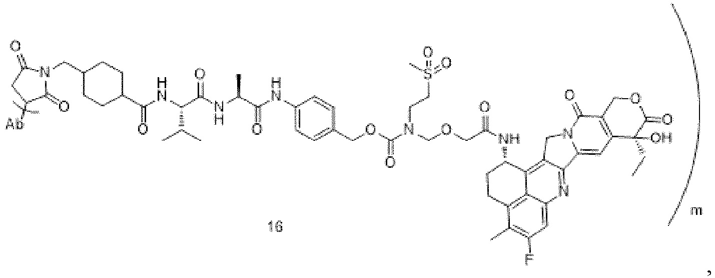 Ab ist der B7-H3-Antikörper und m ist 7,78; 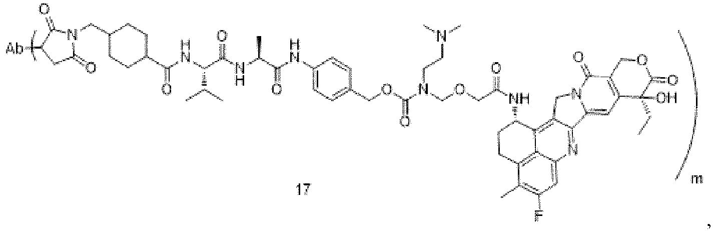 Ab ist der B7-H3-Antikörper und m ist 7,62; 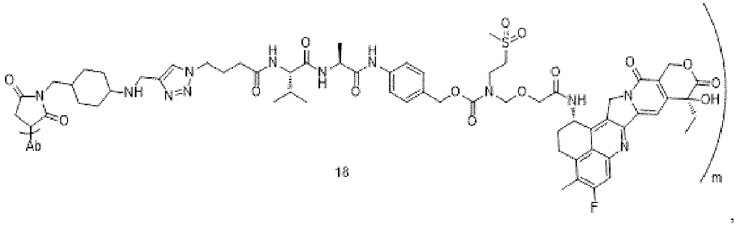 Ab ist der B7-H3-Antikörper und m ist 7,48; 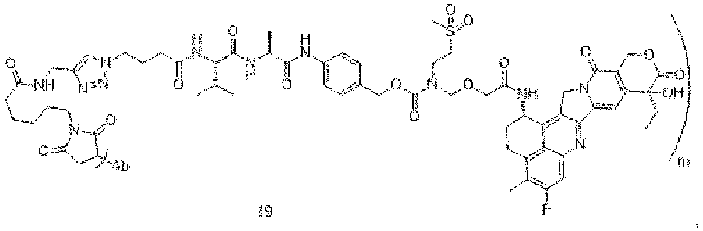 Ab ist der B7-H3-Antikörper und m ist 7,52; 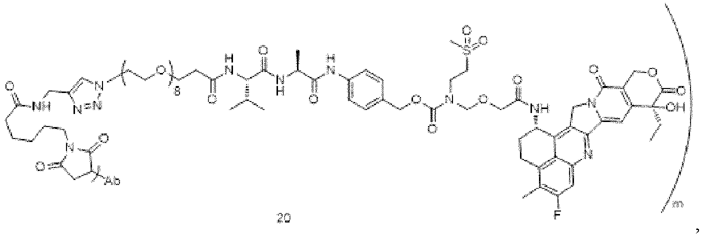 Ab ist der B7-H3-Antikörper und m ist 7,47; 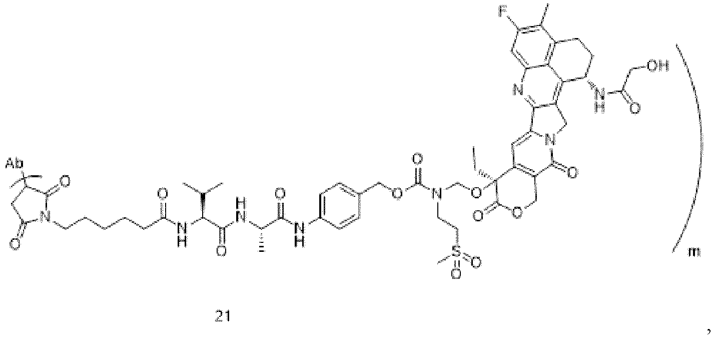 Ab ist der B7-H3-Antikörper und m ist 7,85; oder 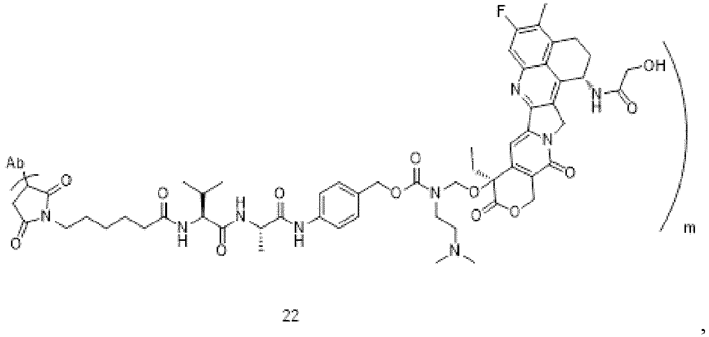 Ab ist der B7-H3-Antikörper und m ist 7,72.

8. Verfahren zum Herstellen des Antikörper-Wirkstoff-Konjugats nach einem der Ansprüche 1 bis 7, das die folgenden Schritte umfasst: Ausführen einer Kopplungsreaktion zwischen einer Verbindung der Formel II und Ab-Wasserstoff, wie unten gezeigt;

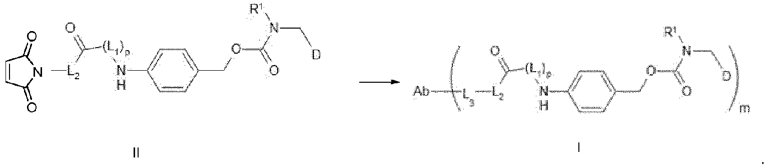

9. Pharmazeutische Zusammensetzung, die Substanz X und einen pharmazeutisch verträglichen Träger umfasst, wobei die Substanz X das Antikörper-Wirkstoff-Konjugat, das pharmazeutisch verträgliche Salz davon, das Solvat davon oder das Solvat des pharmazeutisch verträglichen Salzes davon nach einem der Ansprüche 1 bis 7 ist und die Menge der Substanz X bevorzugt eine therapeutisch wirksame Menge ist.

10. Substanz X zur Verwendung beim Hemmen des B7-H3-Proteins, wobei die Substanz X das Antikörper-Wirkstoff-Konjugat, das pharmazeutisch verträgliche Salz davon, das Solvat davon oder das Solvat des pharmazeutisch verträglichen Salzes davon nach einem der Ansprüche 1 bis 7 ist.

11. Substanz X zur Verwendung beim Behandeln und/oder Vorbeugen eines Tumors, wobei die Substanz X das Antikörper-Wirkstoff-Konjugat, das pharmazeutisch verträgliche Salz davon, das Solvat davon oder das Solvat des pharmazeutisch verträglichen Salzes davon nach einem der Ansprüche 1 bis 7 ist.

12. Substanz X zur Verwendung nach Anspruch 11, wobei der Tumor ein B7-H3-positiver Tumor ist; und der B7-H3-positive Tumor einer oder mehr als einer von B7-H3-positivem Lungenkrebs, Eierstockkrebs, Melanom, Bauchspeicheldrüsenkrebs, Brustkrebs, Hirngliom, Prostatakrebs und Magenkrebs ist.

13. Substanz X zur Verwendung nach Anspruch 12, wobei die Lungenkrebszellen *NCI-*1703-Zellen oder Calu-6-Zellen sind;
und/oder die Eierstockkrebszellen PA-1-Zellen sind;
und/oder die Melanomzellen A375-Zellen sind;
und/oder die Bauchspeicheldrüsenkrebszellen Hs-700T-Zellen sind;
und/oder die Brustkrebszellen MDA-MB-468-Zellen sind;
und/oder die Hirngliomzellen LN-229-Zellen sind;
und/oder die Magenkrebszellen NCI-N87-Zellen sind.

## Revendications

1. Conjugué anticorps-médicament, sel pharmaceutiquement acceptable de celui-ci, solvate de celui-ci ou solvate du sel pharmaceutiquement acceptable de celui-ci, dans lequel le conjugué anticorps-médicament présente une structure illustrée dans la formule **I;** 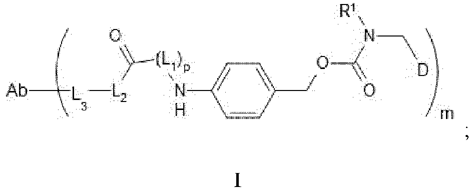
dans lequel Ab est un anticorps B7-H3 ou une variante de l'anticorps B7-H3 ; m vaut de 2 à 8 ;
la séquence d'acides aminés de la chaîne légère de l'anticorps B7-H3 est présentée dans SEQ ID NO : 1, et la séquence d'acides aminés de la chaîne lourde est présentée dans SEQ ID NO : 2 ;
le variant de l'anticorps B7-H3 est au moins 70 %, 75 %, 80 %, 85 %, 90 %, 95 %, 98 %, ou 99 % identique à l'anticorps B7-H3 ;
D est un inhibiteur de topoisomérase de médicament cytotoxique, dans lequel l'inhibteur de topoisomérase de médicament cytotoxique
est 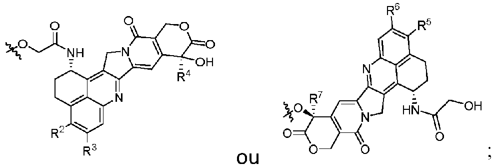 R² et R⁵ sont chacun indépendamment H, alkyle en C₁-C₆ ou halogène ; R³ et R⁶ sont chacun indépendamment H, alkyle en C₁-C₆ ou halogène ; R⁴ et R⁷ sont chacun indépendamment alkyle en C₁-C₆ ;
R¹ est alkyle en C₁-C₆ substitué par un ou plusieurs -NR¹⁻¹R¹⁻², alkyle en C₁-C₆ substitué par un ou plusieurs R¹⁻³S(O)₂₋, alkyle en C₁-C₆, cycloalkyle en C₃-C₁₀, aryle en C₆-C₁₄ ou hétéroaryle à 5 à 14 chaînons ; l'hétéroatome dans l'hétéroaryle à 5 à 14 chaînons est choisi parmi un ou plusieurs parmi N, O et S, et le nombre d'hétéroatomes est de 1, 2, 3 ou 4 ; les R¹⁻¹, R¹⁻² et R¹⁻³ sont chacun indépendamment alkyle en C₁-C₆ ;
L₁ est indépendamment un ou plusieurs éléments parmi un résidu de phénylalanine, un résidu d'alanine, un résidu de glycine, un résidu d'acide glutamique, un résidu d'acide aspartique, un résidu de cystéine, un résidu d'histidine, un résidu d'isoleucine, un résidu de leucine, un résidu de lysine, un résidu de méthionine, un résidu de proline, un résidu de sérine, un résidu de thréonine, un résidu de tryptophane, un résidu de tyrosine et un résidu de valine ; p vaut de 2 à 4 ;
L₂ est 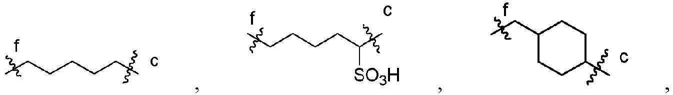 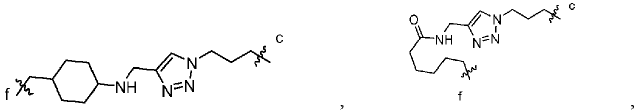 ou 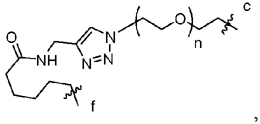 dans lequel n vaut indépendamment 1 à 12, l'extrémité c-terminale est reliée à L₁ par un groupe carbonyle et l'extrémité f-terminale est reliée à l'extrémité d-terminale de L₃ ;
L₃ est 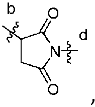 dans lequel l'extrémité b-terminale est reliée à l'Ab, et l'extrémité d-terminale est reliée à l'extrémité f-terminale de L₂.

2. Conjugué anticorps-médicament, sel pharmaceutiquement acceptable de celui-ci, solvate de celui-ci, ou solvate du sel pharmaceutiquement acceptable de celui-ci selon la revendication 1, dans lequel
l'extrémité b-terminale de L₃ est reliée au groupe sulfhydryle de l'anticorps sous la forme d'un thioéther ;
et/ou, lorsque R¹ est alkyle en C₁-C₆ substitué par un ou plusieurs -NR¹⁻¹R¹⁻², l'alkyle en C₁-C₆ est méthyle, éthyle, n-propyle, isopropyle, n-butyle, isobutyle ou tert-butyle, de préférence éthyle ;
et/ou, lorsque R¹ est alkyle en C₁-C₆ substitué par plus d'un -NR¹⁻¹R¹⁻², le « plus d'un » est deux ou trois ;
et/ou, lorsque R¹⁻¹ et R¹⁻² sont chacun indépendamment alkyle en C₁-C₆, l'alkyle en C₁-C₆ est méthyle, éthyle, n-propyle, isopropyle, n-butyle, isobutyle ou tert-butyle, de préférence méthyle ;
et/ou, lorsque R¹ est alkyle en C₁-C₆ substitué par un ou plusieurs R¹⁻³S(O)₂₋, l'alkyle en C₁-C₆ est alkyle en C₁-C₄, de préférence méthyle, éthyle, n-propyle, isopropyle, n-butyle, isobutyle ou tert-butyle, plus préférentiellement éthyle ;
et/ou, lorsque R¹ est alkyle en C₁-C₆ substitué par plus d'un R¹⁻³S(O)₂₋, le « plus d'un » est deux ou trois ;
et/ou, lorsque R¹⁻³ est alkyle en C₁-C₆, l'alkyle en C₁-C₆ est méthyle, éthyle, n-propyle, isopropyle, n-butyle, isobutyle ou tert-butyle, de préférence méthyle ;
et/ou, lorsque R¹ est alkyle en C₁-C₆, l'alkyle en C₁-C₆ est méthyle, éthyle, n-propyle, isopropyle, n-butyle, isobutyle ou tert-butyle, de préférence méthyle ;
et/ou, m est un nombre entier ou non entier, de préférence de 4 à 8, plus préférentiellement de 6 à 8, encore plus préférentiellement de 7 à 8 ;
et/ou, p vaut 2 ;
et/ou, n vaut de 8 à 12 ;
et/ou, lorsque R¹⁻¹, R¹⁻² et R¹⁻³ sont indépendamment alkyle en C₁-C₆, l'alkyle en C₁-C₆ est alkyle en C₁-C₄.

3. Conjugué anticorps-médicament, sel pharmaceutiquement acceptable de celui-ci, solvate de celui-ci, ou solvate du sel pharmaceutiquement acceptable de celui-ci selon la revendication 1, dans lequel
lorsque R² et R⁵ sont chacun indépendamment alkyle en C₁-C₆, l'alkyle en C₁-C₆ est alkyle en C₁-C₄, de préférence encore méthyle, éthyle, n-propyle, isopropyle, n-butyle, isobutyle ou tert-butyle, le plus préférentiellement méthyle ;
et/ou, lorsque R² et R⁵ sont chacun indépendamment halogène, l'halogène est fluor, chlore, brome ou iode, de préférence fluor ;
et/ou, lorsque R³ et R⁶ sont chacun indépendamment alkyle en C₁-C₆, l'alkyle en C₁-C₆ est alkyle en C₁-C₄, de préférence encore méthyle, éthyle, n-propyle, isopropyle, n-butyle, isobutyle ou tert-butyle, le plus préférentiellement méthyle ;
et/ou, lorsque R³ et R⁶ sont chacun indépendamment halogène, l'halogène est fluor, chlore, brome ou iode, de préférence encore fluor ;
et/ou, lorsque R⁴ et R⁷ sont chacun indépendamment alkyle en C₁-C₆, l'alkyle en C₁-C₆ est alkyle en C₁-C₄, de préférence encore méthyle, éthyle, n-propyle, isopropyle, n-butyle, isobutyle ou tert-butyle, le plus préférentiellement éthyle ;
et/ou, lorsque R¹ est alkyle en C₁-C₆ substitué par un ou plusieurs -NR¹⁻¹R¹⁻², le -NR¹⁻¹R¹⁻² est -N(CH₃)₂ ;
et/ou, lorsque R¹ est alkyle en C₁-C₆ substitué par un -NR¹⁻¹R¹⁻², l'alkyle en C₁-C₆ substitué par un -NR¹⁻¹R¹⁻² est 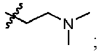
et/ou, lorsque R¹ est alkyle en C₁-C₆ substitué par un R¹⁻³S(O)₂₋, l'alkyle en C₁-C₆
substitué par un R¹⁻³S(O)₂- est 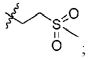
et/ou, m vaut de 7,4 à 7,85, tel que 7,47, 7,48, 7,52, 7,62, 7,64, 7,65, 7,67, 7,72, 7,78, 7,83 ou 7,85 ;
et/ou, (L₁)ₚ est 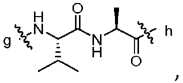 dans lequel l'extrémité g-terminale est reliée à l'extrémité c-terminale de L₂ par un groupe carbonyle ;
et/ou, n vaut 8, 9, 10, 11 ou 12 ;
et/ou, lorsque R¹⁻¹, R¹⁻² et R¹⁻³ sont chacun indépendamment alkyle en C₁-C₆, l'aklyle en C₁-C₆ est méthyle, éthyle, n-propyle, isopropyle, n-butyle, isobutyle ou tert-butyle, de préférence méthyle.

4. Conjugué anticorps-médicament, sel pharmaceutiquement acceptable de celui-ci, solvate de celui-ci, ou solvate du sel pharmaceutiquement acceptable de celui-ci selon la revendication 1, dans lequel
R² et R⁵ sont chacun indépendamment alkyle en C₁-C₆ ;
et/ou, R³ et R⁶ sont chacun indépendamment halogène ;
et/ou, R⁴ et R⁷ sont éthyle ;
et/ou, L₁ est un ou plusieurs éléments parmi le résidu de phénylalanine, le résidu d'alanine, le résidu de glycine, le résidu d'isoleucine, le résidu de leucine, le résidu de proline et le résidu de valine ; de préférence un ou plusieurs éléments parmi le résidu de phénylalanine, le résidu d'alanine, le résidu de glycine et le résidu de valine ; encore plus préférentiellement le résidu de valine et/ou le résidu d'alanine, et le « plusieurs » est de préférence deux ou trois ;
et/ou, R¹ est alkyle en C₁-C₆ substitué par un ou plusieurs -NR¹⁻¹R¹⁻², alkyle en C₁-C₆ substitué par un ou plusieurs R¹⁻³S(O)₂₋, alkyle en C₁-C₆ ou cycloalkyle en C₃-C₁₀ ; de préférence alkyle en C₁-C₆ substitué par un ou plusieurs -NR¹⁻¹R¹⁻², alkyle en C₁-C₆ substitué par un ou plusieurs R¹⁻³S(O)₂₋, ou alkyle en C₁-C₆ ; de préférence encore alkyle en C₁-C₆ substitué par un ou plusieurs -NR¹⁻¹R¹⁻², ou alkyle en C₁-C₆ substitué par un ou plusieurs R¹⁻³S(O)₂₋ ; le plus préférentiellement alkyle en C₁-C₆ substitué par un ou plusieurs R¹⁻³S(O)₂₋ ;
de préférence, D est

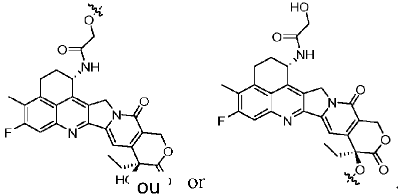

5. Conjugué anticorps-médicament, sel pharmaceutiquement acceptable de celui-ci, solvate de celui-ci, ou solvate du sel pharmaceutiquement acceptable de celui-ci selon l'une quelconque des revendications 1 à 4, dans lequel le conjugué anticorps-médicament est l'un quelconque des systèmes suivants :
système I :
Ab est l'anticorps B7-H3 ou le variant de l'anticorps B7-H3, de préférence l'anticorps B7-H3 ;
D est 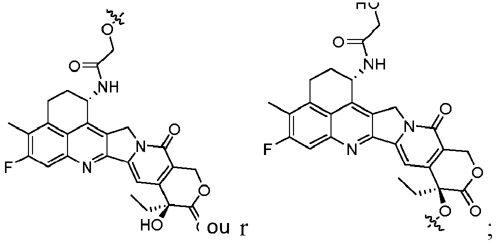
R¹ est alkyle en C₁-C₆ substitué par un ou plusieurs -NR¹⁻¹R¹⁻², alkyle en C₁-C₆ substitué par un ou plusieurs R¹⁻³S(O)₂₋, alkyle en C₁-C₆ ou cycloalkyle en C₃-C₁₀ ;
L₁ est indépendamment un ou plusieurs éléments parmi le résidu de phénylalanine, le résidu d'alanine, le résidu de glycine, le résidu d'isoleucine, le résidu de leucine, le résidu de proline et le résidu de valine ;
système Il :
Ab est l'anticorps B7-H3 ou le variant de l'anticorps B7-H3, de préférence l'anticorps B7-H3 ;
D est 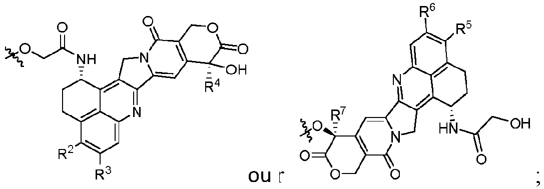 dans lequel R² et R⁵ sont chacun indépendamment alkyle en C₁-C₆ ; R³ et R⁶ sont chacun indépendamment halogène ; D est de préférence 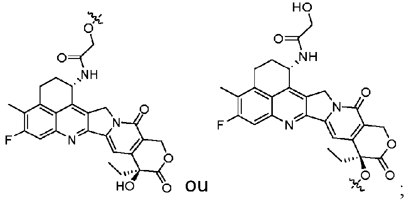
R¹ est alkyle en C₁-C₆ substitué par un ou plusieurs -NR¹⁻¹R¹⁻², alkyle en C₁-C₆ substitué par un ou plusieurs R¹⁻³S(O)₂₋, ou alkyle en C₁-C₆ ;
L₁ est indépendamment un ou plusieurs éléments parmi le résidu de phénylalanine, le résidu d'alanine, le résidu de glycine, le résidu d'isoleucine, le résidu de leucine, le résidu de proline et le résidu de valine ;
système III :
Ab est l'anticorps B7-H3 ou le variant de l'anticorps B7-H3, de préférence l'anticorps B7-H3 ;
D est 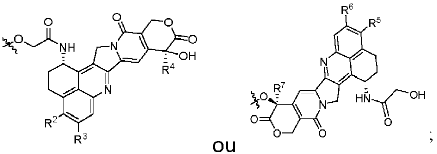 dans lequel R² et R⁵ sont chacun indépendamment alkyle en C₁-C₆; R³ et R⁶ sont chacun indépendamment halogène ; D est de préférence 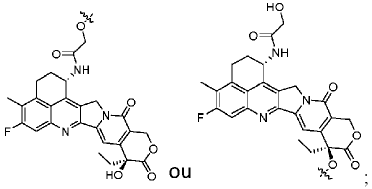
m vaut 7 à 8 ;
R¹ est alkyle en C₁-C₆ substitué par un ou plusieurs -NR¹⁻¹R¹⁻², alkyle en C₁-C₆ substitué par un ou plusieurs R¹⁻³S(O)₂₋, ou alkyle en C₁-C₆ ;
L₁ est indépendamment le résidu de valine et/ou le résidu d'alanine ;
système IV :
Ab est l'anticorps B7-H3 ou le variant de l'anticorps B7-H3, de préférence l'anticorps B7-H3 ;
D est 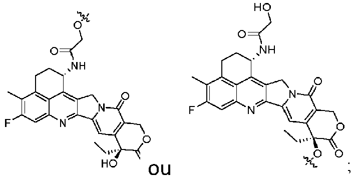
R¹ est alkyle en C₁-C₆ substitué par un ou plusieurs -NR¹⁻¹R¹⁻², alkyle en C₁-C₆ substitué par un ou plusieurs R¹⁻³S(O)₂₋, ou alkyle en C₁-C₆ ;
système V :
Ab est l'anticorps B7-H3 ou le variant de l'anticorps B7-H3, de préférence l'anticorps B7-H3 ;
D est 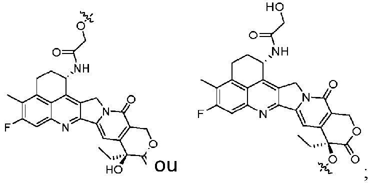
m vaut 7 à 8 ;
R¹ est alkyle en C₁-C₆ substitué par un ou plusieurs -NR¹⁻¹R¹⁻², alkyle en C₁-C₆ substitué par un ou plusieurs R¹⁻³S(O)₂₋, ou alkyle en C₁-C₆ ;
L₁ est indépendamment le résidu de valine et/ou le résidu d'alanine.

6. Conjugué anticorps-médicament, sel pharmaceutiquement acceptable de celui-ci, solvate de celui-ci, ou solvate du sel pharmaceutiquement acceptable de celui-ci selon la revendication 1,
dans lequel le conjugué anticorps-médicament est l'un quelconque des composés suivants : 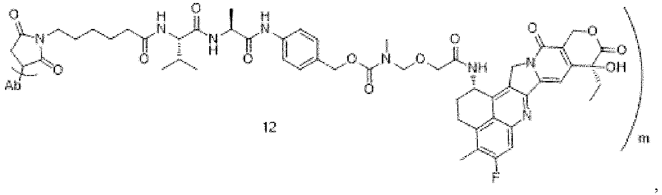 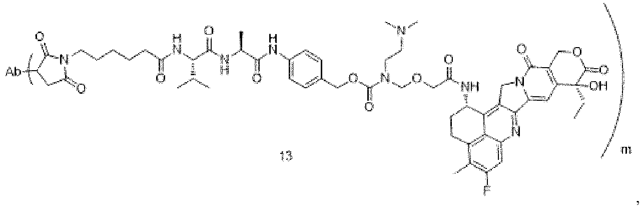 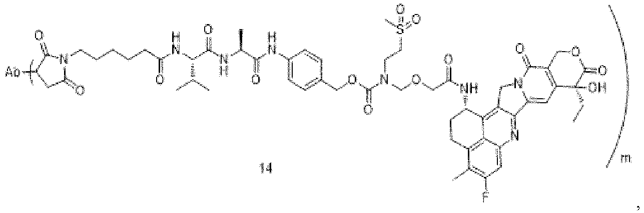 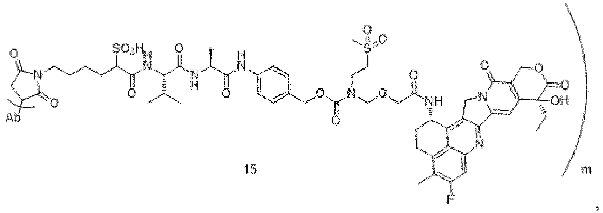 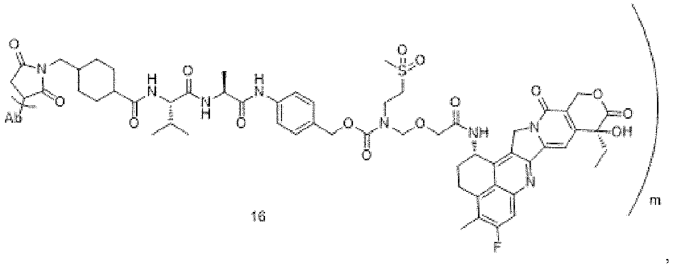 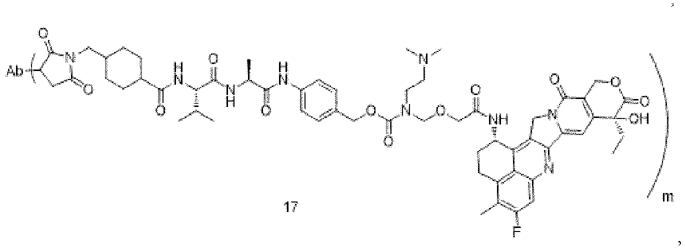 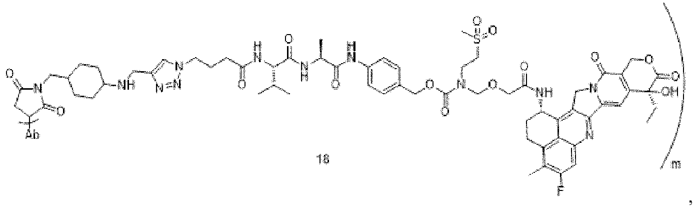 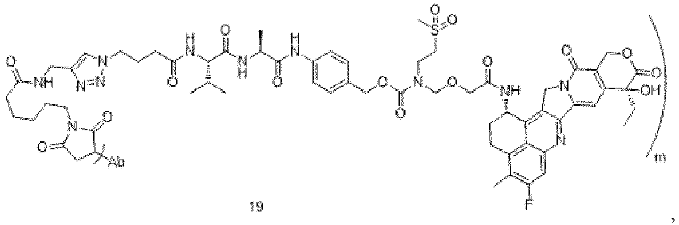 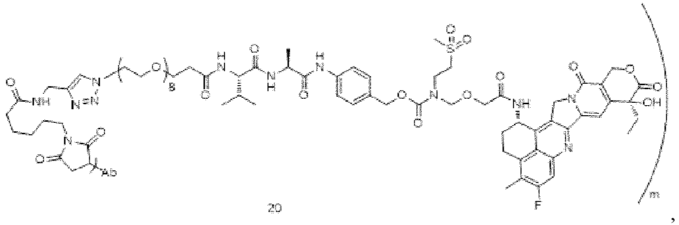 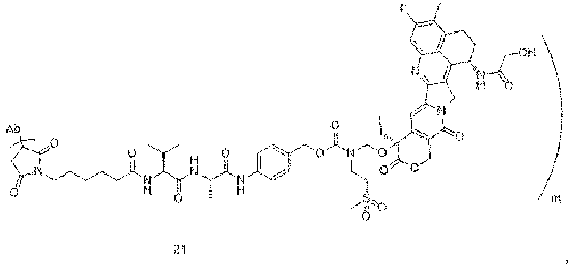 ou 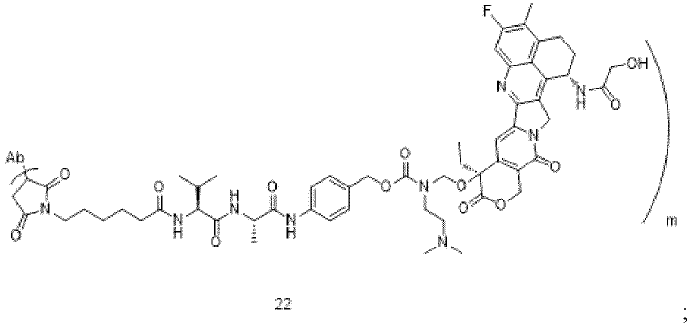
dans lequel, Ab est l'anticorps B7-H3 ou le variant de l'anticorps B7-H3, et m vaut 7,47, 7,48, 7,52, 7,62, 7,64, 7,65, 7,67, 7,72, 7,78, 7,83 ou 7,85.

7. Conjugué anticorps-médicament, sel pharmaceutiquement acceptable de celui-ci, solvate de celui-ci, ou solvate du sel pharmaceutiquement acceptable de celui-ci selon la revendication 1, dans lequel le conjugué anticorps-médicament est l'un quelconque des composés suivants : 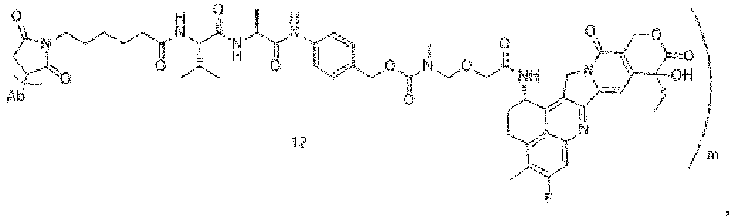 Ab est l'anticorps B7-H3 et m vaut 7,64 ; 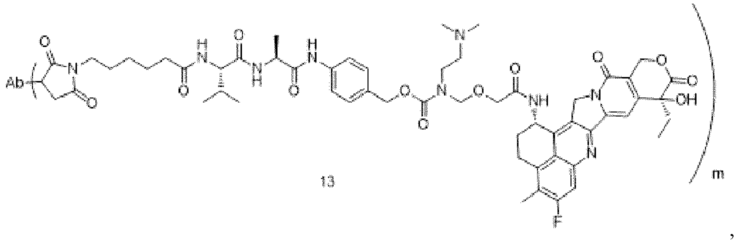 Ab est l'anticorps B7-H3 et m vaut 7,67 ; 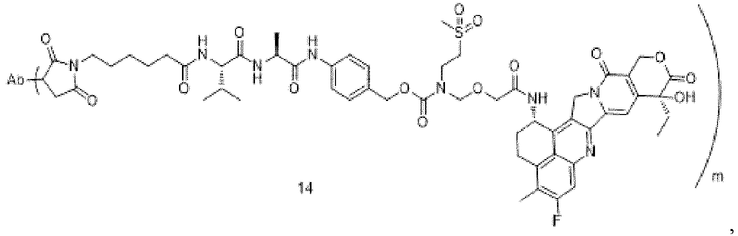 Ab est l'anticorps B7-H3 et m vaut 7,83 ; 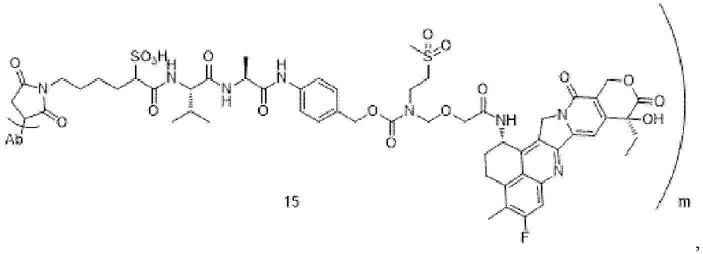 Ab est l'anticorps B7-H3 et m vaut 7,65 ; 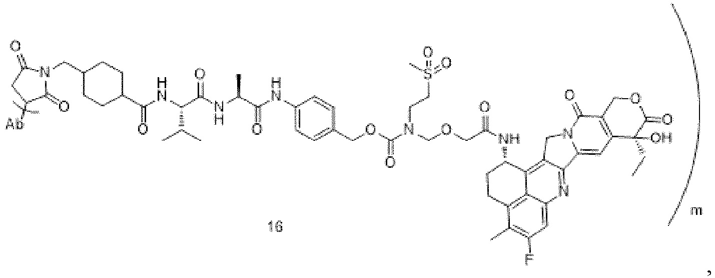 Ab est l'anticorps B7-H3 et m vaut 7,78 ; 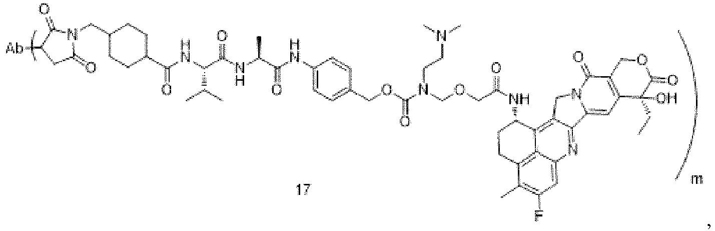 Ab est l'anticorps B7-H3 et m vaut 7,62 ; 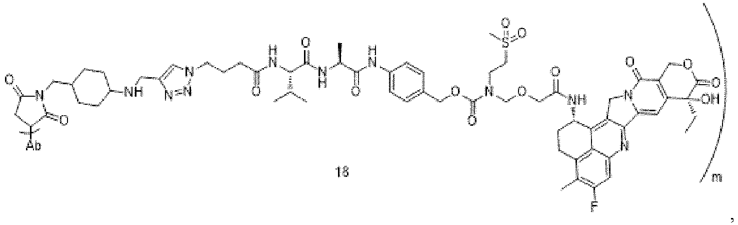 Ab est l'anticorps B7-H3 et m vaut 7,48 ; 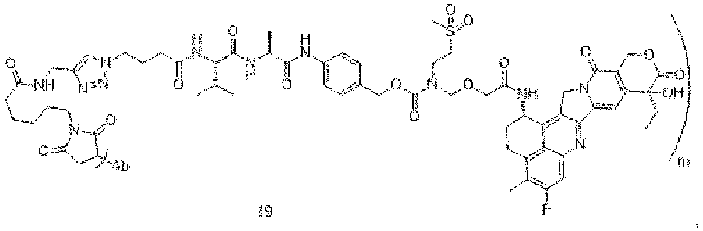 Ab est l'anticorps B7-H3 et m vaut 7,52 ; 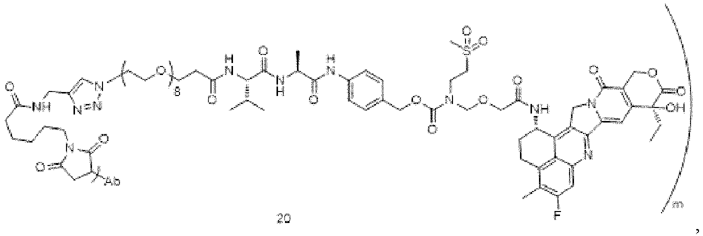 Ab est l'anticorps B7-H3 et m vaut 7,47 ; 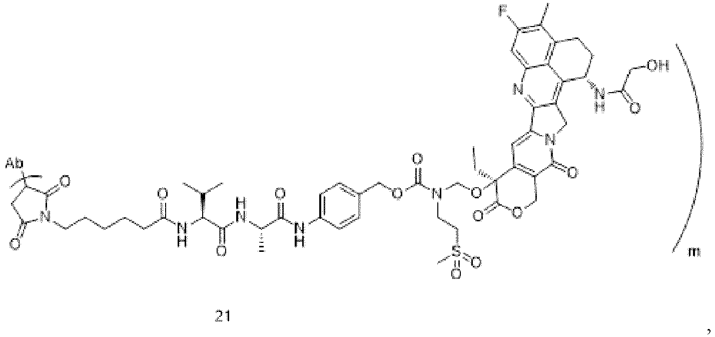 Ab est l'anticorps B7-H3 et m vaut 7,85 ; ou 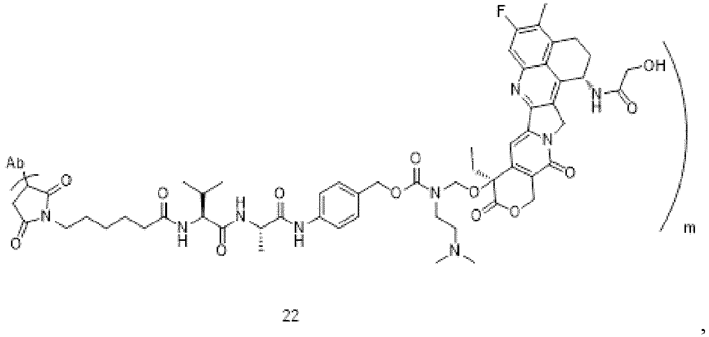 Ab est l'anticorps B7-H3 et m vaut 7,72.

8. Procédé de préparation du conjugué anticorps-médicament selon l'une quelconque des revendications 1 à 7, comprenant les étapes suivantes : la réalisation d'une réaction de couplage entre un composé de formule II et Ab-hydrogène comme indiqué ci-dessous ;

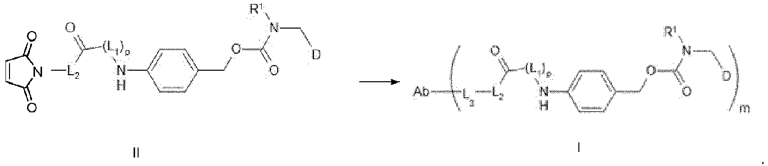

9. Composition pharmaceutique, comprenant une substance X et un excipient pharmaceutiquement acceptable, dans laquelle la substance X est le conjugué anticorps-médicament, le sel pharmaceutiquement acceptable de celui-ci, le solvate de celui-ci ou le solvate du sel pharmaceutiquement acceptable de celui-ci selon l'une quelconque des revendications 1 à 7, la quantité de la substance X est de préférence une quantité thérapeutiquement efficace.

10. Substance X destinée à être utilisée pour inhiber la protéine B7-H3, dans laquelle la substance X est le conjugué anticorps-médicament, le sel pharmaceutiquement acceptable de celui-ci, le solvate de celui-ci ou le solvate du sel pharmaceutiquement acceptable de celui-ci selon l'une quelconque des revendications 1 à 7.

11. Substance X pour utilisation dans le traitement et/ou la prévention d'une tumeur, dans laquelle la substance X est le conjugué anticorps-médicament, le sel pharmaceutiquement acceptable de celui-ci, le solvate de celui-ci ou le solvate du sel pharmaceutiquement acceptable de celui-ci selon l'une quelconque des revendications 1 à 7.

12. Substance X pour utilisation selon la revendication 11, dans laquelle la tumeur est une tumeur B7-H3 positive ; et la tumeur B7-H3 positive est un ou plusieurs éléments parmi un cancer du poumon B7-H3 positif, un cancer de l'ovaire, un mélanome, un cancer du pancréas, un cancer du sein, un gliome cérébral, un cancer de la prostate et un cancer de l'estomac.

13. Substance X pour utilisation selon la revendication 12, dans laquelle les cellules cancéreuses du poumon sont des cellules *NCI-1703* ou des cellules Calu-6 ;
et/ou, les cellules cancéreuses ovariennes sont des cellules PA-1 ;
et/ou, les cellules du mélanome sont des cellules A375 ;
et/ou, les cellules cancéreuses du pancréas sont des cellules Hs-700T ;
et/ou, les cellules cancéreuses du sein sont des cellules MDA-MB-468 ;
et/ou, les cellules du gliome cérébral sont des cellules LN-229 ;
et/ou, les cellules cancéreuses de l'estomac sont des cellules NCI-N87.
